# FASCICULE DE BREVET EUROPEEN

(11) **EP 1 161 481 B1**
(45) Date de publication et mention de la délivrance du brevet: **04.12.2002**
(21) Numéro de dépôt: 00900590.1
(22) Date de dépôt: 14.01.2000
(51) Int. Cl.: C08G 79/06

(54) **POLYMERE LINEAIRE OPTIQUEMENT ACTIF UTILISABLE COMME LIGAND DANS LA PREPARATION DE COMPLEXES METALLIQUES DESTINES A LA CATALYSE ASYMETRIQUE**
LINEAR, OPTISCH AKTIVES POLYMER, VERWENDBAR ALS LIGAND IN DER HERSTELLUNG VON ASYMMETRISCHEN METALLKOMPLEXKATALYSATOREN
OPTICALLY ACTIVE LINEAR POLYMER USED AS LIGAND IN THE PREPARATION OF METALLIC COMPLEXES DESIGNED FOR ASYMMETRIC CATALYSIS

(30) Priorité: 01.03.1999 FR 9902510
(43) Date de publication de la demande: 12.12.2001
(73) Titulaire: RHODIA CHIMIE, 92512 Boulogne Billancourt Cedex (FR); CENTRE NATIONAL DE LA RECHERCHE SCIENTIFIQUE (CNRS), 75016 Paris (FR)
(72) Inventeur: LEMAIRE, Marc, F-69100 Villeurbanne (FR); TER HALLE, Rob, F-69660 Collonges au Mont d'Or (FR); SCHULZ, Emmanuelle, F-69110 Sainte Foy lès Lyon (FR); COLASSON, Benoît, F-44980 Sainte Luce sur Loire (FR); SPAGNOL, Michel, F-69330 Meyzière (FR); SALUZZO, Christine, F-69003 Lyon (FR); LAMOUILLE, Thierry, F-69007 Lyon (FR)
(74) Mandataire: Bernasconi, Jean Raymond
(86) Numéro de dépôt international: FR0000082
(87) Numéro de publication internationale: WO00052081

(56) Documents cités:
- EP-A- 0 728 768
- WO-A-97/02232
- WO-A-98/12202

## Description

L'invention concerne un ligand polymérique diphosphoré, optiquement actif, utile dans la préparation de complexes métalliques destinés à la catalyse asymétrique.

La catalyse asymétrique a connu ces dernières années un essor considérable. Elle présente l'avantage de conduire directement à la préparation d'isomères optiquement purs par induction asymétrique sans qu'il soit nécessaire de procéder au dédoublement de mélanges racémiques.

Le 2,2'-bis(diphénylphosphino)-1,1'-binaphtyl (BINAP) est un exemple de ligand diphosphoré utilisé couramment pour la préparation de complexes métalliques pour la catalyse asymétrique des réactions d'hydrogénation, de carbonylation, d'hydrosylilation, de formation de liaisons C-C (telles que les substitutions allyliques ou les couplages croisés de Grignard) ou même d'isomérisation asymétrique d'allylamines.

Les complexes métalliques préparés à partir du BINAP sont des complexes du rhodium, de l'iridium, du palladium, du platine ou du ruthénium qui sont des métaux d'un prix de revient très élevé.

Du fait du coût élevé des métaux les constituant, les procédés industriels utilisant ces complexes métalliques comme catalyseur comprennent nécessairement des étapes consacrées à leur récupération et à leur recyclage.

Or, le plus souvent, les complexes métalliques du type de ceux préparés à partir du BINAP sont mis en oeuvre dans la catalyse homogène, c'est-à-dire qu'ils sont utilisés en solution dans le milieu réactionnel.

Dans ces circonstances, les étapes d'extraction du catalyseur et de récupération sont complexes et rendent délicate et laborieuse la mise en oeuvre de ces procédés, à l'échelle industrielle.

L'invention vise à résoudre ce problème en fournissant notamment un procédé plus économique dans lequel les étapes d'extraction du catalyseur, de récupération et de recyclage sont simplifiées et compatibles avec une industrialisation du procédé. Selon ce procédé, le catalyseur métallique est solide et séparé du milieu réactionnel par simple filtration, la catalyse ayant lieu en phase hétérogène.

L'originalité de l'invention repose sur la nature particulière du complexe métallique utilisé comme catalyseur et plus précisément sur la nature du ligand phosphoré utilisé pour préparer le complexe métallique catalyseur, laquelle détermine le caractère peu soluble du catalyseur.

La mise au point de complexes métalliques phosphorés utilisables dans la catalyse hétérogène et séparable du milieu réactionnel par filtration a déjà été envisagée dans la technique.

D.J. Bayston et al. ont décrit dans J. Org. Chem., 1998, 63, 3137-3140, un catalyseur préparé à partir d'un ligand polymère constitué d'une diphosphine chirale greffée sur un support polymérique. La formule de ce ligand peut être schématisée comme suit, où PS désigne un support polymérique constitué de polystyrène et Ph désigne le radical phényle :

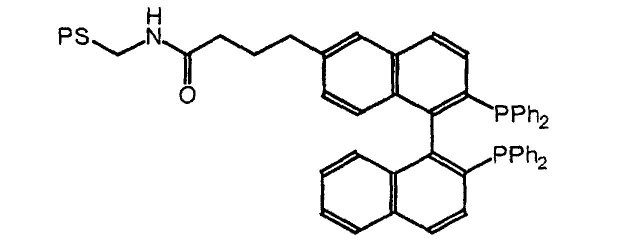

Plus généralement, WO 98/12202 décrit des ligands polymères pour la préparation de complexes métalliques destinés à la catalyse asymétrique. Ces ligands, de formule : 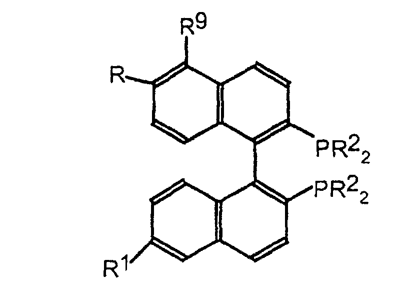 dans laquelle R¹ représente -Y⁰-X⁰-R⁴ où R⁴ comprend un support insoluble dérivé d'un polystyrène, d'un polyamide ou d'une résine polymère, sont également constitués d'une diphosphine chirale greffée sur un support polymérique.

Les complexes catalyseurs préparés à partir des ligands de l'art antérieur ne présentent qu'un unique site chiral par chaîne polymère, ce qui nécessite la mise en jeu de masses catalytiques importantes pour une catalyse efficace, la masse de la chaîne polymère s'ajoutant à la masse de la molécule chirale greffée.

De plus, les ligands de l'art antérieur ne présentent pas la symétrie d'axe C₂ de la molécule BINAP. Or, on sait que les ligands polymériques à symétrie d'axe C₂ conduisent à d'excellentes énantiosélectivités dans la catalyse de réactions asymétriques.

Le polymère de l'invention, utilisable comme ligand dans la préparation de complexes catalyseurs, présente plusieurs centres optiquement actifs, et une structure modulable, ce qui permet notamment la préparation de polymères présentant dans leur ensemble une symétrie d'axe C₂.

Le polymère de l'invention est constitué d'un enchaînement de deux types de motifs.

Le premier type de motif est le reste d'une diphosphine chirale présentent un axe de symétrie C₂, à l'exclusion de tout autre élément de symétrie, et portant deux groupes fonctionnels identiques polymérisables.

Le second type de motif est le reste d'un monomère polymérisable avec lesdits groupes fonctionnels, c'est-à-dire un monomère comprenant deux groupes fonctionnels identiques capables de réagir avec les groupes fonctionnels de la diphosphine chirale.

Du fait de la récurrence du motif chiral dans la chaîne polymère, des quantités bien inférieures du catalyseur métallique correspondant sont nécessaires pour une catalyse efficace des réactions asymétriques. De plus, à partir d'un monomère polymérisable présentant également un axe de symétrie C₂, on obtient un polymère présentant dans son ensemble une symétrie d'axe C₂, lequel conduit à une énantiosélectivité bien supérieure par comparaison aux polymères greffés de l'art antérieur.

Plus précisément, l'invention concerne un polymère optiquement actif, utilisable comme ligand dans la préparation de complexes métalliques destinés à la catalyse asymétrique. Ces polymères peuvent être obtenus par polymérisation d'une diphosphine chirale présentant un axe de symétrie C₂, à l'exclusion de tout autre élément de symétrie, avec un ou plusieurs monomères polymérisables, Selon l'invention, la diphosphine chirale est constituée d'un corps chiral (ou squelette chiral) portant deux groupes fonctionnels identiques capables de réagir avec les monomères polymérisables.

La notion de plan de symétrie et d'axe de symétrie C₂ est décrite dans Kurt Mislow in "Introduction to stereochemistry", W.A. Benjamin, Inc. New York, Amsterdam - 1965.

Une molécule présentant un axe de symétrie C₂ est telle que, par rotation de 180° de cette molécule autour de l'axe de symétrie, on obtient une molécule qui lui est exactement superposable.

Parmi les diphosphines chirales utilisables dans la préparation des polymères de l'invention, on distingue les molécules dont la chiralité résulte de l'agencement spatial des atomes les constituant (ces molécules sont dites atropoisomères), les molécules dont la chiralité est portée par les atomes de phosphore et les molécules dont la chiralité est portée par des atomes de carbone.

Les diphosphines de type atropoisomère ne contiennent pas de carbone asymétrique. Dans ces molécules, la rotation autour de liaisons simples est empêchée ou grandement ralentie.

Des diphosphines atropoisomères particulièrement préférées sont celles ayant un corps chiral (ou squelette) répondant à la formule suivante : 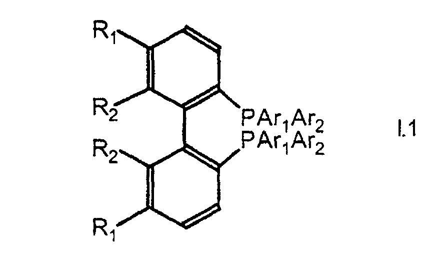 dans laquelle :
- Ar₁, Ar₂ représentent indépendamment un carbocycle saturé, insaturé ou aromatique ;
- R₁, R₂ représentent indépendamment un atome d'hydrogène ; un groupe Z ; ou un groupe -XZ où X représente O, S ou -NT ; et
- Z et T sont indépendamment choisis parmi un groupe hydrocarboné aliphatique saturé éventuellement interrompu par O, S et/ou N ; un groupe carbocyclique saturé, insaturé ou aromatique; ou un groupe hydrocarboné aliphatique saturé substitué par un ou plusieurs groupes carbocycliques saturés, insaturés ou aromatiques, dans lequel le groupe aliphatique est éventuellement interrompu par O, S et/ou N ; étant entendu que T peut représenter en outre un atome d'hydrogène ; ou bien
deux groupes R₁ et R₂ rattachés au même noyau phényle forment ensemble un carbocycle insaturé ou aromatique, ou encore forment ensemble un hétérocycle insaturé ou aromatique.

Dans le cadre de l'invention, on entend par radical carbocyclique, un radical monocyclique ou polycyclique éventuellement substitué, de préférence en C₃-C₅₀. De façon avantageuse, il s'agit d'un radical en C₃-C₁₈ de préférence mono-, bi- ou tricyclique.

Lorsque le radical carbocyclique comprend plus d'un noyau cyclique (cas des carbocycles polycycliques), les noyaux cycliques peuvent être condensés deux à deux ou rattachés deux à deux par des liaisons σ. Deux noyaux condensés peuvent être orthocondensés ou péricondensés.

Le radical carbocyclique peut comprendre une partie saturée et/ou une partie aromatique et/ou une partie insaturée.

Des exemples de radicaux carbocycliques saturés sont les groupes cycloalkyle.

De manière préférée, les groupes cycloalkyle sont en C₃-C₁₈, mieux encore en C₃-C₁₀. On peut citer notamment les radicaux cyclopentyle, cyclohexyle, cycloheptyle, cyclooctyle, adamantyle ou norbornyle.

Le carbocycle insaturé ou toute partie insaturée présente une ou plusieurs insaturations éthyléniques. Il présente de préférence de 6 à 50 atomes de carbone, mieux encore de 6 à 20, par exemple de 6 à 18.

Des exemples de carbocycles insaturés sont les groupes cycloalcényles en C₆-C₁₀.

Des exemples de radicaux carbocycliques aromatiques sont les groupes (C₆-C₁₈)aryle et notamment phényle, naphtyle, anthryle et phénanthryle.

Par groupe hydrocarboné aliphatique, on entend un groupe linéaire ou ramifié, saturé, de préférence comprenant de 1 à 25 atomes de carbone, éventuellement substitué.

Avantageusement, ledit groupe hydrocarboné aliphatique représente alkyle de 1 à 12 atomes de carbone, mieux encore de 1 à 6 atomes de carbone.

Des exemples de groupes alkyle sont les radicaux méthyle, éthyle, propyle, isopropyle, butyle, isobutyle, t-butyle, pentyle, isopentyle, néopentyle, 2-méthylbutyle, 1-éthylpropyle, hexyle, isohexyle, néohexyle, 1-méthylpentyle, 3-méthylpentyle, 1,1-diméthylbutyle, 1,3-diméthylbutyle, 2-éthylbutyle, 1-methyl-1-éthylpropyle, heptyle, 1-méthylhexyle, 1-propylbutyle, 4,4-diméthylpentyle, octyle, 1-méthylheptyle, 2-éthylhexyle, 5,5-diméthylhexyle, nonyle, décyle, 1-méthylnonyle, 3,7-diméthyloctyle et 7,7-diméthyloctyle.

Les substituants des radicaux carbocycliques (St1) peuvent être des groupes hydrocarbonés aliphatiques saturés éventuellement interrompus par O, S et/ou N ou des groupes -XZ dans lesquels X et Z sont tels que définis ci-dessus.

Les substituants des radicaux aliphatiques hydrocarbonés (St2) sont des groupes carbocycliques saturés ou insaturés, eux-mêmes éventuellement substitués par un ou plusieurs des substituants (St1 ) définis ci-dessus.

De manière préférée, Ar₁ et Ar₂ représentent indépendamment (C₃-C₈)cycloalkyle ou (C₆-C₁₈)aryle, éventuellement substitué par un ou plusieurs (C₁-C₆)alkyle et/ou (C₁-C₆)alcoxy; et R₁ et R₂ représentent indépendamment un atome d'hydrogène ; (C₃-C₈)cycloalkyle ; (C₁-C₆)alkyle ; (C₁-C₆)alcoxy ou (C₆-C₁₈)aryle, les groupes cycloalkyle et aryle étant éventuellement substitués par (C₁-C₆)alkyle et/ou (C₁-C₆)alcoxy.

Lorsque R₁ et R₂ forment ensemble un carbocycle ou un hétérocycle insaturé, celui-ci présente de préférence une seule insaturation qui est celle partagée avec le noyau phényle porteur des groupes R₁ et R₂.

Les carbocycles aromatiques que forment ensemble R₁ et R₂ sont de préférence tels que définis ci-dessus.

Les carbocycles insaturés que forment ensemble R₁ et R₂ sont mono- ou polycycliques, la définition de ces termes étant telle que proposée ci-dessus. Ces carbocycles comprennent de préférence de 6 à 50 atomes de carbone, mieux encore de 6 à 20 atomes de carbone. Des exemples en sont notamment cycloalcényle en C₆-C₁₀.

Par hétérocycle, on entend selon l'invention des radicaux mono- ou polycycliques, et notamment mono-, bi- ou tricycliques comprenant un ou plusieurs hétéroatomes choisis parmi O, S et/ou N, de préférence 1 à 4 hétéroatomes,

Lorsque l'hétérocycle est polycyclique, celui-ci peut être constitué de plusieurs monocycles condensés deux à deux (orthocondensés ou péricondensés) et/ou de plusieurs monocycles rattachés deux à deux par des liaisons σ.

De préférence, les monocycles ou le monocycle constituant l'hétérocycle, présente de 5 à 12 chaînons, mieux encore de 5 à 10 chaînons, par exemple de 5 à 6 chaînons.

Lorsque R₁ et R₂ forment un hétérocycle, celui-ci comprend une partie insaturée et/ou une partie aromatique, étant entendu que la partie insaturée comprend de préférence une seule double liaison.

Des exemples d'hétérocycles mono- ou polycycliques, insaturés ou aromatiques, sont la pyridine, le furane, le thiophène, le pyrrole, le pyrrazole, l'imidazole, le thiazole, l'isoxazole, l'isothiazole, la pyridazine, la pyrimidine, la pyrazine, les triazines, l'indolizine, l'indole, l'isoindole, le benzofurane, le benzothiophène, l'indazole, le benzimidazole, le benzothiazole, la purine, la quinoline, l'isoquinoline, la cinnoline, la phtalazine, la quinazoline, la quinoxaline, la ptéridine, les naphthyridines, le carbazole, la phénothiazine, la phénoxazine, l'acridine, la phénazine, l'oxazole, le pyrazole, l'oxadiazole, le triazole, le thiadiazole et leurs dérivés insaturés. D'autres exemples sont les dérivés insaturés de la pyrrolidine, du dioxolane, de l'imidazolidine, de la pyrazolidine, de la pipéridine, du dioxane, de la morpholine, du dithiane, de la thiomorpholine, de la pipérazine et du trithiane.

Des hétérocycles particulièrement préférés sont notamment la pyridine, le furane, le thiophène, le pyrrole, le benzofurane et le benzothiophène.

On préfère, dans le cadre de l'invention, les carbocycles et hétérocycles monocycliques ou bicycliques.

Selon l'invention, lorsque R₁ et R₂ forment ensemble carbocycle ou hétérocycle, ceux-ci peuvent être éventuellement substitués par un ou plusieurs substituants St1 tels que définis ci-dessus.

De façon avantageuse :
- Ar₁, Ar₂ représentent indépendamment un carbocycle monocyclique saturé, insaturé ou aromatique éventuellement substitué par un ou plusieurs groupes (C₁-C₆)alkyle ou (C₁-C₆)alcoxy et présentant de 3 à 8 atomes de carbone ;
- R₁ et R₂ sont indépendamment choisis parmi un atome d'hydrogène, un groupe (C₁-C₆)alkyle ou un groupe (C₁-C₆)alcoxy ; ou bien
- R₁ et R₂ forment ensemble avec les atomes de carbone qui les portent (i) un carbocycle monocyclique ou polycyclique, insaturé ou aromatique présentant de 5 à 13 atomes de carbone, ou (ii) un hétérocycle monocyclique ou polycyclique, insaturé ou aromatique présentent de 4 à 12 atomes de carbone et un ou plusieurs hétéroatomes choisis parmi O, S et N, lesdits hétérocycle et carbocycle étant éventuellement substitués par un ou plusieurs (C₁-C₆)alkyle ou (C₁-C₆)alcoxy.

Dans ce sous-groupe de composés, alkyle représente préférablement un radical hydrocarboné saturé linéaire ou ramifié en C₁-C₆ tel que méthyle, éthyle, propyle, isopropyle, butyle, isobutyle, t-butyle, pentyle, isopentyle, néopentyle, 2-méthylbutyle, 1-éthylpropyle, hexyle, isohexyle, néohexyle, 1-méthylpentyle, 3-méthylpentyle, 1,1-diméthylbutyle, 1,3-diméthylbutyle, 2-éthylbutyle, 1-méthyl-1-éthylpropyle.

De préférence, le radical alkyle comprend 1 à 4 atomes de carbone.

Le terme alcoxy désigne le radical -O-alkyle où alkyle est tel que défini ci-dessus.

Lorsque le carbocycle est insaturé, il comprend plus particulièrement 1 à 2 doubles liaisons éthyléniques.

Des exemples préférés de carbocycles sont les cycloalkyles en C₃-C₁₁, les aryles en C₆-C₁₀ et leurs dérivés plus ou moins saturés.

A titre de cycloalkyle, le cyclobutyle, le cyclopentyle, le cyclohexyle, le cycloheptyle et le cyclooctyle sont préférés, les cyclopentane et cyclohexane étant les plus avantageux.

A titre de dérivé aryle en C₆-C₁₀ préférés, on peut citer les noyaux phényle et naphtyle.

Dans la formule I.1 ci-dessus, les substituants R₁, R₂, Ar₁ et Ar₂ sont tels que le corps chiral et donc, la diphosphine, possède un axe de symétrie C₂.

Ainsi, lorsque Ar₁, Ar₂, R₁ et/ou R₂ représente un carbocycle ou un hétérocycle substitué, la position et la nature des substituants est choisie de façon à conserver une symétrie d'axe C₂ de la molécule dans son entier.

Des significations préférées des substituants Ar₁ et Ar₂ sont (C₃-C₈)cycloalkyle éventuellement substitué ou (C₅-C₆)aryle éventuellement substitué. Mieux encore, on choisit Ar₁ et Ar₂ parmi phényle éventuellement substitué par un ou plusieurs (C₁-C₆)alkyle ou (C₁-C₆)alcoxy ; ou (C₄-C₈)cycloalkyle éventuellement substitué par un ou plusieurs groupes (C₁-C₆)alkyle.

Selon un mode de réalisation particulièrement préféré, Ar₁ et Ar₂ sont indépendamment cyclohexyle, phényle ou tolyle.

Des composés de formule I.1 particulièrement avantageux sont ceux pour lesquels Ar₁ et Ar₂ sont identiques.

R₁ et R₂ peuvent former, ensemble avec les atomes de carbone qui les portent, un carbocycle ou un groupe hétérocyclique, insaturé ou aromatique.

Dans ce cas, lorsque le carbocycle, respectivement l'hétérocycle, est insaturé, on préfère qu'il présente une seule insaturation, cette insaturation étant commune au phényle portant le groupe PAr₁Ar₂ et au carbocycle, respectivement à l'hétérocycle. Et de fait, les deux carbones portant les substituants R₁ et R₂ sont des carbones de type sp₂.

L'hétérocycle formé par R₁, R₂ et les atomes de carbone portant R₁ et R₂ est de préférence un hétérocycle comprenant 1 ou 2 hétéroatomes endocycliques.

Un premier groupe de composés préférés est constitué des composés de formule I.1 pour lesquels R₁ et R₂ sont un atome d'hydrogène, un groupe (C₁-C₆)alkyle (de préférence méthyle), ou un groupe (C₁-C₆)alkoxy (de préférence méthoxy).

Un second groupe de composés préférés est constitué des composés de formule I.1 pour lesquels R₁ et R₂ forment ensemble (C₃-C₁₁)cycloalcényle éventuellement substitué, (C₆-C₁₀)aryle éventuellement substitué ou (C₄-C₈)hétéroaryle comprenant 1 ou 2 hétéroatomes endocycliques, ledit hétéroaryle étant éventuellement substitué.

En tant que radical cycloalcényle préféré, on peut mentionner les radicaux hydrocarbonés cycliques ne comportant qu'une seule insaturation éthylénique, c'est-à-dire ceux dans lesquels les seuls carbones sp₂ sont ceux portant les substituants R₁ et R₂.

Parmi ce second groupe de composés préférés, les composés pour lesquels R₁ et R₂ ensemble avec les atomes de carbone qui les portent, forment un radical phényle éventuellement substitué ou un groupe cyclohexényle à une seule insaturation, éventuellement substitué, sont particulièrement avantageux.

Des exemples du corps chiral convenant pour la diphosphine atropoisomère sont notamment les suivants :

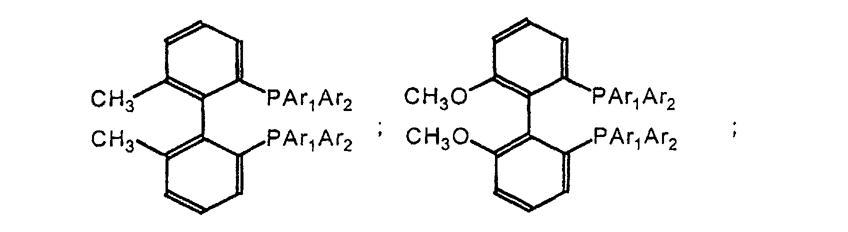

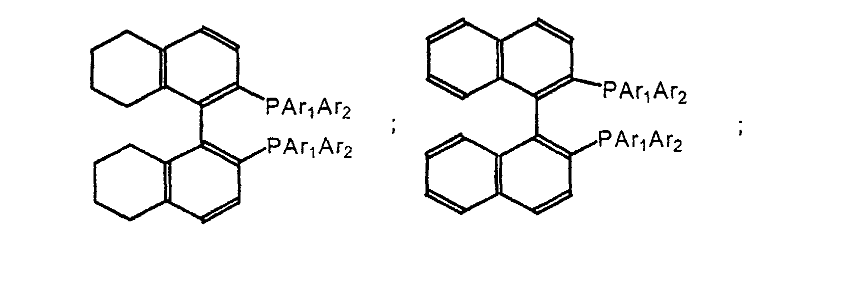

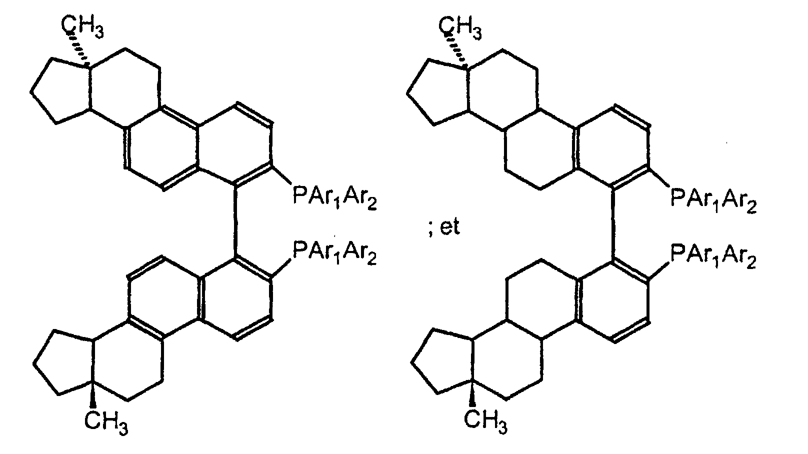

Selon un autre mode de réalisation de l'invention, la diphosphine chirale a un corps chiral répondant à la formule :

R₅R₆P-A-PR₅R₆ I.2.

dans laquelle :
- A représente un groupe hydrocarboné aliphatique divalent saturé ; un groupe carbocyclique divalent saturé ou aromatique ; ou un groupe hydrocarboné aliphatique divalent saturé interrompu par un groupe carbocyclique divalent saturé ou aromatique ;
- R₅ et R₆ sont différents et représentent un groupe hydrocarboné aliphatique saturé ; un groupe carbocyclique aromatique ou hétérocyclique aromatique.

Dans ce cas la chiralité est portée par les atomes de phosphore.

Il doit être entendu que chacun des groupes divalents représentant A peut être substitué par un ou plusieurs radicaux -XZ où X et Z sont tels que définis ci-dessus. De préférence Z représente en ce cas un groupe hydrocarboné aliphatique saturé tel que défini ci-dessus.

Les substituants des groupes hydrocarboné aliphatique et carbocyclique saturé ou aromatique sont tels qu'ils ne perturbent pas la symétrie d'axe C₂ du corps chiral.

Les groupes hydrocarbonés aliphatiques saturés, les groupes carbocycliques et hétérocycliques aromatiques représentant R₅ et R₆ sont tels que définis ci-dessus. Ils peuvent être substitués par un ou plusieurs substituants -Z ou -XZ dans lesquels X et Z sont tels que définis ci-dessus.

Un groupe carbocyclique divalent saturé préféré est un radical cycloalkylène mono- ou polycyclique.

Par cycloalkylène, on entend un radical bivalent correspondant à un cycloalkane dans lequel deux atomes d'hydrogène ont été remplacés par deux liaisons. Un cycloalkylène préféré est monocyclique et répond à la formule : 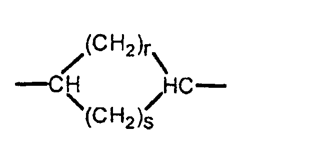 où r et s sont des nombres entiers de 0 à 4 où r + s ≥ 1. Des exemples particulièrement préférés en sont les radicaux cyclohexylène et cyclobutylène, tels que 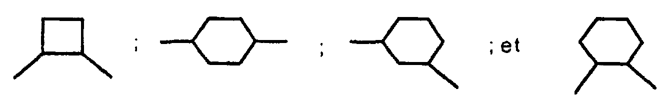 le radical cyclohexylène étant le plus avantageux.

Un groupe carbocyclique divalent aromatique préféré est un radical arylène mono- ou polycyclique.

Par arylène, on entend un radical bivalent correspondant à un groupe aryle dans lequel deux des atomes d'hydrogène ont été remplacés par deux liaisons. De manière préférée, arylène représente phényfène et notamment ortho-phénylène et para-phénylène ou un radical naphtylène tel que :

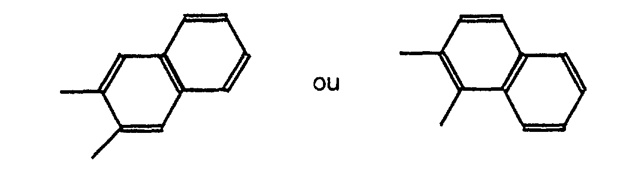

De façon avantageuse, lorsque R₅ et/ou R₆ représente un hétérocycle aromatique monocyclique, celui-ci comprend préférablement 1 à 2 hétéroatomes endocycliques. Des exemples en sont furyle, thiényle, imidazolyle, pyridyle ou pyrrolyle.

Lorsque R₅ et/ou R₆ représente (C₆-C₁₀)aryle, on préfère les significations phényle ou naphtyle, mieux encore phényle.

Lorsque R₅ et/ou R₆ représentent alkyle, celui-ci est linéaire ou ramifié et présente de préférence de 1 à 4 atomes de carbone. Très préférablement, alkyle représente en ce cas méthyle.

De façon avantageuse :
- A représente une chaîne alkylène en C₁-C₆ éventuellement substituée par un ou plusieurs groupes (C₁-C₆)alcoxy, di(C₁-C₆)alkylamino ou (C₁-C₆)alkylthio ; un groupe (C₃-C₈)cycloalkylène éventuellement substitué par un ou plusieurs groupes (C₁-C₆)alcoxy, di(C₁-C₆)alkylamino ou (C₁-C₆)alkylthio ; un groupe (C₆-C₁₀)arylène éventuellement substitué par un ou plusieurs groupes (C₁-C₆)alcoxy, di(C₁-C₆)alkylamino ou (C₁-C₆)alkylthio ; ou un groupe -(CH₂)ⱼ-B"-(CH₂)ⱼ- où j représente un nombre entier de 1 à 3 et B" représente (C₃-C₈)cycloalkylène éventuellement substitué par un ou plusieurs (C₁-C₆)alcoxy, di(C₁-C₆)alkylamino ou (C₁-C₆)alkylthio ou (C₆-C₁₀)arylène éventuellement substitué par un ou plusieurs (C₁-C₆)alcoxy, di(C₁-C₆)alkylamino ou (C₁-C₆)alkylthio ;
- R₅ et R₆ sont différents et représentent un hétérocycle monocyclique aromatique présentant de 3 à 7 atomes de carbone et un ou plusieurs hétéroatomes choisis parmi O, N et S ; un groupe (C₆-C₁₀)aryle, ledit hétérocycle et ledit groupe aryle étant éventuellement substitués par un ou plusieurs groupes (C₁-C₆)alkyle ou (C₁-C₆)alcoxy ; ou bien (C₁-C₆)alkyle éventuellement substitué par un ou plusieurs (C₁-C₆)alcoxy.

De manière préférée alkylène représente une chaîne hydrocarbonée ramifiée ou linéaire comprenant préférablement 1 à 4 atomes de carbone.

Une signification préférée de A est -(CH₂)ₙ- où n est compris entre 1 et 4, par exemple -CH₂- et plus particulièrement la chaîne éthylène de formule -CH₂-CH₂-.

L'introduction de substituants au niveau des groupes R₅ et R₆ doit être modulée par l'homme du métier de façon à ce que la symétrie d'axe C₂ soit conservée.

Un corps chiral répondant à la formule I.2 est, par exemple :

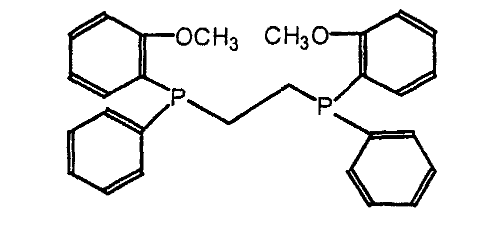

Selon encore un autre mode de réalisation de l'invention, le corps chiral de la diphosphine a pour structure : 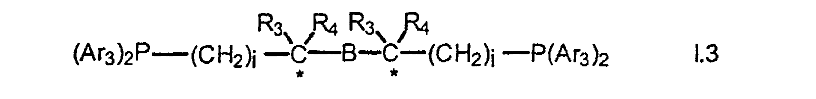 dans laquelle :
- * désigne un centre asymétrique ;
- i représente 0 ou 1 ;
- R₃ et R₄ représentent indépendamment un atome d'hydrogène ou un groupe hydrocarboné aliphatique saturé, ou bien encore les radicaux R₄ sont tels que définis ci-dessus et les radicaux R₃ forment ensemble une chaîne hydrocarbonée aliphatique divalente saturée éventuellement interrompue par deux groupes X, X étant tels que définis ci-dessus, de préférence par deux groupes X identiques ;
- B représente une liaison ou bien est tel que défini ci-dessus pour A à la formule I.2;
- Ar₃ est tel que défini ci-dessus pour R₅ et R₆, à la formule I.2.

Dans le cas du corps chiral de formule I.3, la chiralité est portée par deux atomes de carbone de la chaîne reliant les deux atomes de phosphore.

Selon ce mode de réalisation, le groupe hydrocarboné aliphatique saturé est tel que défini ci-dessus et est non substitué.

La chaîne hydrocarbonée divalente saturée est telle que définie pour A ci-dessus. Elle est de préférence interrompue par deux atomes d'oxygène, ou par deux atomes de soufre.

De manière préférée :
- R₃ et R₄ sont indépendamment choisis parmi un atome d'hydrogène et un groupe (C₁-C₆)alkyle ; ou bien les deux groupes R₃ forment ensemble une chaîne (C₁-C₆)alkylène éventuellement interrompue par deux atomes d'oxygène ou de soufre, et R₄ est tel que défini ci-dessus ; et
- Ar₃ représente un hétérocycle monocyclique aromatique présentant de 3 à 7 atomes de carbone et un ou plusieurs hétéroatomes choisis parmi O, N et S ; un groupe (C₆-C₁₀)aryle, ledit hétérocycle et ledit groupe aryle étant éventuellement substitués par un ou plusieurs groupes (C₁-C₆)alkyle ou (C₁-C₆)alcoxy ; ou bien (C₁-C₆)alkyle éventuellement substitué par un ou plusieurs (C₁-C₆)alcoxy.

Les groupes alkyle et alkylène de la formule I.3 sont linéaires ou ramifiés et présentent de préférence 1 à 4 atomes de carbone.

Lorsque B comprend un radical cycloalkylène ou arylène, ceux-ci sont tels que définis ci-dessus pour la formule I.2.

Des significations particulièrement préférées de B sont une liaison ; un groupe (C₁-C₆)alkylène; un groupe (C₆-C₈)cycloalkylène ; ou bien une chaîne -(CH₂)ₚ-B'-(CH₂)_{q}- où p et q représentent indépendamment un nombre entier compris entre 1 et 3 et B' représente (C₆-C₈)cycloalkylène.

Un premier groupe de composés préférés de formule I.3 est constitué des composés pour lesquels R₃ et R₄ représentent un atome d'hydrogène ou un groupe méthyle, éthyle, n-propyle ou isopropyle.

Pour un second groupe de composés préférés, les deux groupes R₃ forment ensemble une chaîne alkylène. auquel cas les groupes R₄ représentent idéalement un atome d'hydrogène.

De manière particulièrement avantageuse, le groupe B est choisie parmi : une liaison ; -CH₂- ; -CH₂-CH₂- ;

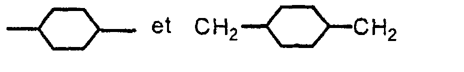

Concernant Ar₃, sa définition correspond à celles de R₅ et R₆ de la formule I.2. L'homme du métier sélectionnera les significations préférées de Ar₃ parmi celles préférées dans le cas des substituants R₅ et R₆.

Des exemples de corps chiral répondant à la formule I.3 sont les suivants: 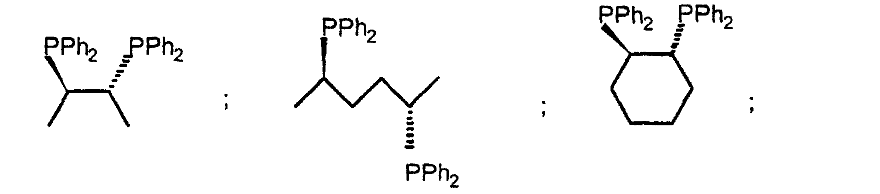 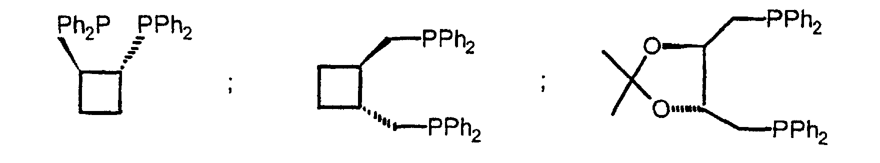 où Ph représente phényle,
ou l'une des formes énantiomères de ces structures.

De façon préférée, le corps chiral peut encore répondre à la formule I.4 suivante : 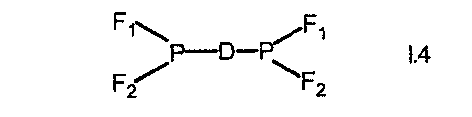 dans laquelle :
- D est tel que défini ci-dessus pour A à la formule I.2 ;
- F₁ et F₂ sont identiques et représentent un groupe hydrocarboné aliphatique saturé, ledit groupe portant au moins un centre chiral, un groupe carbocyclique saturé portant au moins un centre chiral ; ou bien
- F₁ et F₂ forment ensemble une chaîne hydrocarbonée aliphatique divalente saturée. éventuellement interrompue par deux groupes X, X étant tels que définis ci-dessus, deux des carbones de ladite chaîne constituant des centres asymétriques.

Dans le cas des composés de formule I.4, la chiralité est portée par des atomes de carbone des groupes F₁ et F₂.

Les substituants des groupes divalents représentant D sont tels qu'ils ne perturbent pas la symétrie d'axe C₂ du corps chiral.

Lorsque F₁ et/ou F₂ représentent un groupe hydrocarboné aliphatique saturé ou un groupe carbocyclique saturé, ceux-ci sont tels que définis ci-dessus et sont éventuellement substitués par un ou plusieurs groupes -XZ dans lesquels X et Z sont tels que définis ci-dessus. De préférence Z est en ce cas un groupe hydrocarboné aliphatique saturé tel que défini ci-dessus.

Lorsque F₁ et F₂ représentent ensemble une chaîne hydrocarbonée aliphatique divalente interrompue par deux groupes X, ceux-ci sont de préférence choisis parmi O et S.

De manière préférée :
- D représente une chaîne alkylène en C₁-C₆ éventuellement substituée par un ou plusieurs groupes (C₁-C₆)alcoxy, di (C₁-C₆)alkylamino ou (C₁-C₆)alkylthio ; un groupe (C₃-C₈)cycloalkylène éventuellement substitué par un ou plusieurs groupes (C₁-C₆)alcoxy, di(C₁-C₆)alkylamino ou (C₁-C₆)alkylthio ; un groupe (C₆-C₁₀)arylène éventuellement substitué par un ou plusieurs groupes (C₁-C₆)alcoxy, di(C₁-C₆)alkylamino ou (C₁-C₆)alkylthio ; ou un groupe -(CH₂)ⱼ-B"-(CH₂)ⱼ- où j et B" sont tels que définis ci-dessus pour la formule I.2 ;
- F₁ et F₂ sont identiques et représentent (C₁-C₆)alkyle éventuellement substitué par un ou plusieurs (C₁-C₆)alcoxy, ledit groupe alkyle portant au moins un centre chiral ; (C₃-C₈)cycloalkyle substitué par un ou plusieurs (C₁-C₆)alcoxy ou (C₁-C₆)alkyle, ledit cycloalkyle portant au moins un centre chiral ; ou bien F₁ et F₂ forment ensemble une chaîne (C₁-C₆)alkylène éventuellement interrompue par deux atomes d'oxygène ou de soufre, ladite chaîne étant substituée par un ou plusieurs groupes (C₁-C₆)alkyle ou (C₁-C₆)alcoxy, deux des carbones de ladite chaîne constituant des centres asymétriques.

Les chaînes alkylène et les radicaux alkyle des groupes alkyle, alcoxy, alkylthio et dialkylamino comprennent préférablement 1 à 4 atomes de carbone.

Lorsque D comprend cycloalkylène ou arylène, ceux-ci sont tels que définis ci-dessus pour la formule I.2.

Les substituants des groupes alkylène, cycloalkylène et arylène sont tels qu'ils ne perturbent pas la symétrie d'axe C₂ du corps chiral.

De façon particulièrement avantageuse, F₁ et F₂, ensemble avec l'atome de phosphore qui les porte, forment un cycle à cinq ou à six sommets, substitué par deux radicaux identiques choisis parmi (C₁-C₆)alkyle et (C₁-C₆)alcoxy. De préférence, le cycle à cinq ou six sommets est un hétérocycle comprenant l'atome de phosphore comme seul hétéroatome.

Une signification également préférée de F₁ et F₂ est le radical menthyle, et notamment le radical 2-isopropyl-5-méthyl-cyclohexyloxy.

De façon avantageuse, le corps chiral de formule I.4 a pour structure

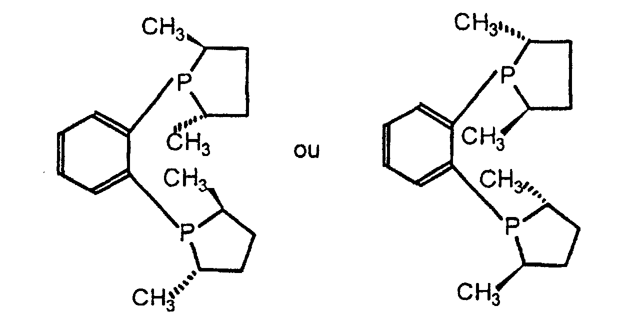

Selon un autre mode de réalisation de l'invention, le corps chiral a l'une des formules I.5 ou I.6 suivante :

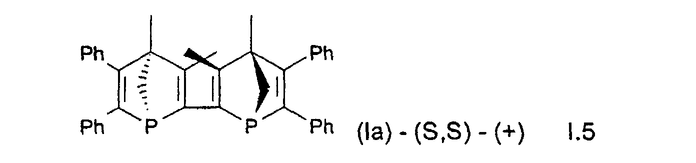

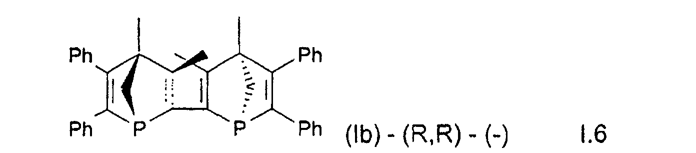

Ces composés et leurs méthodes de préparation sont notamment décrits dans la demande R 94 167.

Selon encore un autre mode de réalisation, le corps chiral a pour formule, la formule I.7 : 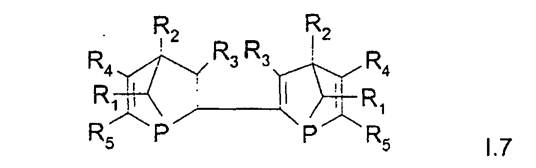 dans laquelle
- R₁, R₂, R₃, R₄, R₅, identiques ou différents, représentent un atome d'hydrogène ou un radical hydrocarboné, éventuellement substitué, ayant de 1 à 40 atomes de carbone qui peut être un radical aliphatique acyclique saturé ou insaturé, linéaire ou ramifié ; un radical carbocyclique ou hétérocyclique saturé, insaturé ou aromatique, monocyclique ou polycyclique ; un radical aliphatique saturé ou insaturé, linéaire ou ramifié, porteur d'un substituant cyclique,
- R₂ et R₃ peuvent former ensemble avec les atomes de carbone qui les portent, un cycle saturé ou insaturé,
- R₅ peut représenter un radical de type 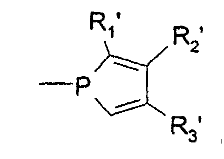 dans lequel R₁', R₂' et R₃' ont la même signification que celle donnée pour R₁, R₂ et R₃,
- R₄ et R₅ ne peuvent pas représenter simultanément un groupe phényle.

Ces composés et leus méthodes de préparation sont décrits dans la demande R 97 014.

Des exemples de diphosphines répondant à I.7 ci-dessus sont plus précisément les suivantes :

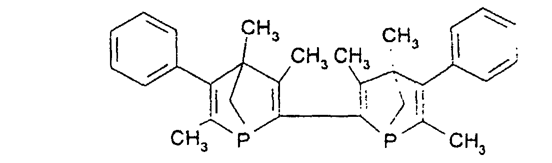

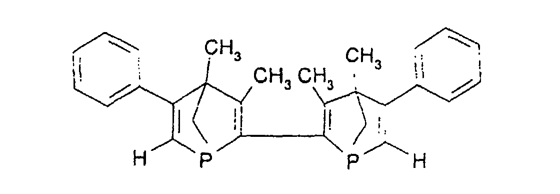

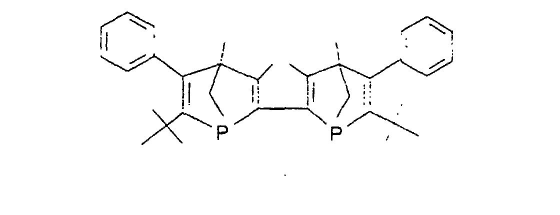

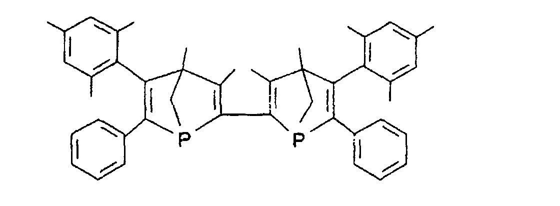

Les formules I.1 à I.7 ci-dessus sont données à titre illustratif mais l'invention n'est pas limitée à ces structures particulières pour le corps chiral de la diphosphine.

Il est essentiel selon l'invention que le corps chiral de la diphosphine porte deux fonctions capables de réagir avec les monomères polymérisables.

L'identicité de ces deux fonctions est nécessaire pour assurer la symétrie d'axe C₂ de la molécule. La nature de ces fonctions n'est pas critique selon l'invention.

Le choix de ces fonctions, combiné au choix des monomères polymérisables, détermine la nature du polymère résultant.

Des exemples de groupes fonctionnels sont les groupes amino, halogène, hydroxy, thiol, carboxy, isocyanate et thioisocyanate.

La présente invention englobe tous les types de polymères et notamment les polymères linéaires tels que les polyester, polyamide, polyurée, polythiourée, polyimide, polycarbonate, polytéréphtalate et polysulfone.

Bien que l'invention n'entende pas s'y limiter spécifiquement, les polyamides, polyurées, polythiourées et polyimides, vont faire l'objet d'une description plus détaillée.

Selon l'invention, on préfère les polymères résultant de la polymérisation d'une diphosphine chirale avec un seul monomère polymérisable.

Les polyurées, polyamides, polythiourées et polyimides de l'invention peuvent être préparés au départ d'une diphosphine chirale constitué d'un corps chiral portant, à titre de groupes fonctionnels, deux groupes amino ou deux groupes aminométhyle. L'incorporation de ces groupes amino ou aminométhyle peut être réalisée de façon quelconque, la méthode utilisée pour ce faire n'étant pas cruciale selon l'invention.

A titre d'illustration, la préparation des diphosphines de formule II : 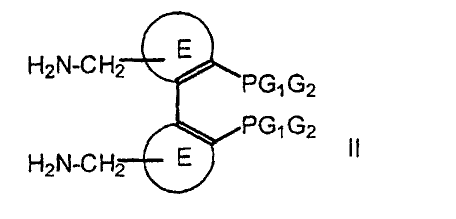 dans laquelle :
- E représente phényle ou naphtyle ; et
- G₁ et G₂ représentent indépendamment un groupe carbocyclique saturé ou aromatique,
est décrite ci-dessous.

Selon cette définition, phényle et naphtyle sont éventuellement substitués.

Les radicaux carbocycliques sont généralement tels que définis ci-dessus. Ils peuvent être substitués.

Les radicaux carbocycliques présentent, dans le cas de G₁ et/ou G₂, une partie saturée et/ou aromatique.

Les substituants des radicaux phényle, naphtyle et carbocycliques de la formule Il ci-dessus sont inertes dans les conditions de mise en oeuvre du procédé de préparation des composés Il.

De façon préférée, ces substituants sont alkyle ou alcoxy (par exemple en C₁-C₂₅, C₁-C₁₂ou C₁-C₆).

De façon avantageuse :
- E représente phényle ou naphtyle, éventuellement substitué par un ou plusieurs radicaux choisis parmi (C₁-C₆)alkyle et (C₁-C₆)alcoxy ; et
- G₁, G₂ représentent indépendamment un groupe phényle éventuellement substitué par un ou plusieurs (C₁-C₆)alkyle ou (C₁-C₆)alcoxy ; ou un groupe (C₄-C₈)cycloalkyle éventuellement substitué par un ou plusieurs groupes (C₁-C₆)alkyle,
est décrite ci-dessous.

Le composé de formule II est facilement obtenu par réduction, au moyen d'un agent réducteur, du nitrile correspondant de formule III ci-dessous : 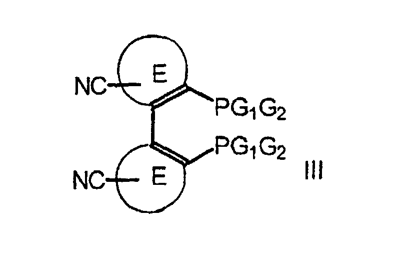 où G₁, G₂ et E sont tels que définis ci-dessus.

Un agent réducteur approprié est l'hydrure de lithium et aluminium (AlLiH₄).

La réaction est préférablement mise en oeuvre dans un solvant ou un mélange de solvants.

Lorsque l'agent réducteur est AlLiH₄, le solvant comprend de façon avantageuse un ou plusieurs hydrocarbures aromatiques (tels que le benzène, le toluène et le xylène) en mélange avec un ou plusieurs éthers.

A titre d'éther, on peut citer les éthers d'alkyle en C₁-C₆ (éther diéthylique et diisopropylique), les éthers cycliques (dioxane, tétrahydrofurane), le diméthoxyéthane et l'éther diméthylique de diéthylèneglycol.

Lorsque l'agent réducteur est AlLiH₄, on optera plus préférablement pour un mélange de toluène et de tétrahydrofurane dans des proportions variant entre (v/v) 70-50/30-50 : toluène/tétrahydrofurane (par exemple 60/40 : toluène/THF).

La réduction pourra être conduite à une température comprise entre 20° C et 100° C, de préférence entre 40° C et 80° C.

Habituellement, on utilise un large excès de l'agent réducteur. Ainsi, le rapport molaire de l'agent réducteur au composé de formule III varie généralement entre 1 et 30, par exemple entre 2 et 20, notamment entre 5 et 18.

La concentration des réactifs dans le milieu est variable ; elle pourra être maintenue entre 0,005 et 1 mol/l.

Les nitriles de formule III peuvent être préparés simplement par mise en oeuvre des étapes i) à iv) suivantes :
i) on réalise tout d'abord la bromation d'un diol de formule IV : 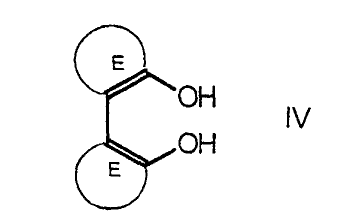 dans laquelle E est tel que défini ci-dessus, au moyen d'un agent de bromation approprié de façon à obtenir un composé dibromé de formule V : 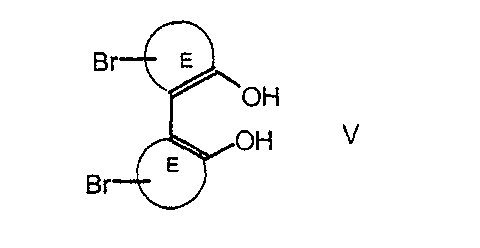 dans laquelle E est tel que défini ci-dessus ;
ii) on estérifie le composé de formule V obtenu à l'étape précédente par action d'un acide sulfonique ou d'une forme activée de celui-ci de façon à obtenir le disulfonate correspondant ;
iii) on réalise ensuite la substitution des deux atomes de brome par des groupes cyano par réaction du disulfonate obtenu à l'étape précédente avec un agent nucléophile approprié de façon à obtenir le nitrile correspondant ;
iv) couplage d'une phosphine de formule VI :

   X'PG₁G₂ VI
dans laquelle X' représente un atome d'hydrogène ou un atome d'halogène et G₁ et G₂ sont tels que définis ci-dessus, avec le nitrile obtenu à l'étape précédente en présence d'un catalyseur à base d'un métal de transition, de façon à obtenir le composé de formule III attendu.

A l'étape (i) le noyau phényle, respectivement naphtyle, du diol de formule IV, est bromé par action d'un agent de bromation approprié.

Les groupes hydroxyles présents sur les noyaux naphtyle, respectivement phényle orientent la réaction électrophile de telle sorte que la position des atomes de brome sur ces noyaux est bien déterminée.

Lorsque E est un noyau phényle non substitué ou portant en position méta par rapport au groupe OH un substituant tel que (C₁-C₆)alkyle ou (C₁-C₆)alcoxy, le diol correspondant de formule IVa : 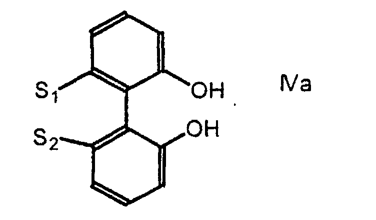 où S₁ et S₂ sont indépendamment des substituants inertes, notamment choisis parmi un atome d'hydrogène, un groupe alkyle ou alcoxy, de préférence en C₁-C₆,
conduit au composé bromé correspondant de formule Va :

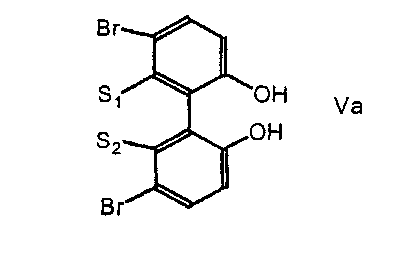

Lorsque E est un noyau naphtyle, la bromation du diol correspondant de formule IVb : 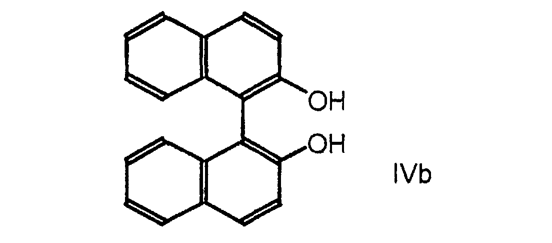 conduit au composé Vb suivant :

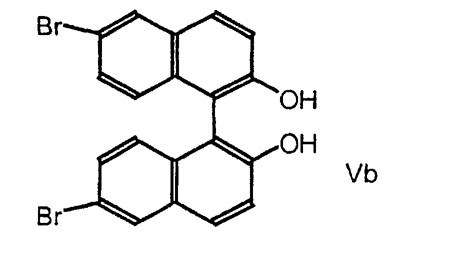

La réaction de bromation de noyaux phényle ou naphtyle est une réaction électrophile qui est facilement réalisée par action de Br₂ sur le diol correspondant.

Cette réaction peut être mise en oeuvre en présence d'un catalyseur tel qu'un acide de Lewis et notamment le chlorure de fer. Cependant, dans la mesure où les groupes hydroxyle présents sur les noyaux phényle et naphtyle activent ces noyaux, la bromation est facilement réalisée en l'absence de tout catalyseur.

Les diols de formule IV sont tellement réactifs qu'il est souhaitable d'effectuer la bromation à basse température, par exemple entre -78° et -30° C, de préférence entre -78° C et -50° C.

Selon un mode de réalisation préféré de l'invention, la bromation a lieu dans un solvant aprotique inerte tel qu'un hydrocarbure aromatique (par exemple chlorobenzène et dichlorobenzène) ; un hydrocarbure aromatique nitré tel qu'un nitrobenzène ; un hydrocarbure aliphatique éventuellement halogéné tel que hexane, heptane, chlorure de méthylène, tétrachlorure de carbone ou dichloroéthane ; ou un hydrocarbure aliphatique.

De manière générale, les hydrocarbures aromatiques présentant des noyaux aromatiques appauvris en électrons, c'est-à-dire portant un ou plusieurs substituants électroattracteurs, peuvent être utilisés.

A titre de solvant préféré, on peut citer les hydrocarbures aliphatiques halogénés et notamment le chlorure de méthylène.

En variante, il est possible d'opérer dans l'acide acétique glacial comme solvant. Dans ces conditions, on ajoute généralement goutte à goutte une solution du brome dans l'acide acétique à une solution du diol IV dans l'acide acétique.

Que l'on opère en présence d'acide acétique ou non, on utilise un excès de l'agent de bromation par rapport au diol IV.

De manière préférée, le rapport molaire de l'agent de bromation au diol IV varie entre 2 et 5, mieux encore entre 2 et 3.

Lorsqu'on travaille en solution, la concentration des réactifs peut varier très largement entre 0,01 et 10 mol/l, par exemple entre 0,05 et 1 mol/l.

A l'étape (ii), les fonctions hydroxyle du diol V sont estérifiées par action d'un acide sulfonique ou d'une forme activée de celui-ci, de façon à obtenir le disulfonate correspondant.

Selon l'invention, la nature de l'acide sulfonique utilisé n'est pas déterminante en soi.

De manière avantageuse, l'acide sulfonique a pour formule :

P-SO₂-OH

où P représente un groupe aliphatique hydrocarboné ; un groupe carbocyclique aromatique ; ou un groupe aliphatique substitué par un groupe carbocyclique aromatique.

Par groupe aliphatique hydrocarboné, on entend notamment un groupe alkyle tel que défini ci-dessus, éventuellement substitué. La nature du substituant est telle que celui-ci ne réagit pas dans les conditions de la réaction d'estérification. Un exemple préféré de substituant de groupe alkyle est un atome d'halogène tel que fluor, chlore, brome ou iode.

Par groupe carbocyclique aromatique, on entend les groupes aromatiques mono- ou polycycliques et notamment les groupes mono-, bi- ou tricycliques définis ci-dessus et par exemple, phényle, naphtyle, anthryle ou phénanthryle.

Le groupe carbocyclique aromatique est éventuellement substitué. La nature du substituant n'est pas critique dès lors que celui-ci ne réagit pas dans les conditions de l'estérification. De façon avantageuse, le substituant est alkyle éventuellement halogéné, alkyle étant tel que défini ci-dessus et halogène représentant chlore, fluor, brome ou iode et, de préférence chlore. A titre d'exemple, alkyle éventuellement halogéné, désigne alkyle perfluoré tel que trifluorométhyle ou pentafluoroéthyle.

Selon un mode de réalisation préféré de l'invention,

P représente (C₆-C₁₀)aryle éventuellement substitué par un ou plusieurs (C₁-C₆)alkyle éventuellement halogéné ; (C₁-C₆)alkyle éventuellement halogéné ; ou (C₆-C₁₀)aryle-(C₁-C₆)alkyle dans lequel le groupe aryle est éventuellement substitué par un ou plusieurs (C₁-C₆)alkyle éventuellement halogéné et le groupe alkyle est éventuellement halogéné.

Des exemples appropriés de tels acides sulfoniques sont l'acide paratoluènesulfonique, l'acide méthanesulfonique et l'acide trifluorométhanesulfonique, ce dernier étant plus particulièrement préféré.

Selon un mode de réalisation préféré de l'invention, on utilise un dérivé activé de l'acide sulfonique. Par dérivé activé, on désigne un acide sulfonique dans lequel la fonction acide -SO₃H est activée, par exemple par formation d'une liaison anhydride ou du groupe -SO₂Cl.

Un dérivé d'acide sulfonique particulièrement avantageux est l'anhydride symétrique de l'acide trifluorométhanesulfonique, de formule (CF₃-SO₂)₂O.

Lorsque l'acide sulfonique utilisé a la formule P-SO₃H ci-dessus ou est une forme activée de cet acide, le disulfonate obtenu à l'issue de l'étape ii) répond à la formule VII : 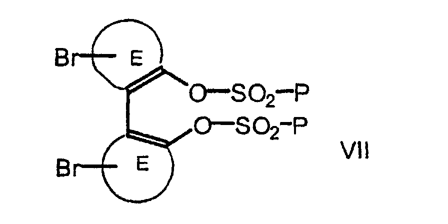 dans laquelle E et P sont tels que définis ci-dessus.

Les conditions de la réaction d'estérification seront facilement mises au point par l'homme du métier. Celles-ci dépendant notamment de la nature de l'agent d'estérification. Lorsque l'agent d'estérification est un acide sulfonique, une température de réaction plus élevée, comprise entre 20 et 100° C, peut s'avérer nécessaire. A l'inverse, au départ d'une forme activée de cet acide, telle qu'un anhydride ou un chlorure de sulfonyle, une température plus basse peut convenir. Généralement, une température comprise entre -30° C et 50° C, de préférence entre -15 et 20° C, peut en ce cas suffire.

L'estérification est préférablement mise en oeuvre dans un solvant. Les solvants appropriés sont notamment les hydrocarbures aliphatiques, aromatiques ou cycliques éventuellement halogénés, tels que ceux définis ci-dessus. On peut citer le tétrachlorure de carbone et le dichlorométhane. Le dichlorométhane est particulièrement préféré. Les éthers sont également utilisables comme solvant. On citera par exemple les éthers de dialkyle en C₁-C₆ (éther diéthylique et éther diisopropylique), les éthers cycliques (tétrahydrofuranne et dioxanne), le diméthoxyéthane et l'éther diméthylique du diéthylèneglycol.

Lorsque l'agent d'estérification est autre que l'acide trifluorométhanesulfonique, il est souhaitable d'introduire une base dans le milieu réactionnel. Des exemples de base sont la N-méthylmorpholine, la triéthylamine, la tributylamine, la diisopropyléthylamine, la dicyclohexylamine, la N-méthylpipéridine, la pyridine, la 2,6-diméthylpyridine, la 4-(1-pyrrolidinyl)pyridine, la picoline, la 4-(N,N-diméthylamino)pyridine, la 2,6-di-t-butyl-4-méthylpyridine, la quinoléine, la N,N-diméthylaniline et la N,N-diéthylaniline.

Comme bases préférées, on retiendra essentiellement la pyridine et la 4-diméthylaminopyridine.

La réaction peut également être réalisée dans un mélange biphasique d'eau et d'un solvant organique tel qu'un hydrocarbure aliphatique halogéné (par exemple le tétrachlorure de carbone). Dans ce cas, il est préférable d'utiliser un agent d'estérification sous forme d'anhydride et d'opérer en présence d'une base soluble dans l'eau telle que KOH, NaOH ou K₂CO₃, de préférence KOH.

La réaction de l'acide sulfonique ou de son dérivé activé sur le diol bromé V est stoechiométrique. Néanmoins, il est préférable d'opérer en présence d'un excès de l'acide ou de sa forme activée. Ainsi, un rapport de l'acide éventuellement sous forme activée, au diol V compris entre 2 et 5, mieux encore entre 2 et 3, est-il recommandé.

Lorsque la réaction est réalisée en solution, la concentration des réactifs, qui n'est pas un paramètre critique selon l'invention, pourra varier entre 0,1 et 10 mol/l, avantageusement entre 1 et 5 mol/l.

L'homme du métier pourra s'inspirer des conditions opératoires illustrées dans J. Org. Chem., vol. 58, n° 7, 1993, 1945-1948 et Tetrahedron Letters, vol. 31, n° 7, 985-988, 1990 pour la mise en oeuvre de l'estérification.

L'étape suivante (iii) est une substitution nucléophile. Les deux atomes de brome portés par les noyaux E sont déplacés par des groupes cyano par action d'un agent nucléophile approprié.

De façon à réaliser cette substitution, l'homme du métier pourra utiliser l'une quelconque des méthodes connues dans la technique.

Selon un mode de réalisation préféré de l'invention, l'agent nucléophile utilisé est le cyanure de cuivre.

Le rapport molaire du cyanure de cuivre au composé VI est de préférence supérieur à 2, il peut varier avantageusement entre 2 et 4, préférablement entre 2 et 3.

La réaction est de préférence mise en oeuvre dans un solvant. Comme exemple de solvants, on peut citer les amides tels que le formamide, le diméthylformamide, le diméthylacétamide, la N-méthyl-2-pyrrolidinone et l'hexaméthylphosphorylamide. Le diméthylformamide est nettement préféré. La pyridine est également un solvant approprié. La température de la réaction est maintenue avantageusement entre 50 et 200° C, mieux encore entre 80 et 180° C.

La concentration des réactifs dans le milieu réactionnel oscille généralement entre 0,1 et 10 mol/l, par exemple entre 2 et 7 mol/l.

L'isolement du nitrile implique la décomposition du complexe intermédiaire formé et le piégeage de l'excès de cyanure.

L'hydrolyse du complexe intermédiaire pourra être réalisée soit par action de chlorure de fer hydraté, soit par action d'éthylènediamine aqueux.

Dans le premier cas, on verse le milieu réactionnel dans une solution aqueuse de chlorure de fer à 50-80 % (g/ml) contenant de l'acide chlorhydrique concentré. La solution résultante est chauffée à 40-80° C jusqu'à décomposition complète du complexe. Puis le milieu est décanté et extrait de façon conventionnelle.

Dans le second cas, le milieu réactionnel est versé dans une solution aqueuse d'éthylènediamine (éthylènediamine/eau : 1/5 - 1/1 (v/v), par exemple 1/3) puis l'ensemble est agité vigoureusement. Le milieu est alors décanté et extrait de façon connue en soi.

L'homme du métier pourra s'inspirer des travaux de L. Friedman et al. publiés dans J.O.C. 1961, 26, 1522, pour isoler le nitrile.

Au départ du disulfonate de formule VII mentionné ci-dessus, on obtient à l'issue de cette étape le nitrile de formule VIII : 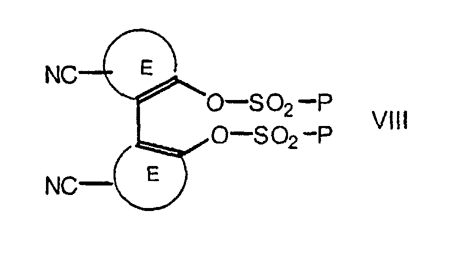 dans laquelle E et P sont tels que définis ci-dessus et la position du groupe cyano sur le noyau E est la même que celle du brome dans le composé VII.

A l'étape (iv), on opère à un couplage croisé d'une phosphine de formule VI :

X'PG₁G₂ VI

dans laquelle X' est un atome d'halogène ou d'hydrogène et G₁, G₂ sont tels que définis ci-dessus avec le nitrile obtenu à l'étape précédente, en présence d'un catalyseur à base d'un métal de transition.

Ce couplage conduit directement au composé attendu de formule III.

Des exemples de catalyseurs appropriés sont des catalyseurs à base de nickel, de palladium, de rhodium, de ruthénium, de platine ou d'un mélange de ces métaux.

Les catalyseurs préférés sont les catalyseurs à base de nickel tels que ceux choisis parmi NiCl₂ ; NiBr₂ ; NiCl₂(dppp) ; NiCl₂(dppb) ; NiCl₂(dppf) ; NiCl₂ (dppe) ; NiCl₂(PPh₃)₂ ; Ni(CO)₂(PPh₃)₂ ; Ni(PPh₃)₄ et Ni[P(PhO)₃]₄ où dppe signifie (diphénylphosphino)éthane, dppp signifie (diphénylphosphino)propane, dppb signifie (diphénylphosphino)butane, et dppf signifie (diphénylphosphino)ferrocényl.

Parmi ces catalyseurs, on préfère NiCl₂(dppe).

La réaction est généralement mise en oeuvre à une température de 50 à 200° C, de préférence de 80 à 130° C.

Le rapport molaire du composé VI au nitrile est d'au moins 2. Il varie généralement entre 2 et 4, par exemple entre 2 et 3.

La quantité de catalyseur est de préférence telle que le rapport molaire du composé de formule VII au catalyseur varie entre 5 et 100, notamment entre 5 et 80.

La réaction est de préférence réalisée dans un solvant aprotique polaire et notamment un amide tel que ceux mentionnés ci-dessus. Là encore, le N,N-diméthylformamide est préféré. D'autres types de solvants polaires sont néanmoins utilisables tels que les (C₁-C₆)alcanols (éthanol), les hydrocarbures aromatiques (toluène, xylène et benzène), les éthers (dioxane) et l'acétonitrile.

Les conditions réactionnelles précises dépendent de la nature du composé de formule VI mis en jeu dans la réaction.

Lorsque le composé VI est HPG₁G₂, la réaction est avantageusement réalisée en présence d'une base.

Des bases convenant particulièrement bien sont la pyridine, la 4-diméthylaminopyridine, la 2,6-di-tertbutylpyridine, le 1,8-diazabicyclo[5.4.0]undec-7-ène (DBU), le 1,5-diazabicyclo[4.3.0]non-5-ène (DBN) et le 1,4-diazabicyclo[2.2.2]octane (DABCO ou triéthylènediamine). On utilisera avantageusement le DABCO comme base. En ce cas, on préfère que le rapport molaire du nitrile au catalyseur soit compris entre 5 et 20, par exemple entre 7 et 15.

Lorsque le composé de formule VI est halPG₁G₂ où hal est un atome d'halogène, de préférence Cl ou Br (mieux encore CI), il est nécessaire d'ajouter du zinc au milieu réactionnel.

La quantité de zinc est préférablement telle que le rapport molaire du zinc à halPG₁G₂ varie entre 1 et 2, de préférence entre 1,2 et 1,7.

Dans ce cas, il est souhaitable de refroidir le mélange réactionnel contenant le solvant, le nitrile et le composé VI à une température comprise entre -10 et 20° C pendant toute l'addition du zinc au milieu réactionnel. Puis, la réaction a lieu par chauffage à une température appropriée comprise entre 50 et 200° C.

Lorsque le composé de formule VI est halPG₁G₂, on préfère que le rapport molaire du nitrile au catalyseur soit compris entre 40 et 80, par exemple entre 50 et 70.

Pour plus de précisions sur la mise en oeuvre de ces réactions de couplage, l'homme du métier se rapportera à D. Cai et al. J.O.C. 1994, 59, 7189 et D.J. Ager et al. Chem. Comm., 1997, 2359.

Ainsi, lorsque E représente phényle éventuellement substitué de préférence par (C₁-C₆)alkyle ou (C₁-C₆)alcoxy, le composé obtenu à l'issue de l'étape (iv) a pour formule Illa : 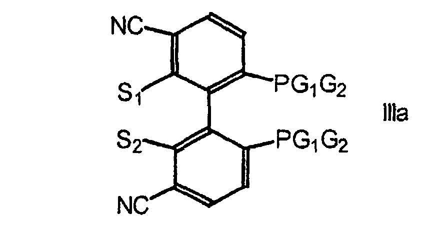 dans laquelle G₁, G₂, S₁ et S₂ sont tels que définis ci-dessus pour la formule IVa.

Lorsque E représente naphtyle, le composé obtenu à l'issue de l'étape (iv) a pour formule IIIb : 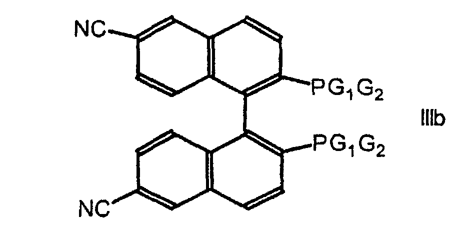 dans laquelle G₁ et G₂ sont tels que définis ci-dessus.

Dans le cas où la diphosphine présente une structure distincte de la formule II ci-dessus, l'homme du métier utilisera ses connaissances de base de la chimie organique pour réaliser sa synthèse à partir de produits commerciaux.

De façon générale, l'introduction de groupes aminométhyle sur le corps chiral pourra être réalisée par transformation de substituants méthyle convenablement positionnés sur un corps chiral approprié en groupes aminométhyle.

Pour ce faire, on pourrait réaliser tout d'abord la bromation des substituants méthyle d'un composé diméthylé de départ par action d'un agent de bromation approprié, puis réaliser une substitution nucléophile des atomes de brome ainsi introduits par action d'amines primaires ou secondaires appropriées de façon à pouvoir régénérer facilement un groupe -NH₂. Un exemple de telle amine est notamment la benzylamine, laquelle après réaction nucléophile peut être facilement soumise à une réaction de débenzylation par hydrogénation catalytique.

Une autre méthode consiste à réaliser la bromation des substituants méthyle, suivie de la réaction des dérivés bromés correspondants avec NaN₃, puis réduction par hydrogénation catalytique ou action d'un agent réducteur approprié.

En variante, on peut envisager la bromation des substituants méthyle, suivie de la réaction avec un phtalimide de métal alcalin, puis hydrolyse du composé résultant.

Il doit être entendu que lorsque le composé diméthylé de départ comprend une ou plusieurs fonctions sensibles, et donc réactives dans des conditions oxydantes, il convient au préalable de les protéger. Ainsi, lorsque le composé diméthylé de départ comprend un atome de phosphore, celui-ci est préalablement protégé, par exemple par oxydation.

Lorsque le polymère visé est une polyurée, celle-ci peut être synthétisée par polymérisation d'une diphosphine portant deux groupes -NH₂ ou -CH₂-NH₂ avec un ou plusieurs diisocyanates.

La nature du diisocyanate n'est pas critique en soi. De manière préférée, le diisocyanate est un diisocyanate de formule IX :

O=C=N-J-N=C=O IX

dans laquelle J représente un radical divalent hydrocarboné à caractère aliphatique, alicyclique et/ou aromatique. La taille du radical J sera ajustée par l'homme du métier en fonction de l'utilisation finale du ligand et notamment en fonction de la réaction que doit catalyser le complexe métallique formé à partir de ce ligand polymère.

Les sites catalytiques du polymère de l'invention sont situés au niveau des motifs issus de la diphosphine. La taille du radical J détermine donc l'espacement des sites catalytiques.

Le radical J est par exemple une chaîne alkylène en C₁-C₁₆, de préférence C₁-C₁₂, éventuellement interrompue par un ou plusieurs (de préférence 1 à 4, mieux encore 1 à 2) hétéroatomes choisis parmi O, N et S, ladite chaîne comprenant éventuellement une ou plusieurs insaturations (de préférence 1 à 4, mieux encore 1 à 2) ; un radical -(CH₂)ₐ-K-(CH₂)_{b}- où a et b sont indépendamment un nombre entier de 0 à 6 et K représente (C₆-C₈)cycloalkylène ; un radical -(CH₂)ₐ-L-(CH₂)_{b}- où a et b sont tels que définis ci-dessus et L représente (C₆-C₁₀)arylène ; un radical -(CH₂)ₐ-Vₒ-(CH₂)_{b}- où a et b sont tels que définis ci-dessus et Vₒ représente hétéroarylène de 5 à 8 chaînons comprenant 1 à 3 hétéroatomes choisi parmi O, N et S ; ou bien encore un radical -Mₒ-Q-Mₒ- dans lequel Mₒ est choisi parmi (C₃-C₈)cycloalkylène et (C₆-C₁₀)arytène et Q représente une liaison, un atome de soufre, un atome d'oxygène, alkylène en (C₁-C₄), -SO-, -SO₂- ou -CO-.

Lorsque J contient une chaîne alkylène, celle-ci est linéaire ou ramifiée et comporte de préférence 1 à 6 atomes de carbone. Lorsque cette chaîne alkylène comprend un atome d'azote, celui-ci porte un radical (C₁-C₆)alkyle ou un atome d'hydrogène.

Lorsque J contient cycloalkylène, on préfère que J soit cyclohexylène.

Lorsque J contient arylène, on préfère que J soit phénylène ou naphtalène.

Lorsque J représente -(CH₂)ₐ-L-(CH₂)_{b}-, -(CH₂)ₐ-K-(CH₂)_{b}- ou -(CH₂)ₐ-Vₒ-(CH₂)_{b}-, on préfère que a et b soient identiques.

Par hétéroarylène, on entend un radical bivalent correspondant à un hétérocycle dans lequel deux atomes d'hydrogène ont été remplacés par deux liaisons.

On préfère les hétéroarylènes dérivés des hétérocycles : furanne, thiophène, pyrrole, oxazole, thiazole, imidazole, pyrrazole, isoxazole, isothiazole, pyridine, pyridazine, pyrimidine, pyrazine, indolizine, indole, isoindole, benzofuranne, benzothiophène, benzimidazole, benzothiazole, quinoléine, isoquinoléine, cinnoline, phtalazine, quinazoline, naphtyridine et ptéridine. De façon très avantageuse, hétéroarytène est dérivé de imidazole, benzimidazole, pyrimidine ou quinazoline.

Lorsque J représente -Mₒ-Q-Mₒ-, on préfère que Q soit (C₁-C₂)alkylène, une liaison, et Mₒ soit cyclohexylène ou phénylène.

Le radical J tel que défini ci-dessus peut porter un ou plusieurs substituants choisis parmi un atome d'halogène, un groupe alkyle en C₁-C₆, un groupe alcoxy en C₁-C₆, un groupe oxo et un groupe di(C₁-C₆)alkylamino.

Des exemples de diisocyanates particulièrement appropriés sont :
- le diisocyanato-1,2-propane ;
- le diisocyanato-1,2-butane ;
- le diisocyanato-1,3-butane ;
- le diisocyanato-2,4-toluène ;
- le 4,4'-diisocyanato-3,3',5,5'-tétraéthyl-diphénylméthane;
- le 1,5-diisocyanatohexane; et
- le 5-isocyanato-1-isocyanatométhyl-1,3,3-triméthylcyclohexane.

Parmi les diisocyanates préférablement utilisés selon l'invention on préfère ceux présentant un axe de symétrie C₂ ou un plan de symétrie.

Un groupe particulièment préféré de diisocyanates est notamment constitué des composés suivants, lesquels présentent une symétrie d'axe C₂:
- le diisocyanato-1,12-dodécane
- le diisocyanato-1,8-octane
- le trans-1,4-cyclohexanediisocyanate
- le diisocyanato-2,6-toluène
- le diisocyanato-2,3-xylène
- le diisocyanato-2,6-xylène
- le diisocyanato-3,3'-biphényle
- le diisocyanato-4,4'-biphényle
- le diisocyanato-3,3'-diphénylméthane
- le diisocyanato-4,4'-diphénylméthane
- le diisocyanato-1,6-hexane
- le diisocyanato-1,3-benzène
- le diisocyanato-1,4-benzène
- le 2-méthyl-1,3-phénylènediisocyanate
- le 4-méthyl-1,3-phénylènediisocyanate
- le 1,3-phénylènediisocyanate
- le diisocyanato-4,4'-diméthyl-3,3'-diphényle
- le diisocyanato-4,4'-diméthyl-3,3'-diphénylméthane
- le diisocyanato-4,4'-diphényléthane
- le diisocyanato-3,3'-diphényléther
- le diisocyanato-4,4'-diphényléther
- la diisocyanato-3,3'-diphénylsulfone
- la diisocyanato-4,4'-diphénylsulfone
- le diisocyanato-3,3'-benzophénone
- la diisocyanato-4,4'-benzophénone
- le diisocyanato-3,3'-dicyclohexylméthane
- le diisocyanato-4,4'-dicyclohexyléthane
- le diisocyanato-1,5-naphtalène
- le diisocyanato-4,4'-dichloro-3,3'-biphényle
- le diisocyanato-4,4'-diméthoxy-3,3'-biphényle.

Plus particulièrement, les diisocyanates suivants à symétrie d'axe C₂ sont préférés :
- le diisocyanato-1,6-hexane
- le diisocyanato-2,6-toluène
- le diisocyanato-2,4-xylène
- le diisocyanato-4,4'-biphényle
- le diisocyanato-4,4'-diphénylméthane
- le diisocyanato-4,4'-diphényléther
- la diisocyanato-4,4'-diphénylsulfone
- le diisocyanato-4,4'-benzophénone
- le diisocyanato-4,4'-dicylohexyléthane
- le diisocyanato-1,5-naphtalène.

La condensation du diisocyanate avec la diphosphine est mise en oeuvre dans des conditions appropriées qui sont facilement déterminées par l'homme du métier.

Ces conditions de polymérisation sont préférablement ajustées de façon à obtenir un polymère présentant un degré de polymérisation de 2 à 100, de préférence de 5 à 100, par exemple de 2 à 50, mieux encore de 4 à 25.

Des polyurées d'un degré de polymérisation de 3 à 8 conviennent particulièrement bien.

L'homme du métier choisira le degré de polymérisation de façon à ce que le polymère résultant soit insoluble dans le solvant ou mélanges de solvants utilisés dans la réaction asymétrique devant être catalysée.

Le choix de la méthode de polymérisation n'est pas critique selon l'invention.

Une méthode particulièrement appropriée est la polymérisation en solution.

Le solvant est généralement un solvant polaire aprotique choisi parmi un hydrocarbure aliphatique éventuellement halogéné, par exemple le chlorure de méthylène, le chloroforme, le tétrachlorure de carbone ou le dichloroéthane ; un hydrocarbure aromatique éventuellement halogéné, par exemple le chlorobenzène ou le dichlorobenzène ; un éther tel que l'éther de diéthyle, l'éther de diisopropyle, le tétrahydrofuranne, le dioxanne, l'éther diméthylique de diéthylèneglycol, les glymes et notammnent le 1,2-diméthoxyéthane; un amide tel que le formamide, le diméthylformamide, le diméthylacétamide, la N-méthyl-2-pyrrolidinone ou l'hexaméthylphosphorylamide ; un nitrile tel que l'acétonitrile ou l'isobutyronitrile ; et le diméthylsulfoxyde.

La concentration des réactifs dans la solution varie très largement en fonction de la solubilité des réactifs. Elle est généralement comprise entre 0,05 et 1 mol/l, de préférence entre 0,01 et 1 mol/l, par exemple 0,1 mol/l.

De manière préférée, le diisocyanate est utilisé en excès par rapport à la diphosphine, bien qu'en toute rigueur un rapport stoechiométrique de ces deux composés puisse convenir.

Ainsi, le rapport molaire du diisocyanate à la diphosphine est généralement fixé entre 1 et 1,5, par exemple entre 1 et 1,3.

La température à laquelle est mise en oeuvre la polymérisation est déterminée en fonction de la réactivité des différents réactifs et du degré de polymérisation souhaité. A titre d'indication, la température varie entre -20° C et 100° C, de préférence entre la température ambiante et 100° C, par exemple entre 15 et 100° C, mieux encore entre 15 et 40° C. Avantageusement, elle est de 20° C.

La polymérisation est mise en oeuvre de façon conventionnelle par solubilisation des réactifs dans le solvant, mélange, éventuellement chauffage du milieu réactionnel, puis isolement du polymère, par exemple par filtration du milieu réactionnel. On notera qu'il peut être nécessaire, avant isolement du polymère, de désactiver les extrémités de la chaîne polymère, et notamment les fonctions isocyanates n'ayant pas réagi, par addition d'un alcanol en C₁-C₆, par exemple du propanol, de l'isopropanol, du méthanol, de l'éthanol ou même de l'alcool tert-butylique.

Lorsque le polymère est un polyamide, celui-ci peut être préparé par condensation d'une diphosphine chirale portant deux fonctions amino ou aminométhyle avec un ou plusieurs acides dicarboxyliques ou dérivés activés de ceux-ci.

L'acide dicarboxylique répond avantageusement à la formule X suivante :

HOOC-M-COOH X

dans laquelle M est tel que défini pour J ci-dessus. Les significations préférées de J indiquées ci-dessus sont également des significations préférées de M. Le radical M peut être substitué par un ou plusieurs atomes d'halogène ou groupes oxo, (C₁-C₆)alkyle, (C₁-C₆)alcoxy ou di(C₁-C₆)alkylamino.

Parmi ces acides dicarboxyliques, on préfère ceux présentant un axe de symétrie C₂ tels que :
- les acides aliphatiques choisis parmi :
   - l'acide malonique
   - l'acide succinique
   - l'acide glutarique
   - l'acide adipique
   - l'acide diméthyl-2,4-adipique
   - l'acide pimélique
   - l'acide subérique
   - l'acide azélaïque
   - l'acide sébacique
   - l'acide dodécanedioïque
   - l'acide fumarique
   - l'acide maléique
   - l'acide méthyliminodiacétique
   - l'acide diméthylamino-3-hexanedioïque,
- les acides cycloalcanedicarboxyliques et notamment :
   - l'acide cyclohexane-1,4-dicarboxylique
- les acides aromatiques dicarboxyliques choisis parmi :
   - l'acide phtalique
   - l'acide isophtalique
   - l'acide téraphtalique
   - l'acide phénylènediacétique
   - l'acide naphtalène-1,5-dicarboxylique
   - l'acide diphényl-4,4'-dicarboxylique
   - l'acide diphényl-3,3'-dicarboxylique
   - la dicarboxy-4,4'-diphénylsulfone
   - la dicarboxy-3,3'-diphénylsulfone.

D'autres acides dicarboxyliques sont néanmoins utilisables tels que, par exemple :
- l'acide diméthylamino-3-cyclopentane-1,2-dicarboxylique
- l'acide naphtalène-1,6-dicarboxylique
- les acides pyrimidinedicarboxyliques ; et
- les acides imidazoledicarboxyliques.

Un groupe particulièrement préféré d'acides dicarboxyliques est constitué des acides suivants :
- l'acide succinique
- l'acide adipique
- l'acide fumarique
- l'acide isophtalique
- l'acide téréphtalique
- l'acide naphtalène-1,5- dicarboxylique
- l'acide diphényl-4,4'-dicarboxylique
- l'acide diphényl-3,3'-dicarboxylique.

Le dérivé activé de l'acide dicarboxylique désigne plus généralement le composé acide dicarboxylique dans lequel une ou deux des fonctions carboxyliques ont été modifiées de façon à accroître leur réactivité.

Des dérivés activés d'acide dicarboxylique sont par exemple obtenus par formation d'une liaison anhydride ou d'un groupe -COY où Y est un atome d'halogène tel que brome ou chlore.

D'autres dérivés activés des acides dicarboxyliques sont ceux portant en lieu et place des fonctions carboxyliques, des groupes -COT où T désigne un groupe azide, imidazolide, p-nitrophénoxy, 1-benzotriazole, N-O-succinimide, acyloxy (tel que pivaloyloxy), (alcoxy en C₁-C₄)carbonyloxy, dialkyl- ou dicycloalkyl-O-uréide.

Un exemple de polymère particulièrement préféré est un polymère présentant comme motif récurrent : 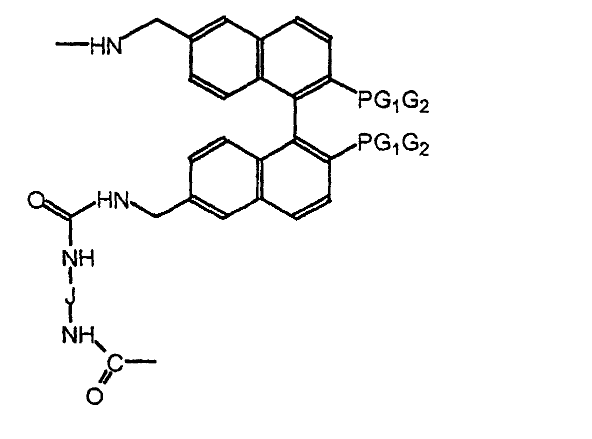 dans lequel
G₁ et G₂ représentent indépendamment un groupe carbocyclique saturé ou aromatique ; et
J représente un radical divalent hydrocarboné à caractère aliphatique, alicyclique et/ou aromatique ;
le degré de polymérisation étant préférablement compris entre 2 et 100, mieux encore entre 2 et 50.

Un autre exemple de polymère préféré est un polymère présentant comme motif récurrent : 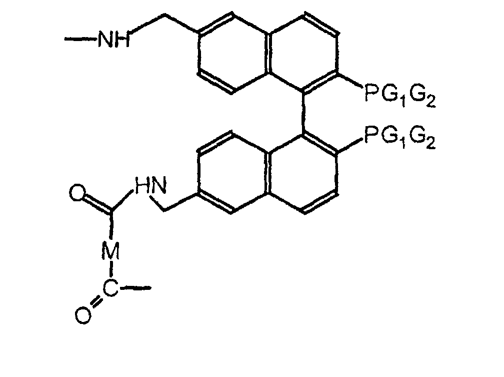 dans lequel
G₁ et G₂ sont tels que définis ci-dessus et M est tel que défini pour J.

Des significations préférées de G₁, G₂ et J sont telles que définies ci-dessus.

La condensation de la diphosphine avec l'acide dicarboxylique ou son dérivé activé est généralement mise en oeuvre dans un solvant.

Lorsque l'acide dicarboxylique est utilisé en tant que tel, il peut être avantageux de réaliser la condensation en présence d'un catalyseur, par exemple un acide fort tel que l'acide chlorhydrique ou sulfurique ou bien en présence d'un agent de condensation tel que ceux couramment utilisés en synthèse peptidique.

Parmi les agents de condensation connus, on peut citer les dérivés N-hydroxylés tels que le N-hydroxysuccinimide et le 1-hydroxybenzotriazole ; les disulfures tels que le 2,2'-disulfure de dipyridyle ; les dérivés d'acide succinique tels que le carbonate de N,N'-disuccinimidyle ; les chlorures phosphiniques tels que le chlorure N,N'-bis-(2-oxo-3-oxazolidinyl)phosphinique ; les oxalates tels que l'oxalate de N,N'-disuccinimidyle (DSO), le N,N'-oxalate de diphtalimide (DPO), l'oxalate de N,N'-bis(norbornénylsuccinimidyle) (BNO), l'oxalate de 1,1'-bis(benzotriazolyle) (BBTO), l'oxalate de 1,1'-bis(6-chlorobenzotriazolyle) (BCTO) ou l'oxalate de 1,1'-bis(6-trifluorométhylbenzotriazolyle) (BTBO) ; les triarylphosphines telles que la triphénylphosphine ; une association d'un azodicarboxylate de di(alkyle inférieur) et d'une triarylphosphine, telle qu'une association d'azodicarboxylate de diéthyle et de triphénylphosphine ; les N-(alkyle inférieur)-5-aryl-isoxazolium-3'-sulfonates tels que le N-éthyl-5-phényl-isoxazolium-3'-sulfonate; les dérivés de carbodiimide, incluant des N',N'-dicycloalkylcarbodiimides tels que le N',N'-dicyclohexylcarbodiimide (DCC) ou le 1-éthyl-3-(3-diméthylaminopropyl)carbodiimide (EDAPC) ; les diséléniures de dihétéroaryle tels que le diséléniure de di-2-pyridyle ; les arylsulfonyltriazolides tels que le p-nitrobenzène-sulfonyltriazolide ; les halogénures de 2-halogéno-1-(alkyle inférieur)pyridinium tels que l'iodure de 2-chloro-1-méthylpyridinium; les diarylphosphorylazides tels que le diphénylphosphorylazide (DPPA) ; les dérivés d'imidazole tels que le 1,1'-oxalyldiimidazole ou le N,N'-carbonyldiimidazole ; les dérivés de benzotriazole tels que le 1-hydroxybenzotriazole (HOBT) ; et les dérivés de dicarboximide tels que le N-hydroxy-5-norbornène-2,3-dicarboximide (HONB). Parmi ceux-ci, on préfère les dérivés de carbodiimides.

La réaction peut avoir lieu dans un large intervalle de température.

Suivant la réactivité des réactifs mis en présence, la température réactionnelle oscille entre -20° C et 100° C.

Lorsque la polymérisation implique la réaction d'un dérivé activé de l'acide dicarboxylique sur une diphosphine, une température relativement basse, préférablement comprise entre 0° C et 40° C, est suffisante,

A l'inverse, lorsque l'acide dicarboxylique en tant que tel est mis en jeu dans la réaction, la température est préférablement comprise entre 50 et 80° C.

La concentration des réactifs dans le milieu réactionnel n'est pas déterminante selon l'invention. Elle peut varier entre 0,05 et 1 mol/l.

De façon générale, le rapport molaire de l'acide dicarboxylique ou de son dérivé activé à la diphosphine varie entre 0,8 et 1,5, de préférence entre 0,9 et 1,2.

Un mode opératoire typique, illustrant la préparation d'un polyamide au départ d'un chlorure d'acide carboxylique, est le suivant.

A une solution de 4,16 mmol de diphosphine dans 5 ml de N,N-diméthylacétamide, on ajoute 3,75 mmol du chlorure d'acide carboxylique. Le mélange réactionnel est maintenu une nuit sous agitation à température ambiante (18 à 30° C). Puis le polyamide est précipité dans 150 ml d'eau distillée. Le polymère est filtré sur verre fritté, lavé à l'eau puis à l'isopropanol.

Les conditions générales de mise en oeuvre de la polymérisation et d'isolement du polymère seront facilement déterminées par l'homme du métier, étant entendu que les polyamides préférés de l'invention présentent un degré de polymérisation compris entre 2 et 100, par exemple entre 5 et 100, de préférence entre 2 et 50, mieux encore, entre 4 et 25.

L'homme du métier choisira le degré de polymérisation de façon à ce que le polymère résultant soit insoluble dans le solvant ou mélanges de solvants utilisés dans la réaction asymétrique devant être catalysée.

Lorsque le polymère est une polythiourée, celle-ci peut être préparée par condensation d'une diphosphine chirale portant deux fonctions amino ou aminométhyle avec un ou plusieurs diisothiocyanates.

On utilisera de préférence un diisothiocyanate présentant un axe de symétrie C₂ tel que, par exemple :
- le 1,4-butanediisothiocyanate ;
- le 1,3-propanediisothiocyanate ;
- la bis(4-isothiocyanatophényl)sulfone ; ou
- le 1,4-phénylènediisothiocyanate.

De façon plus générale, le diisothiocyanate répond à la formule :

S=C=N-J-N=C=S

dans laquelle J est tel que défini ci-dessus.

Pour la mise en oeuvre de la condensation, l'homme du métier pourra s'inspirer des conditions réactionnelles décrites ci-dessus pour la préparation des polyurées.

Lorsque le polymère est un polyimide, celui-ci peut être préparé par condensation d'une diphosphine chirale portant deux fonctions amino ou aminométhyle avec un ou plusieurs acides tétracarboxyliques ou dianhydrides d'acide tétracarboxylique.

Pour la préparation de ces polyimides, l'homme du métier pourra s'inspirer de D.C. Sherrington, Chem. Commun, 1998, 2275-2286.

De manière avantageuse, les polyimides sont préparés en deux étapes. Dans une première étape, on forme un polyamide. Cette étape est, par exemple, conduite à une température comprise entre 15 et 50° C, de préférence comprise entre 20 et 30° C, dans un solvant aprotique polaire (tel qu'un amide du type du formamide, du diméthylacétamide, de la N-méthyl-2-pyrrolidinone, préférablement du diméthylacétamide). Dans une deuxième étape, on forme le polyimide. Cette deuxième étape peut être réalisée par traitement avec un mélange d'anhydride acétique et de pyridine à une température comprise entre -100° C et 10° C, de préférence comprise entre -78 et -50° C.

Selon un de ses aspects, l'invention concerne donc un procédé pour la préparation d'un polymère de l'invention comprenant la polymérisation d'une diphosphine chirale présentant un axe de symétrie C₂, à l'exclusion de tout autre élément de symétrie, avec un ou plusieurs monomères polymérisables, ladite phosphine chirale étant constituée d'un corps chiral portant deux groupes fonctionnels identiques capables de reagir avec lesdits monomères polymérisables.

L'invention concerne également le polymère racémique correspondant au polymère optiquement actif de l'invention.

Ce polymère peut être préparé simplement par polymérisation de la diphosphine appropriée avec un ou plusieurs monomères polymérisables, ladite diphosphine portant deux groupes fonctionnels identiques capables de réagir avec lesdits monomères polymérisables.

De manière préférée, les diphosphines utilisées dans cette réaction sont les diphosphines racémiques correspondant aux diphosphines chirales préférées définies ci-dessus. Ainsi, selon un mode de réalisation préféré de l'invention, la diphosphine racémique est constituée d'un squelette de base racémique de formule I.1, I.2, I.3, I.4, I.5, I.6 ou I.7 portant deux groupes fonctionnels identiques.

De même, les monomères polymérisables préférablement utilisés pour cette polymérisation sont ceux décrits ci-dessus pour la préparation des polymères optiquement actifs.

Les conditions opératoires de cette polymérisation seront facilement déterminées par l'homme du métier par analogie à celles proposées pour la réaction de polymérisation conduisant au polymère optiquement actif.

Les polymères de l'invention sont utilisables comme ligands pour la préparation de complexes métalliques destinés à la catalyse asymétrique de nombreuses réactions telles que les réactions d'hydrogénation, d'hydrosilylation, d'hydroboration de composés insaturés, d'époxydation d'alcools allyliques, d'hydroxylation vicinale, d'hydrovinylation, d'hydroformylation, de cyclopropanation, de carbonylation, d'isomérisation d'oléfines, de polymérisation du propylène, d'addition de composés organométalliques à des aldéhydes, d'alkylation allylique, les réactions de type aldol et les réactions de Diels-Alder.

De tels complexes sont notamment les complexes du rhodium, de l'iridium, du ruthénium, du palladium, du platine, du cobalt et du nickel.

Parmi les complexes précédents, on préfère notamment les complexes du rhodium, du ruthénium, de l'iridium, du platine et du palladium. Dans le cadre de l'invention, les complexes du ruthénium, du rhodium et de l'iridium sont les plus avantageux.

Des exemples spécifiques desdits complexes de la présente invention sont donnés ci-après, sans caractère limitatif.

Dans les formules suivantes, Pₚ représente le polymère.

Un groupe préféré des complexes du rhodium et de l'iridium est défini par la formule :

[MeLig₂Pₚ]Y_{I} XI

dans laquelle :
Pₚ représente le polymère ;
Yₗ représente un ligand anionique coordinant ;
Me représente le rhodium ou l'iridium ;
Lig représente un ligand neutre.

Parmi ces composés, ceux dans lesquels :
- Lig représente une oléfine ayant de 2 à 12 atomes de carbone ;
- Yₗ représente un anion PF₆⁻, PCl₆⁻, BF₄⁻, BCl₄⁻, SbF₆⁻, SbCl₆⁻, BPh₄⁻, ClO₄⁻, CN⁻, CF₃SO₃⁻, halogène, de préférence Cl⁻ ou Br⁻, un anion 1,3-dicétonate, alkylcarboxylate, halogénoalkylcarboxylate avec un radical alkyle inférieure (de préférence en C₁-C₆) et/ou des atomes d'halogène, sont particulièrement préférés.

Dans la formule XI, Lig₂ peut représenter deux ligands Lig tels que définis ci-dessus ou un ligand bidente tel que ligand bidente, linéaire ou cyclique, polyinsaturé et comprenant au moins deux insaturations.

On préfère selon l'invention que Lig₂ représente le 1,5-cyclooctadiène, le norbornadiène ou bien que Lig représente l'éthylène.

Par radicaux alkyle inférieurs, on entend généralement un radical alkyle linéaire ou ramifié ayant de 1 à 4 atomes de carbone.

D'autres complexes d'iridium sont ceux de formule :

[IrLigPₚ]Yₗ XII

dans laquelle Lig, Pₚ et Yₗ sont tels que définis pour la formule XI.

Un groupe préféré de complexes du ruthénium est constitué des composés de formule :

[RuYₗ¹Yₗ²Pₚ] XIII

dans laquelle :
- Pₚ représente le polymère de l'invention ;
- Y_{I}¹ et Y_{I}², identiques ou différents, représentent un anion PF₆⁻, PCl₆⁻, BF₄⁻, BCl₄⁻, SbF₆⁻, SbCl₆⁻, BPh₄⁻, ClO₄⁻, CF₃SO₃⁻, un atome d'halogène, plus particulièrement chlore ou brome ou un anion carboxylate, préférentiellement acétate, trifluoroacétate.

D'autres complexes du ruthénium sont ceux répondant à la formule XIV suivante :

[RuY_{I}³arPₚY_{I}⁴] XIV

dans laquelle :
- Pₚ représente le polymère de l'invention ;
- ar représente le benzène, le p-méthylisopropylbenzène ou l'hexaméthylbenzène ;
Yₗ³ représente un atome d'halogène, de préférence chlore ou brome ;
Yₗ⁴ représente un anion, de préférence un anion PF₆⁻,PCl₆⁻, BF₄⁻, BCl₄⁻, SbF₆⁻, SbCl₆⁻, BPh₄⁻, ClO₄⁻, CF₃SO₃⁻.

Il est également possible de mettre en oeuvre dans le procédé de l'invention des complexes à base de palladium et de platine.

Comme exemples plus spécifiques desdits complexes, on peut mentionner entre autres Pd(hal)₂Pₚ et Pt(hal)₂Pₚ où Pₚ représente le polymère de l'invention et hal représente halogène tel que, par exemple, le chlore.

Les complexes comprenant le polymère de l'invention comme ligand et le métal de transition peuvent être préparés selon les procédés connus décrits dans la littérature.

Les complexes sont généralement préparés à partir d'un précatalyseur dont la nature varie suivant le métal de transition sélectionné.

Dans le cas des complexes du rhodium, le précatalyseur est par exemple l'un des composés suivants : [Rh^{l}(CO)₂Cl]₂ ; [Rh^{l}(COD)Cl]₂ où COD désigne le cyclooctadiène ; ou le Rh^{l}(acac)(CO)₂ où acac désigne l'acétylacétonate.

Dans le cas des complexes du ruthénium, des précatalyseurs convenant particulièrement bien sont le bis-(2-méthylallyl)-cycloocta-1,5-diène ruthénium et le [RuCl₂(benzène)]₂. On peut citer également le Ru(COD)(η³-(CH₂)₂CHCH₃)₂.

A titre d'exemple, au départ du bis-(2-méthylallyl)-cycloocta-1,5-diène ruthénium, on prépare une solution ou suspension contenant le précatalyseur métallique, le polymère de l'invention et un solvant parfaitement dégazé tel que l'acétone (la concentration en polymère de la solution ou suspension variant entre 0,001 et 1 mol/l), à laquelle on ajoute une solution méthanolique d'acide bromhydrique. Le rapport du ruthénium au brome varie avantageusement entre 1:1 et 1:4, de préférence entre 1:2 et 1:3. Le rapport molaire du ligand au métal de transition est quant à lui d'environ 1. Il peut être compris entre 0,8 et 1,2.

Lorsque le précatalyseur est [RuCl₂(benzène)]₂, le complexe est préparé par mélange du précatalyseur, du ligand polymère et d'un solvant organique et éventuellement maintenu à une température comprise entre 15 et 150° C pendant 1 minute à 24 heures, de préférence 30 à 120° C pendant 10 minutes à 5 heures.

A titre de solvant, on peut mentionner les hydrocarbures aromatiques (tels que benzène, toluène et xylène), les amides (tels que le formamide, le diméthylformamide, le diméthylacétamide, la N-méthyl-2-pyrrolidinone ou l'hexaméthylphosphorylamide), les alcools (tels que l'éthanol, le méthanol, le n-propanol et l'isopropanol) et leurs mélanges.

De manière préférée, lorsque le solvant est un amide, notamment le diméthylformamide, on chauffe le mélange du polymère, du précatalyseur et du solvant entre 80 et 120° C.

En variante, lorsque le solvant est un mélange d'un hydrocarbure aromatique (tel que le benzène) avec un alcool (tel que l'éthanol), on chauffe le milieu réactionnel à une température comprise entre 30 et 70° C.

Le catalyseur est alors récupéré selon les techniques classiques (filtration ou cristallisation) et utilisé dans des réactions asymétriques. Néanmoins, la réaction devant être catalysée par le complexe ainsi préparé peut être mise en oeuvre sans isolement intermédiaire du complexe catalyseur.

Dans la suite, le cas de l'hydrogénation est exposé en détail.

Le substrat insaturé, en solution dans un solvant comprenant le catalyseur, est placé sous pression d'hydrogène.

L'hydrogénation est par exemple effectuée à une pression variant entre 1,5 et 100 bar, et à une température comprise entre 20° C et 100° C.

Les conditions exactes de mise en oeuvre dépendant de la nature du substrat devant être hydrogéné. Néanmoins, dans le cas général, une pression de 20 à 80 bars, de préférence de 40 à 60 bars, et une température de 30 à 70° C, conviennent particulièrement bien.

Le milieu réactionnel peut être constitué du milieu réactionnel dans lequel a été obtenu le catalyseur. La réaction d'hydrogénation a alors lieu in situ.

En variante, le catalyseur est isolé du milieu réactionnel dans lequel il a été obtenu. Dans ce cas, le milieu réactionnel de la réaction d'hydrogénation est constitué d'un ou plusieurs solvants, notamment choisis parmi les alcools aliphatiques en C₁-C₅, tels que le méthanol ou le propanol, et un amide tel que défini ci-dessus, de préférence le diméthylformamide, éventuellement en mélange avec du benzène.

Lorsque la réaction d'hydrogénation a lieu in situ, il est souhaitable d'ajouter au milieu réactionnel un ou plusieurs solvants choisis parmi ceux mentionnés ci-dessus, et plus particulièrement un ou plusieurs alcools aliphatiques.

Selon un mode de réalisation préféré, on ajoute, au milieu réactionnel contenant le complexe, du méthanol parfaitement dégazé et le substrat. La quantité de méthanol, ou plus généralement de solvant, pouvant être ajoutée est telle que la concentration du substrat dans le milieu réactionnel d'hydrogénation est comprise entre 1.10⁻³ et 10 mol/l, de préférence entre 0,01 et 1 mol/l.

Le rapport molaire du substrat au catalyseur varie généralement de 1/100 à 1/100 000, de préférence de 1/20 à 1/2000. Ce rapport est par exemple de 1/1000.

Les complexes du rhodium préparés à partir des ligands polymères de l'invention sont plus spécialement appropriés à la catalyse asymétrique des réactions d'isomérisation d'oléfines.

Les complexes du ruthénium préparés à partir des ligands polymères de l'invention sont plus spécialement appropriés à la catalyse asymétrique des réactions d'hydrogénation de liaisons carbonyle, de liaisons C=C et de liaisons C=N.

Pour ce qui est de l'hydrogénation de doubles liaisons, les substrats appropriés sont de type acide carboxylique α,β-insaturé et/ou dérivés d'acide carboxylique α,β-insaturé. Ces substrats sont décrits dans EP 95943260.0.

L'acide carboxylique α,β-insaturé ou son dérivé répond plus particulièrement à la formule A : 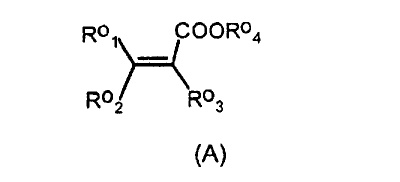 dans laquelle :
- R^{o}₁, R^{o}₂, R^{o}₃ et R^{o}₄, représentent un atome d'hydrogène ou n'importe quel groupe hydrocarboné, dans la mesure où :
   . si R^{o}₁ est différent de R^{o}₂ et différent d'un atome d'hydrogène alors R^{o}₃ peut être n'importe quel groupe hydrocarboné ou fonctionnel désigné par *R*,
   . si R^{o}₁ ou R^{o}₂ représente un atome d'hydrogène et si R^{o}₁ est différent de R^{o}₂, alors R^{o}₃ est différent d'un atome d'hydrogène et différent de -COOR^{o}₄,
   . si R^{o}₁ est identique à R^{o}₂ et représente n'importe quel groupe hydrocarboné ou fonctionnel désigné par *R*, alors R^{o}₃ est différent de -CH-(R)₂ et différent de -COOR^{o}₄,
- l'un des groupes R^{o}₁, R^{o}₂ et R^{o}₃ pouvant représenter un groupe fonctionnel.

Comme exemple spécifique, on peut mentionner entre autres, l'acide 2-méthyl-2-buténoïque.

Un premier groupe de substrats préférés est formé par les acides acryliques substitués précurseurs d'aminoacides et/ou dérivés.

Sous le terme acides acryliques substitués, on entend l'ensemble des composés dont la formule dérive de celle de l'acide acrylique par substitution d'au plus deux des atomes d'hydrogène portés par les atomes de carbone éthylénique par un groupe hydrocarboné ou par un groupe fonctionnel.

Ils peuvent être symbolisés par la formule chimique suivante : 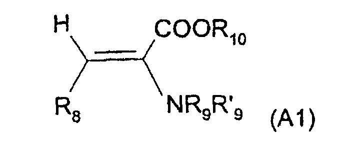 dans laquelle :
- R₉, R'₉, identiques ou différents représentent un atome d'hydrogène, un groupe alkyle, linéaire ou ramifié ayant de 1 à 12 atomes de carbone, un groupe phényle ou un groupe acyle ayant de 2 à 12 atomes de carbone de préférence, un groupe acétyle ou benzoyle,
- R₈ représente un atome d'hydrogène, un groupe alkyle ayant de 1 à 12 atomes de carbone, un radical cycloalkyle ayant de 3 à 8 atomes de carbone, un radical arylalkyle ayant de 6 à 12 atomes de carbone, un radical aryle ayant de 6 à 12 atomes de carbone, un radical hétérocyclique ayant de 4 à 7 atomes de carbone,
- R₁₀ représente un atome d'hydrogène ou un groupe alkyle linéaire ou ramifié, ayant de 1 à 4 atomes de carbone.

On peut citer plus particulièrement :
- l'α-acétamidocinnamate de méthyle,
- l'acétamidoacrylate de méthyle,
- l'acide benzamidocinnamique,
- l'acide α-acétamidocinnamique.

Un second groupe préféré de substrats est constitué de l'acide itaconique et de ses dérivés de formule : 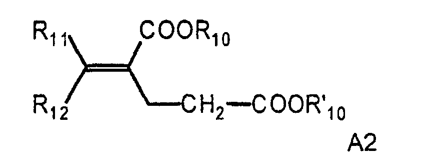 dans laquelle :
- R₁₁, R₁₂, identiques ou différents représentent un atome d'hydrogène, un groupe alkyle linéaire ou ramifié ayant de 1 à 12 atomes de carbone, un radical cycloalkyle ayant de 3 à 8 atomes de carbone, un radical arylalkyle ayant de 6 à 12 atomes de carbone, un radical aryle ayant de 6 à 12 atomes de carbone, un radical hétérocyclique ayant de 4 à 7 atomes de carbone.
- R₁₀, R'₁₀, identiques ou différents représentent un atome d'hydrogène ou un groupe alkyle linéaire ou ramifié, ayant de 1 à 4 atomes de carbone.

Comme exemples plus particuliers, on peut mentionner notamment l'acide itaconique et l'itaconate de diméthyle.

Un troisième groupe préféré de substrats est défini par la formule A3 : 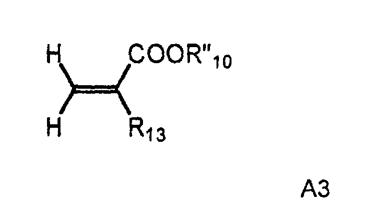 dans laquelle :
- R"₁₀ représente un atome d'hydrogène ou un groupe alkyle linéaire ou ramifié, ayant de 1 à 4 atomes de carbone.
- R₁₃ représente un groupe phényle ou naphtyle, éventuellement porteur d'un ou plusieurs substituants.

Comme exemples spécifiques, on peut citer les substrats conduisant par hydrogénation à l'acide 2-(3-benzoylphényl)propionique (Kétoproféne®), l'acide 2-(4-isobutylphényl)propionique (Ibuprofène®), l'acide 2-(5-méthoxynaphtylpropionique (Naproxène®).

Pour ce qui est de l'hydrogénation de liaisons carbonyle, les substrats appropriés de type cétonique répondent plus préférablement à la formule B : 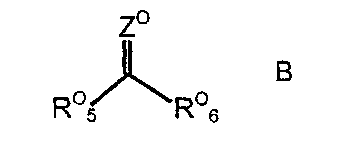 dans laquelle :
- R^{o}₅ est différent de R^{o}₆
- R^{o}₅ et R^{o}₆ représentent un radical hydrocarboné ayant de 1 à 30 atomes de carbone comprenant éventuellement un ou plusieurs groupes fonctionnels,
- R^{o}₅ et R^{o}₆ peuvent former un cycle comprenant éventuellement un autre hétéroatome,
- Z^{o} est ou comprend un hétéroatome, oxygène ou azote ou un groupe fonctionnel comprenant au moins un de ces hétéroatomes.

Ces composés sont précisément décrits dans FR 9608060 et EP 97930607.3.

Un premier groupe préféré de tels substrats cétoniques a pour formule B1 : 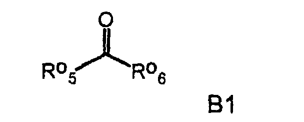 dans laquelle :
- R⁰₅ est différent de R⁰₆, les radicaux R⁰₅ et R⁰₆ représentent un radical hydrocarboné ayant de 1 à 30 atomes de carbone comprenant éventuellement une autre fonction cétone et/ou acide, ester, thioacide, thioester ;
- R⁰₅ et R⁰₆ peuvent former un cycle carbocyclique ou hétérocyclique, substitué ou non, ayant de 5 à 6 atomes.

Parmi ces composés, on préfère tout particulièrement les cétones choisies parmi :
- méthylphénylcétone,
- isopropylphénylcétone,
- cyclopropylphénylcétone,
- allylphénylcétone,
- p-méthylphénylméthylcétone,
- benzylphénylcétone,
- phényltriphénylméthylcétone,
- o-bromoacétophénone,
- α- bromoacétone,
- α-dibromoacétone,
- α-chloroacétone,
- α-dichloroacétone,
- α-trichloroacétone,
- 1-chloro-3,3-dichloroacétone,
- 1-chloro-2-oxobutane,
- 1-fluoro-2-oxobutane,
- 1-chloro-3-méthyl-2-butanone,
- α-chloroacétophénone,
- 1-chloro-3-phénylacétone,
- α-méthylaminoacétone,
- α-diméthylaminoacétone,
- 1-butylamino-2-oxopropane,
- 1-dibutylamino-2-oxopropane.
- 1-méthylamino-2-oxobutane,
- 1-diméthylamino-2-oxobutane,
- 1-diméthylamino-3-méthyl-2-oxobutane,
- 1-diméthylamino-2-oxopentane,
- α-diméthylaminoacétophénone,
- α-hydroxyacétone,
- 1-hydroxy-3-méthyl-2-butanone,
- 1-hydroxy-2-oxobutane,
- 1-hydroxy-2-oxopentane,
- 1-hydroxy-2-oxohexane,
- 1-hydroxy-2-oxo-3-méthylbutane,
- α-hydroxyacétophénone,
- 1-hydroxy-3-phénylacétone,
- α-méthoxyacétone,
- α-méthoxyacétophénone,
- α-éthoxyacétone,
- α-butoxyacétophénone,
- α-chloro-p-méthoxyacétophénone,
- α-naphténone,
- 1-éthoxy-2-oxobutane,
- 1-butoxy-2-oxobutane,
- α-diméthoxyphosphorylacétone,
- 3-oxotétrahydrothiophène.

Les substrats de type aldéhyde/cétone présentant un second groupe carbonyle en position α, β, γ ou δ par rapport au premier groupe carbonyle sont également particulièrement appropriés dans le cadre de l'invention. Des exemples de tels composés dicétoniques sont :
- α-formylacétone,
- diacétyle,
- 3,4-dioxohexane,
- 4,5-dioxooctane,
- 1-phényl-1,2-dioxopropane,
- 1-phényl-2,3-dioxobutane,
- diphénylglyoxal,
- p-méthoxydiphénylglyoxal,
- 1,2-cyclopentanedione,
- 1,2-cyclohexanedione,
- acétylacétone,
- 3,5-heptanedione,
- 4,6-nonanedione,
- 5,7-undecadione,
- 2,4-hexanedione,
- 2,4-heptanedione,
- 2,4-octanedione,
- 2,4-nonanedione,
- 3,5-nonanedione,
- 3,5-décanedione,
- 2,4-dodécanedione,
- 1-phényl-1,3-butanedione,
- 1-phényl-1,3-pentanedione,
- 1-phényl-1,3-hexanedione,
- 1-phényl-1,3-heptanedione,
- 3-méthyl-2,4-pentanedione,
- 1,3-diphényl-1,3-propanedione,
- 1,5-diphényl-2,4-pentanedione,
- 1,3-di(trifluorométhyl)-1,3-propanedione,
- 3-chloro-2,4-pentanedione,
- 1,5-dichloro-2,4-pentanedione,
- 1,5-dihydroxy-2,4-pentanedione,
- 1,5-dibenzyloxy-2,4-pentanedione,
- 1,5-diamino-2,4-pentanedione,
- 1,5-di(méthylamino)2,4-pentanedione,
- 1,5-di(diméthylamino)-2,4-pentanedione,
- 3,5-dioxo-hexanoate de méthyle.
- 3-carbométhoxy-2,4-pentanedione,
- 3-carboéthoxy-2,4-pentanedione,
- 1,3-cyclopentanedione,
- 1,3-cyclohexanedione,
- 1,3-cycloheptanedione,
- 5-carboéthoxy-1,3-cyclopentanedione,
- 2-acétyl-1-cyclopentanone,
- 2-acétyl-1-cyclohexanone.

Comme autres substrats convenant particulièrement bien, on peut citer les cétoacides ou leurs dérivés et les cétothioacides ou leurs dérivés avec un groupe fonctionnel (acide, ester, thioacide ou thioester) en position α, β, γ ou δ par rapport au groupe carbonyle. Des exemples en sont les :
- acide 2-acétylbenzoïque,
- acide pyruvique,
- acide 2-oxobutanoïque,
- acide 3-méthyl-2-oxobutanoïque,
- acide phénylglyoxylique,
- acide phénylpyruvique,
- acide p-méthoxyphénylpyruvique,
- acide 3,4-diméthoxyphénylpyruvique,
- acétoacétate de méthyle,
- acétoacétate d'éthyle,
- acétoacétate de n-propyle,
- acétoacétate d'isopropyle,
- acétoacétate de n-butyle,
- acétoacétate de t-butyle,
- acétoacétate de n-pentyle,
- acétoacétate de n-hexyle,
- acétoacétate de n-heptyle,
- acétoacétate de n-octyle,
- 3-oxopentanoate de méthyle,
- 3-oxohexanoate de méthyle,
- 3-oxoheptanoate de méthyle,
- 3-oxooctanoate d'éthyle,
- 3-oxononanoate d'éthyle,
- 3-oxodécanoate d'éthyle,
- 3-oxoundécanoate d'éthyle,
- 3-oxo-3-phénylpropanoate d'éthyle,
- 4-phényl-3-oxobutanoate d'éthyle,
- 5-phényl-3-oxopentanoate de méthyle,
- 3-oxo-3-p-méthoxyphénylpropanoate d'éthyle,
- 4-chloroacétoacétate de méthyle,
- 4-chloroacétoacétate d'éthyle,
- 4-fluoroacétoacétate de méthyle,
- 3-trifluorométhyl-3-oxopropanoate d'éthyle,
- 4-hydroxy-3-oxobutanoate d'éthyle,
- 4-méthoxyacétoacétate de méthyle,
- 4-tert-butoxyacétoacétate de méthyle,
- 4-benzyloxy-3-oxobutanoate de méthyle,
- 4-benzyloxy-3-oxobutanoate d'éthyle,
- 4-amino-3-oxobutanoate de méthyle,
- 3-méthylamino-3-oxobutanoate d'éthyle,
- 4-diméthylamino-3-oxobutanoate de méthyle,
- 4-diméthylamino-3-oxobutanoate d'éthyle,
- 2-méthylacétoacétate de méthyle,
- 2-méthylacétoacétate d'éthyle,
- 2-chloroacétoacétate d'éthyle,
- 2-acétylsuccinate de diéthyle,
- 2-acétylglutarate de diéthyle,
- acétylmalonate de diméthyle,
- acétoacétate de thiométhyle,
- acétoacétate de thioéthyle,
- acétoacétate de thiophényle,
- pyruvate de méthyle,
- 3-méthyl-2-oxobutanoate d'éthyle,
- phénylglyoxolate d'éthyle,
- phénylpyruvate de méthyle,
- phénylpyruvate d'éthyle,
- 3-oxobutanoic diméthylamide,
- 3-oxobutanoic benzylamide,
- 2-carboéthoxy-cyclopentanone,
- 2-carboéthoxy-cyclohexanone,
- cétopentalactone.
- acide 4-oxopentanoïque,
- acide 4-oxohexanoique,
- acide 4-oxoheptanoique,
- acide 4-oxodécanoïque,
- acide 4-oxododécanoïque,
- acide 4-phényl-4-oxybutyrique,
- acide 4-p-méthoxyphényl-4-oxybutyrique,
- acide 4-(3,4-diméthoxyphényl)-4-oxobutyrique,
- acide 4-(3,4,5-triméthoxyphényl)-4-oxobutyrique,
- acide 4-p-chlorophényl-4-oxybutyrique,
- acide 4-phényl-4-oxobutyrique.

Il est à noter que lorsque l'on a à faire l'hydrogénation asymétrique d'un γ-cétoacide ou dérivé, le produit obtenu est généralement un dérivé de γ-butyrolactone et dans le cas d'un δ-cétoacide, il s'agit d'un dérivé de valérolactone.

Comme autres exemples de cétones, on peut mentionner entre autres, les composés cétoniques cycliques, saturés ou insaturés, monocycliques ou polycycliques suivants : 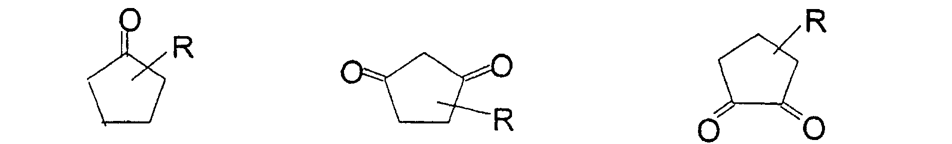 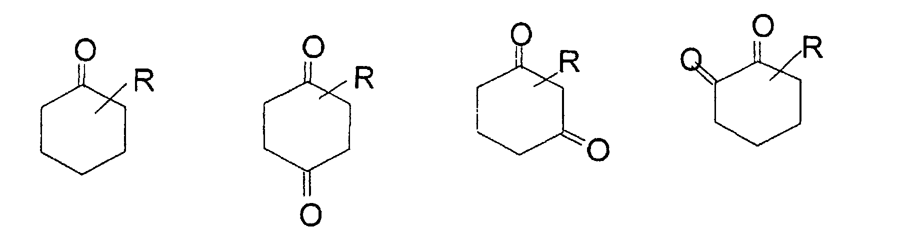 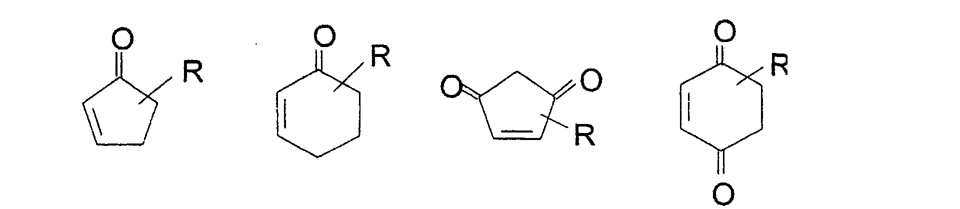 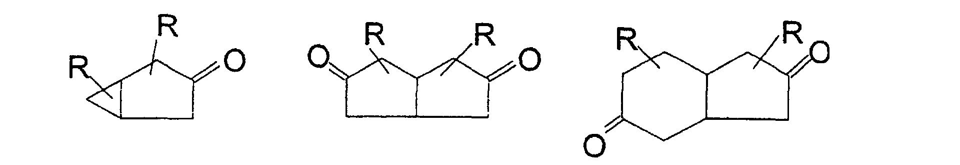 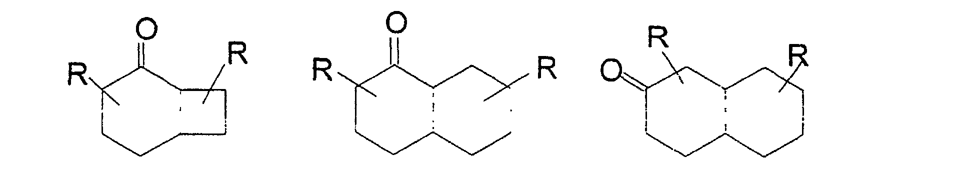 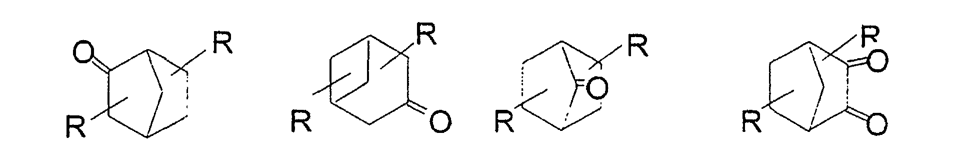 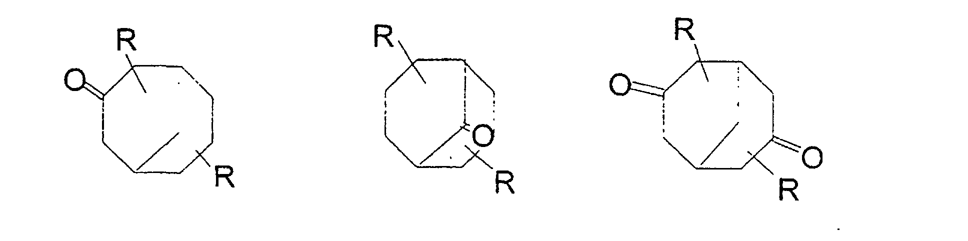 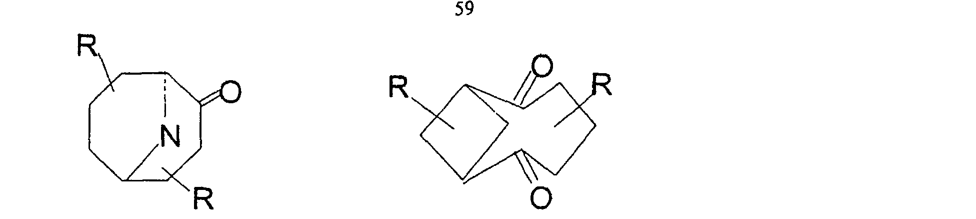 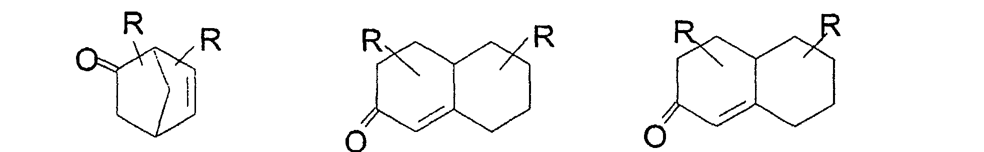 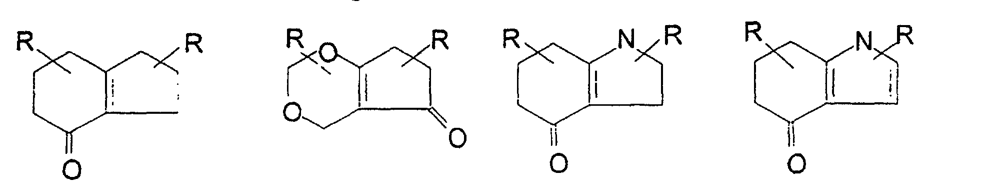 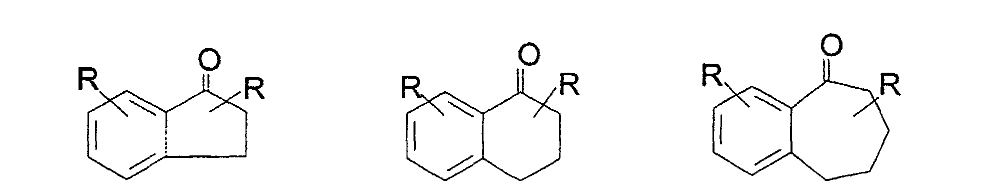 où R représente un phényle substitué ou non par des radicaux alkyle, alcoxy, ou un atome d'halogène ; ou R représente un groupement alkyle ou cycloalkyle substitué ou non par des radicaux alkyle, alcoxy, ou un atome d'halogène, un groupe hydroxyle, éther, amine ; ou R représente un atome d'halogène, un groupe hydroxyle, éther, amine.

On peut également mettre en oeuvre des cétones de type stéroïde (par exemple 3-cholestanone, 5-cholesten-3-one).

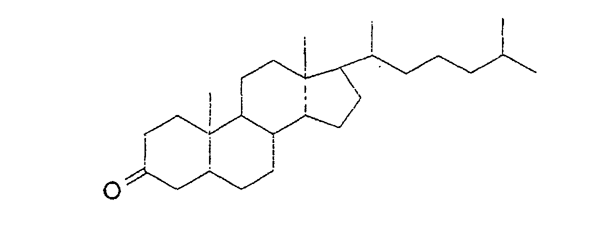

Comme autres substrats cétoniques, on peut citer les composés de formule B2 : 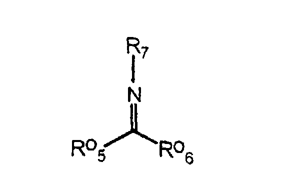 dans laquelle :
- R^{o}₅ différent de R^{o}₆ ont la signification donnée précédemment,
- R₇ représente :
   - un atome d'hydrogène,
   - un groupe hydroxyle,
   - un groupe OR₁₇,
   - un radical hydrocarbone R₁₇,
   - un groupe de formule 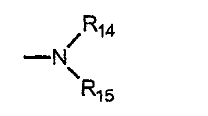
   - un groupe de formule 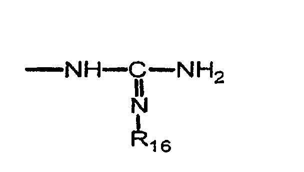
avec R₁₄, R₁₅, R₁₆ et R₁₇ qui représentent un atome d'hydrogène ou un groupement hydrocarboné ayant de 1 à 30 atomes de carbone.

Des exemples de composés de formule B2 sont les :
→ N-alkylcétoimine, tels que :
   - N-isobutyl-2-iminopropane
   - N-isobutyl-1-méthoxy-2-iminopropane
→ N-arylalkylcétoimine, tels que :
   - N-benzyl-1-imino-1-(phényl)éthane
   - N-benzyl-1-imino-1-(4-méthoxyphényl)éthane
   - N-benzyl-1-imino-1-(2-méthoxyphényl)éthane
→ N-arylcétoimine, tels que :
   - N-phényl-2-iminopentane
   - N-(2,6-diméthylphényl)-2-iminopentane
   - N-(2,4,6-triméthylphényl)-2-iminopentane
   - N-phényl-1-imino-1-phényléthane
   - N-phényl-1-méthoxy-2-iminopropane
   - N-(2,6-diméthylphényl)-1-méthoxy-2-iminopropane
   - N-(2-méthyl-6-éthylphényl)-1-méthoxy-2-iminopropane
→ les composés de type hydrazone éventuellement N-acylés ou N-benzoylés :
   - 1-cyclohexyl-1-(2-benzoylhydrazono)éthane,
   - 1-phényl-1-(2-benzoylhydrazono)éthane,
   - 1-p-méthoxyphényl-1-(2-benzoylhydrazono)éthane,
   - 1-p-éthoxyphényl-1-(2-benzoylhydrazono)éthane,
   - 1-p-nitrophényl-1-(2-benzoylhydrazono)éthane,
   - 1-p-bromophényl-1-(2-benzoylhydrazono)éthane,
   - 1-p-carboéthoxyphényl-1-(2-benzoylhydrazono)éthane,
   - 1,2-diphényl-1-(2-benzoylhydrazono)éthane,
   - 3-méthyl-2-(2-p-diméthylaminobenzoylhydrazono)butane,
   - 1-phényl-1-(2-p-méthoxylbenzoylhydrazono)éthane,
   - 1-phényl-1-(2-p-diméthylaminobenzoylhydrazono)éthane,
   - éthyl-2-(2-benzoylhydrazono)propionate
   - méthyl-2-(2-benzoylhydrazono)butyrate
   - méthyl-2-(2-benzoylhydrazono)valérate
   - méthyl-2-phényl-2-(2-benzoylhydrazono)acétate.

D'autres substrats de départ sont les semi-carbazones et les cétoimines cycliques à liaison endo ou exocycliques, telles que :

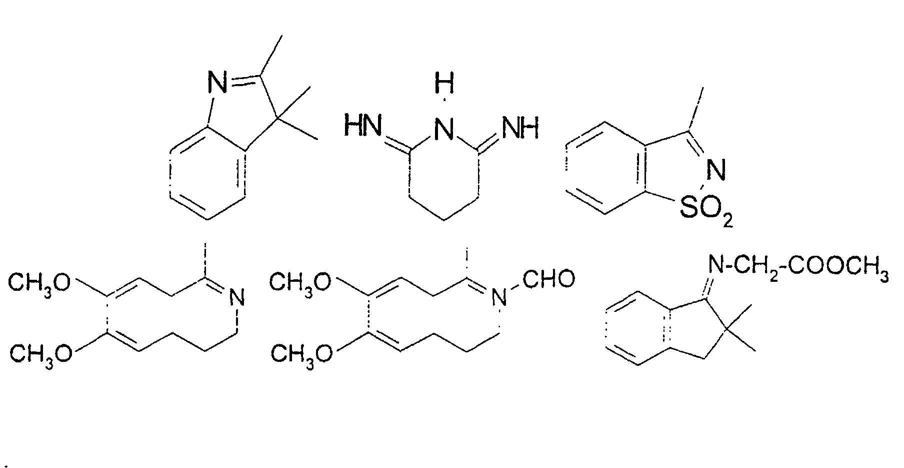

Selon un mode de réalisation préféré de l'invention, le substrat est une cétone aromatique (la phénylméthylcétone, la naphtylméthylcétone, la (p-méthoxyphényl)méthylcétone, la (p-trifluorométhylphényl)méthylcétone et la (o-méthylphényl)méthylcétone), un α-cétoester (tel que le benzoylformiate de méthyle et le pyruvate de méthyle), un β-cétoester (tel que l'acétoacétate de méthyle et le 3-oxovalérate de méthyle), un ester α,β-éthylénique (tel qu'un ester de l'acide tiglique et de l'acide itaconique), ou un aminoacide insaturé ou un de ses dérivés (tels que le 2-acétamidoacrylate de méthyle).

Ainsi, selon un autre de ses aspects, l'invention concerne l'utilisation d'un polymère de l'invention pour la préparation d'un complexe métallique destiné à la catalyse asymétrique, et plus spécialement d'un complexe du ruthénium, de l'iridium ou du rhodium.

Selon un mode de réalisation préféré de l'invention, les complexes métalliques sont destinés à la catalyse asymétrique des réactions d'hydrogénation de liaisons C=O et de liaisons C=C.

L'invention concerne par ailleurs l'utilisation de l'association d'un polymère optiquement actif selon l'invention avec une diamine, chirale ou non, pour la réduction sélective de cétones.

Les diamines utilisables à cet effet sont les diamines optiquement actives décrites dans WO 97/20789 et les diamines racémiques correspondantes.

Selon un mode de réalisation particulièrement préféré de l'invention, la diamine est le 1,2-diamino-1,2-diphényléthane ; le 1,1-bis(p-méthoxyphényl)-2-méthyl-1,2-diaminoéthane ; le 1,1-bis(p-méthoxyphényl)-2-isobutyl-1,2-diaminoéthane ; ou le 1,1-bis(p-méthoxyphényl)-2-isopropyl-1,2-diaminoéthane.

Des exemples de diamines chirales sont plus particulièrement celles de formule : 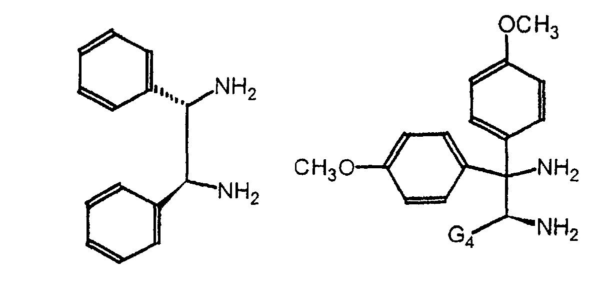 dans laquelle G₄ est alkyle, par exemple méthyle, isobutyle ou isopropyle.

Les cétones pouvant être réduites selon ce procédé sont celles décrites ci-dessus.

Les conditions de mise en oeuvre de la réduction sont celles généralement décrites ci-dessus.

L'invention concerne en outre l'utilisation de l'association d'un polymère racémique selon l'invention avec une diamine chirale, pour la réduction sélective de cétones.

La diamine chirale utilisable est telle que décrit dans WO 97/20789, les cétones et les conditions opératoires étant telles que définies ci-dessus.

### PREPARATION 1

### Préparation du (S)-6,6'-dibromo-2,2'-dihydroxy-1,1'-binaphtyle

7,7 g (26,9 moles) de (S)-2,2'-dihydroxy-1,1'-binaphtyle sont dissous dans 145 ml de dichlorométhane. La solution est refroidie à -75° C puis 3,66 ml de Br₂ (71,7 mmoles) sont ajoutés goutte à goutte pendant 30 minutes sous agitation constante. La solution est agitée 2 heures et demie de plus avant d'être ramenée à température ambiante. Après ajout de 180 ml de bisulfite de sodium (10% massique), la phase organique est lavée par une solution de NaCI saturée et séchée sur Na₂SO₄. Après évaporation du solvant, le solide obtenu est recristallisé dans un mélange toluène/cyclohexane) à 80° C pour donner 9,8 g (22 mmoles, 82% de rendement) de produit attendu.

Le pouvoir rotatoire tel que mesuré sur un polarimètre Perkin-Elmer-241 (I = 10 cm, 25° C, concentration c en g/dm³) est de 124,3 à c = 1,015 et 578 nm.

Pour la préparation du dérivé dibromé du titre, on pourra se reporter à G. Dotsevi et al., J. Am. Chem. Soc., 1979, 101, 3035.

### PREPARATION 2

### Préparation du (S)-6,6'-dibromo-2,2'-bis(trifluorométhanesulfonyloxy)-1,1'-binaphtyle

9,52 g (21,4 mml) de (S)-6,6'-dibromo-2,2'-dihydroxy-1,1'-binaphtyle sont dissous dans un mélange de 40 ml de CH₂Cl₂ et 5,4 ml de pyridine. Après avoir refroidi le mélange à 0° C, 8,7 ml (14,5 g, 51,5 mmol) d'anhydride triflique ((CF₃-SO₂)₂O) sont ajoutés lentement. Après agitation pendant 6 h, le solvant est évaporé et la masse réactionnelle est dissoute dans 100 ml d'acétate d'éthyle. Après avoir lavé avec une solution aqueuse d'HCI à 5%, une solution saturée de NaHCO₃ et une solution saturée de NaCI, la phase organique est séchée sur Na₂SO₄ puis le solvant est évaporé sous pression réduite. L'huile jaune est purifiée par chromatographie sur silice (CH₂Cl₂) pour donner 12,5 g (17,7 mmol, 83% rendement) de produit attendu.
[α]_{D} = 151,3 (c = 1,005, THF), le pouvoir rotatoire étant mesuré dans les mêmes conditions qu'à la préparation 1 mais à la longueur d'onde correspondant à la raie D du sodium.

Pour la préparation du composé du titre, on pourra se reporter également aux travaux de M. Vondenhof, Tetrahedron Letters, 1990, 31, 985.

### PREPARATION 3

### Préparation du (S)-6,6'-dibromo-2,2'-bis(trifluorométhanesulfonyloxy)-1,1'-binaphtyle

En variante, le composé du titre peut être préparé à partir du (R)-6,6'-dibromo-2,2'-dihydroxy-1,l'-binaphtyle en suivant le mode opératoire décrit ci-après.

10,0 g (22,52 mmol) de (R)-6,6'-dibromo-2,2'-dihydroxy-1,1'-binaphtyle sont dissous dans une solution de 6,3 g (0,11 mol de KOH dans 300 ml d'eau dégazée. Le mélange est refroidi à 0° C et ensuite une solution de 11,4 ml (19,1 g, 68 mmol) d'anhydride triflique dans 200 ml de CCl₄ est ajoutée pendant 45 minutes de façon à ce que la température ne dépasse pas 10° C. Après avoir agité pendant 30 min, 300 ml de CH₂Cl₂ sont ajoutés. La phase organique est lavée à l'eau puis séchée sur MgSO₄. 15,89 g de produit brut sont ensuite purifiés par chromatographie sur silice (CH₂Cl₂:cyclohexane 1:1) pour donner 12,94 g (18,27 mmol, 81% rendement) de produit pur.
[α]_{D} = -153,2° (c=0,945, THF), le pouvoir rotatoire étant mesuré dans les mêmes conditions qu'à la préparation 1 mais à la longueur d'onde correspondant à la raie D du sodium.
RMN ¹H (CDCl₃, 200 MHz) : δ (ppm) : 7,07 (d (J_{H-H} = 7,07), CH, 2H) ; 7,48 (dd (J¹_{H-H} = 9,05 ; J²_{H-H} = 1,94), CH, 2H) ; 7,62 (d (J_{H-H} = 9,11), CH, 2H) ; 8,06 (d (J_{H-H} = 9,13), CH, 2H) ; 8,18 (d (J_{H-H} = 1,90), CH, 2H).
RMN ¹³C (CDCl₃, 200 MHz) : δ (ppm) = 118,1 (Cq (J_{C-F} = 320)) ; 120,2 (Cq) ; 120,7 (CH) ; 122,0 (Cq) ; 123,4 (Cq) ; 128,2 (CH) ; 130,5 (CH) ; 131,4 (CH) ; 131,6 (Cq) ; 131,7 (CH) ; 133,4 (Cq).

### PREPARATION 4

### Préparation du (S)-6,6'-dicyano-2,2'-bis(trifluorométhane sulfonyloxy)-1,1'-binaphtyle

12,5 g (17,7 mmol) du composé préparé à la préparation 2 et 3,5 g (38,8 mmol) de CuCN sont agités à 180° C dans 20 ml de N-méthyl-pyrrolidone pendant 4 h. Après avoir refroidi à température ambiante, la suspension noire est versée dans une solution de 15 ml de diaminoéthane dans 35 ml d'eau. La solution est extraite plusieurs fois avec 30 ml de CH₂Cl₂, la phase organique est lavée avec une solution aqueuse à 10% de KCN, et une solution saturée de NaCI. Après séchage sur Na₂SO₄, le solvant est évaporé sous pression réduite. L'huile noire ainsi obtenue est purifiée par chromatographie sur silice (CH₂Cl₂:cyclohexane 9:1) pour donner 6,5 g (10,8 mmol, 61% rendement) de produit pur.
[δ]_{D} = 171,7 (c=1,15, THF), le pouvoir rotatoire étant mesuré dans les mêmes conditions qu'à la préparation 1 mais à la longueur d'onde correspondant à la raie D du sodium.
RMN ¹H (CDCl₃, 200 MHz) : δ (ppm) = 7,30 (d(J_{H-H} = 9,81), CH, 2H) ; 7,59 (dd(J¹_{H-H} = 8,82, J²_{H-H} = 1,65), CH, 2H) ; 7,78 (d(J_{H-H} = 9,11), CH, 2H) ; 8,29 (d(J_{H-H} = 8,09), CH, 2H) ; 8,46 (d(J_{H-H} = 1,29), CH, 2H).
RMN ¹³C (CDCl₃, 200 MHz) : δ (ppm) = 111,7 (CN) ; 118,0 (Cq) ; 118,1 (Cq(J_{C-F} = 320)); 121,6 (CH); 123,3 (Cq); 127,7 (CH) ; 128,9 (CH); 131,4 (Cq); 133,2 (CH); 134,4 (Cq); 134,5 (CH); 147,4 (Cq).

Pour la préparation du composé du titre, l'homme du métier pourra se reporter aux travaux de Friedman & coll., J. Org. Chem. 1961, 26, 2522 et M.S. Neuman & coll., J. Org. Chem., 1961, 26, 2525.

### PREPARATION 5

### Préparation du (S)-6,6'-dicyano-2,2'-bis(diphénylphosphino)-1,1'-binaphtyle

Dans un ballon tricol de 100 ml surmonté d'une arrivée d'argon, une solution de NiCl₂dppe (371 mg, 0,7 mmoles) et de diphénylphosphine (3ml, 17 mmoles) dans 14 ml de DMF (anhydre et dégazé) est chauffée pendant 30 minutes à 100° C. Le (S)-6,6'-dicyano-2,2'-bis(trifluorométhanesulfonyfoxy)-1,1'-binaphtyle (4,4 g, 7,4 mmoles) et le DABCO (3,375 g, 30 mmoles) dissous dans 20 ml de DMF sont ajoutés goutte à goutte. Le milieu réactionnel est laissé à 100° C. Au bout de 1,3 et 7 heures, on ajoute 0,75 ml de diphénylphosphine. La solution est laissée sous agitation pendant 2 jours. Elle est ensuite refroidie à 0° C, puis filtrée sous argon, lavée au méthanol (2 x 10 ml). Le solide est enfin séché sous vide pour fournir le produit attendu avec un rendement de 50 %.

| Analyse élémentaire pour C₄₆H₃₀N₂P₂ | | | | |
|---|---|---|---|---|
| calculée | C = 80,88 ; | H = 4,43 ; | N = 4,10 ; | P = 9,07 ; |
| trouvée | C = 81,61 ; | H = 4,45 ; | N = 4,11 ; | P = 8,99. |

RMN ¹H (CDCl₃, 200 MHz) δ (ppm) : 6,59 (d, 2H, CH) ; 6,87 (dd, 2H, CH) ; 6,92-6,99 (m, 4H, CH) ; 7,09 (t, 4H, CH) ; 7,17-7,31 (m, 12H, CH) ; 7,57 (d, 2H, CH); 7,95 (d, 2H, CH) ; 8,20 (s, 2H, CH).
RMN ¹³C (CDCl₃, 50 MHz) δ (ppm) : 109,8 (CN) ; 119,0 (Cq) ; 126,3 (CH) ; 127,7 (CH) ; 128,4 (CH) ; 128,5 (CH) ; 128,5 (CH) ; 128,6 (CH) ; 128,8 (CH) ; 129,3 (CH) ; 132,0 (CH) ; 132,1 (Cq) ; 132,9 (CH(triplet J_{C-P} = 11,7) ; 133,9 (Cq) ; 134,1 (Cq) ; 134,9 (CH(triplet J_{C-P} = 9,9)) ; 136,8 (Cq) ; 140,6 (Cq).
RMN ³¹P (CDCl₃, 81 MHz) δ (ppm) : -12,75.

### PREPARATION 6

### Préparation du (S)-6,6'-bis(aminométhyl)-2,2'-bis(diphénylphosphino)-1,1'-binaphtyle

Dans un ballon de 250 ml placé sous atmosphère d'argon, on dissout 557 mg (14,7 mmoles) de LiAlH₄ dans un mélange de THF (30 ml)/toluène (60 ml). Le (S)-6,6'-dicyano-2,2'-bis(diphénylphosphino)-1,1'-binaphtyle (650 mg, 0,97 mmoles) est ajouté à cette solution qui est agitée et portée à reflux pendant 4 heures. Elle est ensuite refroidie à 0° C. On ajoute 600 µl d'eau et 600 µl de NaOH à 15 %. Puis 2 g de célite sont rajoutés et le mélange est filtré sur millipore sous argon. 60 ml de dichlorométhane sont ajoutés, le mélange est agité et à nouveau filtré. Cette opération est effectuée trois fois. La phase organique obtenue est lavée avec une solution aqueuse saturée en NaCl puis séchée sur Na₂SO₄. Le solvant est évaporé pour obtenir un solide jaune (657 mg, rendement quantitatif) caractérisé par RMN (proton, carbone et phosphore) correspondant à la structure attendue.

| Analyse élémentaire pour C₄₆H₃₈N₂P₂ | | | | |
|---|---|---|---|---|
| calculée | C = 80,59 ; | H = 6,00 ; | N = 3,55 ; | P = 7,84 ; |
| trouvée | C = 81,14 ; | H = 5,51 ; | N = 3,13 ; | P = 7,90. |

RMN ¹H (CDCl₃, 200 MHz) δ (ppm) : 1,68 (s, 4H, NH₂) ; 3,81 (s, 4H, CH₂) ; 6,72 (s, 4H, CH) ; 6,9-7,3 (m, 20H, CH) ; 7,33 (d, 2H, CH) ; 7,64 (s, 2H, CH) ; 7,76 (d, 2H, CH).
RMN ³¹P (CDCl₃, 81 MHz) δ (ppm) : -15,08.

### EXEMPLE 1

### Préparation d'une polyurée au départ du (S)-6,6'-bis(aminométhyl)-2,2'-bis(diphénylphosphino)-1,1'-binaphtyle.

### a) Polyurée 1

Dans un ballon de 10 ml, le (S)-6,6'-bis(aminométhyl)-2,2'-bis(diphénylphosphino)-1,1'-binaphtyle (200 mg, 0,29 mmoles) est dissous dans 2 ml de dichlorométhane dégazé et le 2,6-diisocyanato-tolylène (51 mg, 0,29 mmoles) est ajouté sous argon. La solution est agitée pendant 12 h. Ensuite, 2 ml d'isopropanol dégazé sont ajoutés. Enfin, le solide est récupéré par filtration sur millipore et lavé à l'isopropanol. On obtient 238 mg de polymère, soit un rendement de 95 %.

Les données physico-chimiques de caractérisation sont les suivantes :
[α_{D}] = -96° C ( c=0,345 DMF) ;
¹H RMN (DMSO, 200 MHz) : 2,04 ppm (CH₃ des groupes tolyle) : 4,36 ppm (CH benzéniques) ; 1,25 ppm (CH₃ isopropyliques, les extrémités des chaînes ayant été désactivées par de l'isopropanol) ;
³¹P RMN (DMSO, 81 MHz) : -15,77 ppm ;

| Analyse élémentaire (pour 8 monomères) | | | | |
|---|---|---|---|---|
| calculée | C = 79,06 ; | H = 4,73 ; | N = 5,98 ; | P = 6,22 |
| trouvée | C = 74,98 ; | H = 5,43 ; | N = 5,90 ; | P = 6,52. |

### b) Polyurée 2

A une solution de 200 mg (0,29 mmol) de (S)-6,6'-bis(aminométhyl)-2,2'-bis(diphénylphosphino)-1,1'-binaphtyle dans 4 ml de CH₂Cl₂ sont ajoutés 42 µl (0,29 mmol) de 2,6-diisocyanato tolylène. La suspension est agitée sous argon pendant la nuit puis 1 ml d'isopropanol est ajouté. Après agitation pendant 2 h, la suspension est filtrée et le solide est lavé deux fois avec 2 ml d'i-PrOH et deux fois avec 2 ml de CH₂Cl₂. Après séchage sous vide, 224 mg d'une poudre jaune sont obtenus (93% de rendement).
[α]_{D} : -96 (c = 0,345, DMF) ;
¹H RMN (DMSO) : δ = 1,21 (d, CH₃) ; 2,04 (s, CH₃) ; 4,36 (s, CH₂) ; 6,64 (d, CH) ; 6,94 (s, CH); 7,20 (s, CH) ; 7,32 (s, CH) ; 7,81 (s, CH) ; 7,93 (s, CH);
³¹P RMN (DMSO) : δ = -15,77;

| Analyse élémentaire : | | | | | |
|---|---|---|---|---|---|
| Calculé pour C₇₄₅H₅₅₀O₂₈N₅₀P₂₄ | C : 78,52 | H : 4,87 | N : 6,15 | P : 6,53 | O : 3,93 |
| Trouvé | C : 74,98 | H : 5,43 | N : 5,90 | P : 6,52 | O : 5,72 |

### EXEMPLE 2

### Préparation d'un catalyseur au ruthénium au départ du polymère préparé à l'exemple 1.

Le catalyseur se prépare in situ. Tous les solvants utilisés ont été soigneusement dégazés et sont anhydres. Le milieu réactionnel est maintenu sous atmosphère d'argon. Dans un réacteur en verre à fond conique de 5 ml sorti de l'étuve et muni d'un agitateur, sont directement pesés le polymère et le précatalyseur métallique, le bis-(2-méthylallyl)cycloocta-1,5-diène ruthénium, dans un rapport molaire polymère/métal de 1:1. Le réacteur est fermé par un septum et l'air est chassé par une arrivée d'argon. De l'acétone est alors ajoutée de façon à obtenir une suspension blanche contenant 0,006 mol/l de polymère. Cette suspension est agitée 30 minutes puis une solution méthanolique de HBr à 0,29 M est ajoutée (rapport Ru/Br de 1/2,3). On observe alors un changement de couleur de la solution qui vire au brun. Cette solution est encore agitée durant 1 heure puis le solvant est évaporé. On obtient alors le catalyseur sous l'aspect d'un solide marron.

Le catalyseur obtenu est désigné catalyseur de l'exemple 2a), lorsque le polymère de départ est la polyurée 1 et le catalyseur obtenu est désigné 2b, lorsque le polymère de départ est la polyurée 2.

### EXEMPLE 3

Cet exemple illustre l'hydrogénation d'α-cétoesters en présence du complexe du ruthénium préparé à l'exemple 2.

Le protocole d'hydrogénation est décrit ci-dessous :

Le méthanol qui a été préalablement séché sur magnésium est ajouté (2,5 ml) sous argon dans le réacteur conique où le catalyseur vient d'être préparé. Le substrat est ensuite ajouté (dans un rapport catalyseur/substrat défini). L'opération consistant à faire le vide et à remplir le réacteur d'argon est répétée trois fois. Le septum est alors remplacé par un bouchon percé puis le réacteur est placé dans un autoclave. L'autoclave est purgé trois fois sous argon puis trois fois sous hydrogène avant de recevoir 40 bars de pression d'hydrogène. L'autoclave est placé sur une plaque chauffante (50° C) et l'agitation est maintenue durant la nuit. Le réacteur conique est enfin récupéré, le bouchon est remplacé par un septum, et l'argon est réinjecté dans ce réacteur. Le réacteur est mis dans une centrifugeuse, puis la solution est extraite à l'aide d'une seringue. Elle est placée dans un ballon de 50 ml et diluée dans 20 ml de méthanol, prête alors à être injectée dans une colonne de chromatographie pour chromatographie en phase gazeuse pour analyse de l'activité et de l'énantiosélectivité de la réaction.

L'hydrogénation a d'abord été mise en oeuvre en sélectionnant le pyruvate de méthyle comme substrat. Puis l'hydrogénation a été réalisée en choisissant le benzoylformiate de méthyle comme substrat.

La détermination des excès énantiomériques est effectuée par chromatographie en phase gazeuse chirale sur une colonne de type Macheray-Nagel (Lipodex A 25 m x 0,25 mm pour le pyruvate de méthyle et Lipodex E 25 x 0,25 mm pour le benzoylformiate de méthyle).

Les résultats obtenus sont rapportés dans le tableau 1 suivant :

**TABLEAU 1**

| Substrat | Catalyseur | Taux de conversion (%) | Excès énantiomérique (%) |
|---|---|---|---|
| Pyruvate de méthyle | Exemple 2a | 82 | 46 |
| Benzoylformiate de méthyle | Exemple 2a | 91 | 26 |

Dans ces exemples, le rapport molaire substrat/catalyseur était fixé à 1000.

L'hydrogénation du benzoylformiate de méthyle conduit au 2-phényl-2-hydroxy-éthanoate de méthyle et l'hydrogénation du pyruvate de méthyle conduit au 2-hydroxy-propanoate de méthyle.

### EXEMPLE 4

Cet exemple illustre la réutilisation du catalyseur polymérique dans l'hydrogénation d'un β-cétoester.
a) Hydrogénation d'un β-cétoester en présence de catalyseur fraichement préparé.
   Le catalyseur utilisé est celui de l'exemple 2a.
   Le protocole opératoire d'hydrogénation est tel que décrit à l'exemple 3, sinon que l'on utilise comme substrat l'acétoacétate de méthyle.
   Les résultats obtenus figurent au tableau 2 ci-dessous, étant entendu que la méthode de détermination des excès énantiomériques est celle décrite à l'exemple 3.
b) Réutilisation du catalyseur dans l'hydrogénation d'un β-cétoester.

Après la réaction d'hydrogénation mise en oeuvre à l'étape a) ci-dessus, le catalyseur est filtré du milieu réactionnel et récupéré.

On ajoute alors sur le catalyseur solide une solution identique du β-cétoester dans le méthanol. L'hydrogénation est alors conduite comme décrit précédemment à l'étape a). Ce protocole est répété plusieurs fois.

Les résultats obtenus ont été recueillis dans le tableau 2 suivant.

**TABLEAU 2**

| Substrat | Catalyseur | Taux de conversion (%) | Excès énantiomérique (%) |
|---|---|---|---|
| Acétoacétate de méthyle | exemple 2a | 100 | 95 |
| Acétoacétate de méthyle | ex. 2a, récupéré après une 1ère utilisation | 100 | > 95 |
| Acétoacétate de méthyle | ex. 2a, récupéré après une 2ème utilisation | 100 | > 95 |
| Acétoacétate de méthyle | ex. 2a, récupéré après une 3ème utilisation | 100 | > 95 |
| Acétoacétate de méthyle | ex. 2a, récupéré après une 4ème utilisation | 100 | > 95 |

L'hydrogénation de l'acétoacétate de méthyle conduit au 3-hydroxybutanoate de méthyle.

On constate que les propriétés avantageuses du catalyseur sont conservées après de nombreuses réutilisations. De fait, l'excès énantiomérique, qui est même amélioré dès la première utilisation après récupération du catalyseur, se maintient au-dessus de 95 %, après 4 utilisations.

### EXEMPLE 5

### Préparation d'un catalyseur au ruthénium au départ du polymère préparé à l'exemple 1

Une solution de 5 mg (0,006 mmol) de polymère et 1,3 mg (0,003 mmol) de [RuCl₂(benzène)]₂ dans 1 ml de diméthylformamide est maintenue sous agitation à 100° C pendant 30 minutes. Après refroidissement à 50° C, le solvant est évaporé sous pression réduite. Après refroidissement à température ambiante, l'évaporation est poursuivie sous pression réduite (0,1 mbar) pour conduire à la formation d'un solide brun.

Le catalyseur obtenu est désigné catalyseur de l'exemple 5a lorsque le polymère utilisé est celui de l'exemple 1a et le catalyseur obtenu est désigné catalyseur de l'exemple 5b lorsque le polymère de départ utilisé est celui de l'exemple 1b.

### EXEMPLE 6

Cet exemple illustre l'hydrogénation de β-cétoesters.

Le protocole d'hydrogénation est tel que décrit à l'exemple 3 sinon que le substrat est choisi parmi le 3-oxovalérate de méthyle, l'acétoacétate de méthyle et le 3-oxo-6-octènoate de méthyle, le catalyseur utilisé étant soit le complexe préparé à l'exemple 2a, soit le complexe préparé à l'exemple 5a.

Les résultats obtenus ont été recueillis dans le tableau 3 suivant, ensemble avec les résultats rapportés dans la littérature dans le cas des réactions d'hydrogénation correspondantes utilisant comme catalyseur les complexes équivalents dérivés du BINAP.

L'hydrogénation de l'acétoacétate de méthyle conduit au 3-hydroxybutanoate de méthyle ; l'hydrogénation du 3-oxovalérate de méthyle conduit au 3-hydroxypentanoate de méthyle ; et l'hydrogénation du 3-oxo-6-octènoate de méthyle conduit à un mélange de 3-hydroxy-octanoate de méthyle et de 3-hydroxy-6-octènoate de méthyle.

**TABLEAU 3**

| Substrat | Complexe catalyseur | Rapport molaire substrat/catalyseur | Taux de conversion (%) | Excès énantiomérique |
|---|---|---|---|---|
| acétoacétate de méthyle | exemple 2a | 1000 | 100 | 94 |
| acétoacétate de méthyle | complexe du BINAP préparé selon le procotole opératoire de l'exemple 5 | 1150 | 100 | 99⁽¹⁾ |
| 3-oxovalérate de méthyle | exemple 2a | 1000 | 100 | 98 |
| 3-oxovalérate de méthyle | complexe du BINAP préparé selon le protocole opératoire de l'exemple 2 | 1200 | 100 | 99 ⁽²⁾ |
| 3-oxo-6-octènoate de méthyle | exemple 2a | 1000 | 90 | 90 |

| | | | | |
|---|---|---|---|---|
| ⁽¹⁾ Kitamura, M. ; Tokunaga, M. : Ohkuma, T. : Noyori R, *Tetrahedron Letters*, 32, 1991, 4163-4166 | | | | |
| ⁽²⁾ Genet et al., *Tetrahedron : Asymmetry*, 5, 1994, 675-690. | | | | |

### EXEMPLE 7

### Préparation d'un catalyseur au ruthénium au départ du polymère préparé à l'exemple 1

Une solution de 5 mg (0,006 mmole) de polymère et 1,3 mg (0,003 mmol) de [RuCl₂(benzène)]₂ dans 1 ml d'un mélange 1/8 de benzène/éthanol, est maintenue sous agitation, à 50° C pendant 2 heures. Après refroidissement à 20° C, le solvant est évaporé sous pression réduite. Un solide brun est obtenu après évaporation du solvant.

On distingue de la même façon que précédemment, le catalyseur de l'exemple 7a du catalyseur de l'exemple 7b suivant que le polymère de départ utilisé est celui de l'exemple la (polyurée 1) ou de l'exemple 1b (polyurée 2).

### EXEMPLE 8

Cet exemple illustre l'hydrogénation de dérivés d'aminoacides insaturés.

Le protocole d'hydrogénation est tel que décrit à l'exemple 3, sinon que l'on fait varier le catalyseur, le substrat, la pression d'hydrogène et le rapport substrat/catalyseur.

Le substrat est choisi parmi un composé de formule :

HN(Ac)-C(=CH₂)-CO-OR L1

dans laquelle R est H ou méthyle ; et
l'acétamidocinnamate de méthyle de formule : 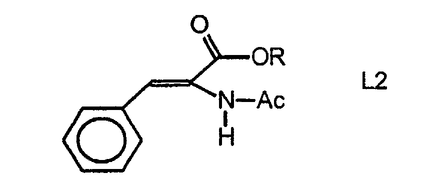 dans laquelle R est H ou méthyle.

Des essais comparatifs ont été réalisés à partir de catalyseurs équivalents dérivés du BINAP.

Le tableau 4 résume les résultats obtenus, étant entendu que la détermination de l'excès énantiomérique est effectuée par chromatographie en phase gazeuse chirale sur une colonne de type Macheray-Nagel (Lipodex A 25 m x 0,25 mm). L'hydrogénation des composés de formule L1 conduit aux composés correspondants de formule HN(Ac)-C(CH₃)-CO-OR et l'hydrogénation de l'acétamidocinnamate de méthyle conduit au 3-phényl-2-acétamidopropanoate de méthyle.

**TABLEAU 4**

| Substrat | Complexe catalyseur | Pression d'hydrogénation | Rapport molaire substrat/catalyseur | Taux de conversion (%) | Excès énantiomérique (%) |
|---|---|---|---|---|---|
| L1 (R = CH₃) | exemple 2b | 40 bar | 100 | 100 | 63 |
| L1 (R = CH₃) | complexe du BINAP préparé selon le protocole opératoire de l'exemple 2 | 40 bar | 100 | 100 | 67 |
| L1 (R = CH₃) | exemple 5b | 40 bar | 100 | 100 | 56 |
| L1 (R = CH₃) | complexe du BINAP préparé selon le protocole opératoire de l'exemple 5 | 40 bar | 100 | 100 | 78 |
| L1 (R = -CH₃) | exemple 2a | 40 bar | 1000 | 100 | 94 |
| L1 (R = -CH₃) | complexe du BINAP préparé selon la protocole opératoire de l'exemple 2 | 40 bar | 1000 | 100 | 63 |
| L1 (R = CH₃) | exemple 7b | 40 bar | 1000 | 100 | 74 |
| L1 (R = H) | exemple 2b | 40 bar | 200 | 100 | 50 |
| L1 (R = H) | exemple 5a | 10 bar | 100 | 95 | 70 |
| L1 (R = H) | exemple 5b | 40 bar | 100 | 100 | 70 |
| L1 (R = H) | complexe du BINAP préparé selon le protocole opératoire de l'exemple 5 | 10 bar | 100 | 95 | 78⁽¹⁾ |
| L1 (R = H) | complexe du BINAP préparé selon le protocole opératoire de l'exemple 2 | 40 bar | 200 | 100 | 69 |
| L1 (R = H) | complexe du BINAP préparé selon le protocole opératoire de l'exemple 5 | 40 bar | 100 | 100 | 78 |
| L2 ( R = H) | exemple 2b | 40 bar | 200 | 100 | 28 |
| L2 (R = H) | exemple 5b | 40 bar | 100 | 100 | 46 |
| L2 ( R = H) | complexe du BINAP préparé selon le protocole opératoire de l'exemple 5 | 40 bar | 100 | 100 | 74 |
| L2 (R = CH₃) | exemple 2b | 40 bar | 100 | 100 | 49 |
| L2 ( R = CH₃) | complexe du BINAP préparé selon le protocole opératoire de l'exemple 2 | 40 bar | 100 | 100 | 72 |
| L2 (R = CH₃) | exemple 5b | 40 bar | 100 | 100 | 72 |
| L2 (R = CH₃) | complexe du BINAP préparé selon le protocole opératoire de l'exemple 5 | 40 bar | 100 | 100 | 80 |
| L2 (R = CH₃) | exemple 7a | 40 bar | 100 | 100 | 72 |
| L2 (R = CH₃) | complexe du BINAP préparé selon le protocole opératoire de l'exemple 7 | 40 bar | 100 | 100 | 74 |

| | | | | | |
|---|---|---|---|---|---|
| ⁽¹⁾ L'excès énantiomérique rapporté dans Genet; J.P. ; Pinel, C. ; Ratovelomanana-Vidal, V. ; Mallart, S. ; Pfister, X.; Cano de Andrade, M. ; Laffitte, J.A. *Tetrahedron Asym*., 1994, 5, 665, est de 75 %. | | | | | |

### EXEMPLE 9 (COMPARATIF)

Dans cet exemple, on a réalisé l'hydrogénation d'un α-cétoester (le benzoylformiate de méthyle) et d'un dérivé d'aminoacide insaturé (le 2-acétamidoacrylate de méthyle) en utilisant comme catalyseur un complexe équivalent au complexe de l'exemple 2 mais comprenant le 2,2'-bis(diphénylphosphino)-1,1'-binaphtyle (BINAP), en tant que tel comme ligand.

Ce catalyseur est préparé par mise en oeuvre du protocole opératoire décrit à l'exemple 2, sinon qu'on utilise le 2,2'-bis(diphénylphosphino)-1,1'-binaphtyle au lieu du polymère, comme ligand.

Le protocole opératoire d'hydrogénation du benzoylformiate de méthyle et du 2-acétamidoacrylate de méthyle est tel que décrit à l'exemple 3, sinon qu'on remplace le catalyseur par le catalyseur dérivé du BINAP.

Les résultats obtenus figurent au tableau comparatif suivant.

| TABLEAU COMPARATIF | | | |
|---|---|---|---|
| Substrat | Catalyseur | Taux de conversion (%) | Excès énantiomérique (%) |
| Benzoylformiate de méthyle | BINAP | 98 | 82 |
| 2-acétamidoacrylate de méthyle | BINAP | 100 | 64 |

Dans ces exemples, le rapport molaire substrat/catalyseur est fixé à 1000.

### EXEMPLE 10

Cet exemple illustre l'hydrogénation de l'acide itaconique et de son diester méthylique.

Le protocole d'hydrogénation est tel qu'illustré à l'exemple 3, sinon que l'on fait varier la pression d'hydrogénation, le complexe catalyseur, le rapport molaire du substrat au catalyseur et la nature du substrat.

Le substrat utilisé a pour formule :

RO-CO-C(=CH₂)-CH₂-COOR L3

dans laquelle R est hydrogène ou méthyle et conduit, après hydrogénation, au composé correspondant de formule : RO-CO-C(CH₃)-CH₂-COOR.

Le tableau 5 rapporte les résultats obtenus en faisant varier la nature du catalyseur.

**TABLEAU 5**

| Substrat | Complexe catalyseur | Rapport molaire substrat/ catalyseur | Pression d'hydrogénation | Taux de conversion (%) | Excès énantiomérique (%) |
|---|---|---|---|---|---|
| L3 (R = CH₃) | exemple 5b ⁽³⁾ | 100 | 40 bar | 100 | 94 |
| L3 (R = CH₃) | complexe du BINAP préparé selon le protocole opératoire de l'exemple 5⁽³⁾ | 100 | 40 bar | 100 | 94 |
| L3 (R = -CH₃) | exemple 7a | 100 | 40 bar | 100 | 94 |
| L3 (R = -CH₃) | complexe dérivé du BINAP préparé à l'exemple 7 | 100 | 40 bar | 100 | >90 (68)⁽¹⁾ |
| L3 (R = H) | exemple 5b ⁽³⁾ | 100 | 40 bar | 100 | 71 |
| L3 (R = H) | complexe du BINAP préparé selon le protocole opératoire de l'exemple 5 ⁽³⁾ | 100 | 40 bar | 100 | 88 |
| L3 (R = H) | exemple 7a | 100 | 10 bar | 100 | 71 |
| L3 (R = H) | complexe dérivé du BINAP préparé à l'exemple 7 | 100 | 10 bar | 100 | 82 (98) ⁽²⁾ |

| | | | | | |
|---|---|---|---|---|---|
| ⁽¹⁾ le chiffre entre parenthèses rapporte le résultat obtenu dans Kawano, H. ; Ikariya, T. : Ishii, Y. ; Saburi, M. ; Yoshikawa, S. ; Uchida, Y. ; Kumobayashi, *J. Chem. Soc. Perk. Trans., I*., 1989, 1571. | | | | | |
| ⁽²⁾ le chiffre entre parenthèses rapporte le résultat obtenu dans Genet, J.P. ; Pinel, C. ; Ratovelomanana-Vidal V. ; Mallart, S. ; Pfister, X. ; Cano de Andrade, M. ; Laffitte, J.A. *Tetrahedron Asym.* 1994, 5, 685. | | | | | |
| ⁽³⁾ le complexe utilisé a été préparé à 110° C au lieu de 100° C. | | | | | |

### EXEMPLE 11

Cet exemple illustre l'hydrogénation de l'acide tiglique de formule CH₃-CH=C(CH₃)-COOH sous une pression d'hydrogène de 40 bar.

Le protocole d'hydrogénation est le même qu'à l'exemple 3, sinon que le substrat est l'acide tiglique, et que l'on fait varier le catalyseur.

Les conditions réactionnelles ainsi que les résultats obtenus sont recueillis dans le tableau 6 suivant, le produit d'hydrogénation étant le CH₃-CH₂-Ch(CH₃)-COOH.

**TABLEAU 6**

| Complexe catalyseur | Rapport molaire substrat/ catalyseur | Taux de conversion (%) | Excès énantiomérique (%) |
|---|---|---|---|
| exemple 7a | 1000 | 100 | 82 |
| complexe dérivé du BINAP et préparé comme à l'exemple 2 | 1000 | 100 | 80 (90)⁽¹⁾ |

| | | | |
|---|---|---|---|
| ⁽¹⁾ le chiffre entre parenthèses rapporte le résultat obtenu dans Genet, J.P. ; Pinel, C. ; Ratvelomanana-Vidal, V. ; Mallart S. ; Pfister, X. ; Cano de Andrade, M. ; Laffitte, J.A. *Tetrahedron Asym.* 1994, 5, 665. | | | |

### EXEMPLE 12

### Préparation d'un catalyseur au ruthénium au départ du polymère préparé à l'exemple 1

Dans un premier temps, on procède comme à l'exemple 7. Puis, le complexe résultant est dissous dans 1 ml de diméthylformamide et 2,5 mg (1 éq.) de (S,S)1,2-diphényléthylènediamine ((S,S)-DPEDA) ou de (R,R)-1,2-diphényléthylènediamine ((R,R)-DPEDA)) sont ajoutés. La solution est agitée pendant 2 heures. Le solvant est évaporé sous pression réduite et le complexe est utilisé tel quel.

On distingue le catalyseur de l'exemple 12a du catalyseur de l'exemple 12b suivant que le polymère de départ utilisé est le polymère de l'exemple 1a (polyurée 1) ou le polymère de l'exemple 1b (polyurée 2).

### EXEMPLE 13

Cet exemple illustre l'hydrogénation de diverses cétones aromatiques.

Le protocole d'hydrogénation est identique à celui de l'exemple 3, sinon que l'on utilise comme solvant une solution de 2,9 mg de tert-butylate de potassium dans 0,75 ml (0,0348 mol/l) d'isopropanol (en lieu et place du méthanol), et qu'on ajoute dans chaque cas 5,8 mmol de substrat.

Les résultats obtenus sont résumés dans le tableau suivant, étant entendu que dans chaque cas le produit d'hydrogénation est l'alcool correspondant.

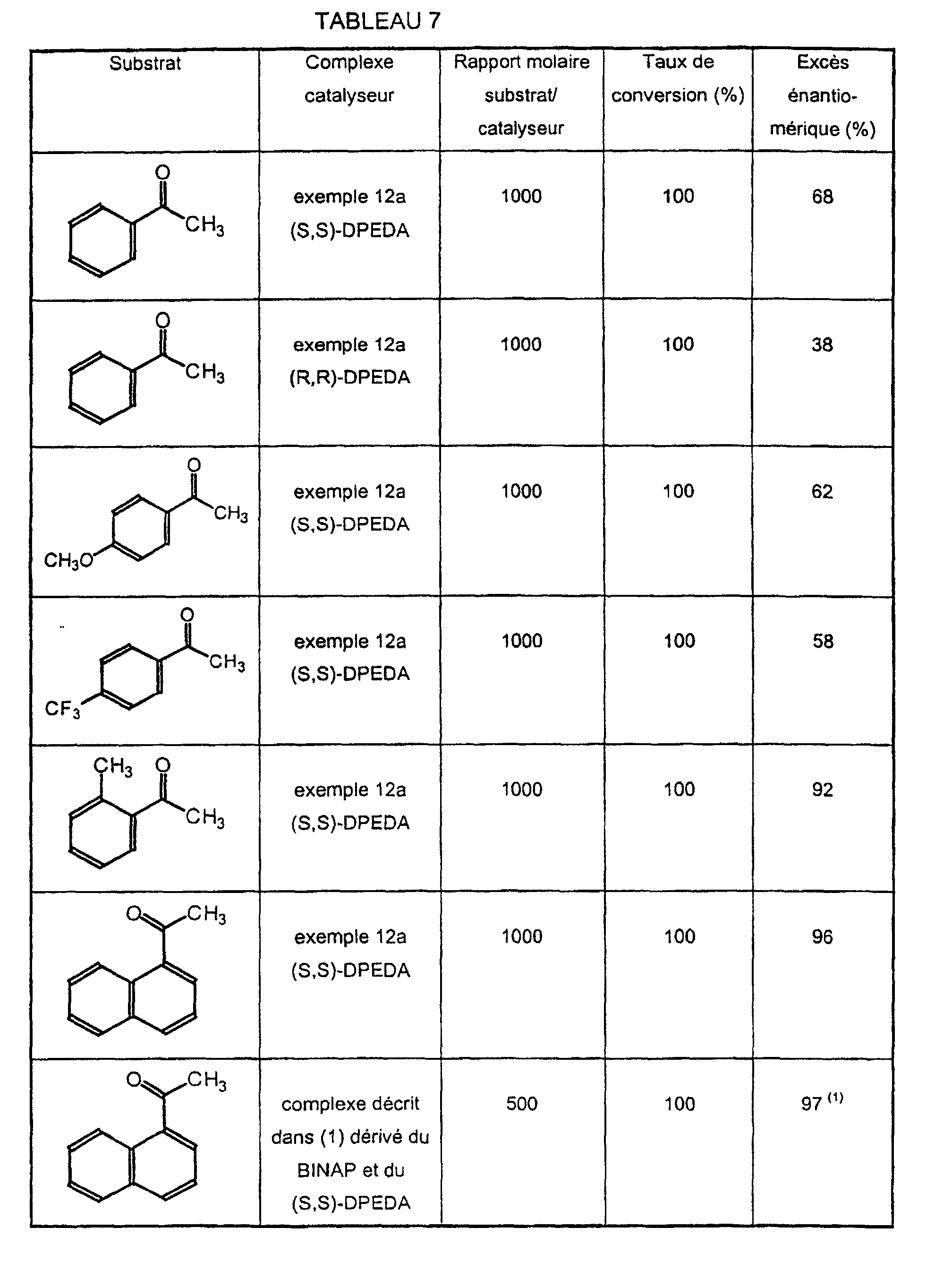

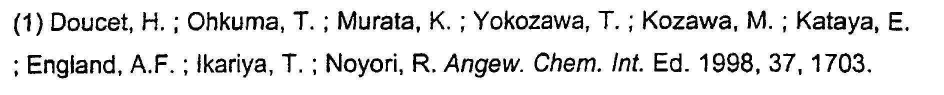

### EXEMPLE 14

Cet exemple illustre la réutilisation du catalyseur polymérique dans l'hydrogénation d'une cétone aromatique.

Le catalyseur utilisé est celui de l'exemple 12a obtenu à partir de la (S,S)-diphénylethylènediamine. Le protocole opératoire utilisé est celui de l'exemple 13, sinon que l'on utilise comme substrat la phénylméthylcétone. La réutilisation du complexe catalyseur est mise en oeuvre comme illustré à l'exemple 4.

Les résultats sont rapportés dans le tableau 8 ci-dessous, l'hydrogénation conduisant dans chaque cas au 1-phényléthanol.

**TABLEAU 8**

| Complexe catalyseur | Taux de conversion (%) | Excès énantiomérique (%) |
|---|---|---|
| exemple 12a (S,S)-DPEDA | 100 | 68 |
| exemple 12a (S,S)-DPEDA récupéré après une première utilisation | 100 | 67 |
| exemple 12a (S,S)-DPEDA récupéré après une deuxième utilisation | 100 | 60 |
| exemple 12a (S,S)-DPEDA récupéré après une troisième utilisation | 100 | 61 |
| exemple 12a (S,S)-DPEDA récupéré après une quatrième utilisation | 36 | 61 |

### EXEMPLE 15

Cet exemple illustre l'hydrogénation d'α-cétoesters de formule :

R-CO-CO-OCH₃

par mise en oeuvre du protocole d'hydrogénation de l'exemple 3 sous une pression d'hydrogène de 40 bar, en faisant varier la nature du catalyseur ainsi que le rapport substrat/catalyseur.

Le produit obtenu à l'issue de l'hydrogénation est R-CH(OH)-COOCH₃.

Des essais comparatifs ont été réalisés à partir de catalyseurs équivalents dérivés du BINAP.

Le tableau 9 résume les résultats obtenus, la détermination de l'excès énantiomérique étant effectuée par chromatographie en phase gazeuse comme précédemment.

**TABLEAU 9**

| Substrat | Complexe catalyseur | Rapport molaire substrat/catalyseur | Taux de conversion (%) | Excès énantiomérique (%) |
|---|---|---|---|---|
| R = CH₃ | exemple 7b | 100 | 100 | 64 |
| R = CH₃ | complexe du BINAP préparé selon le protocole opératoire de l'exemple 7 | 100 | 100 | 67 |
| R = C₆H₅ | exemple 7b | 100 | 64 | 66 |
| R = C₆H₅ | complexe du BINAP préparé selon le protocole opératoire de l'exemple 7 | 100 | 93 | 75 |

### EXEMPLE 16

Cet exemple illustre l'hydrogénation de β-cétoesters de formule :

R-CO-CH₂-CO-OCH₃

par mise en oeuvre du protocole d'hydrogénation de l'exemple 3 sous une pression d'hydrogène de 40 bar, en faisant varier la nature du catalyseur, la nature du substrat et le rapport molaire substrat sur catalyseur.

Le produit obtenu à l'issue de l'hydrogénation est le composé correspondant de formule R-CH(OH)-CH₂-CO-OCH₃. Des essais comparatifs ont été réalisés à partir de catalyseurs équivalents dérivés du BINAP.

Le tableau 10 résume les résultats obtenus, la détermination de l'excès énantiomérique étant effectuée par chromatographie en phase gazeuse comme précédemment.

**TABLEAU 10**

| Substrat | Complexe catalyseur | Rapport molaire substrat/ catalyseur | Taux de conversion (%) | Excès énantiomérique (%) |
|---|---|---|---|---|
| R = CH₃ | exemple 5b ⁽¹⁾ | 1000 | 100 | 98 |
| R = CH₃ | complexe du BINAP préparé selon le protocole opératoire de l'exemple 5⁽¹⁾ | 1000 | 100 | 99 |
| R=CH₃ | exemple 7b | 1000 | 100 | 99 |
| R = CH₃ | complexe du BINAP préparé selon le protocole opératoire de l'exemple 7 | 1000 | 100 | 99 |
| R = CH₂-CH₃ | exemple 2b | 1000 | 100 | 99 |
| R=CH₂-CH₃ | complexe du BINAP préparé selon le protocole opératoire de l'exemple 2 | 1000 | 100 | 99 |

| | | | | |
|---|---|---|---|---|
| ⁽¹⁾ le catalyseur a été préparé à une température de 110° C au lieu de 100° C. | | | | |

### EXEMPLE 17

Cet exemple illustre la réutilisation du catalyseur polymérique dans l'hydrogénation d'un β-cétoester : l'acétoacétate de méthyle.

Le catalyseur utilisé est celui de l'exemple 7b. Le protocole opératoire utilisé est le même qu'à l'exemple 16, le substrat étant l'acétoacétate de méthyle.

Le tableau 11 résume les résultats obtenus.

**TABLEAU 11**

| Complexe catalyseur | Taux de conversion (%) | Excès énantiomérique (%) |
|---|---|---|
| exemple 7b | 100 | 99 |
| exemple 7b récupéré après une première utilisation | 100 | 67 |
| exemple 7b récupéré après une seconde utilisation | 100 | 60 |
| exemple 7b récupéré après une troisième utilisation | 100 | 61 |

### EXEMPLE 18

### Préparation d'un polyamide au départ du (S,S)-6,6'-bis(aminométhyl)-2,2'-bis(diphénylphosphino)-1,1'-binaphtyle

Dans un ballon de 25 ml, le (S)-6,6'-bis(aminométhyl)-2,2'-bis(diphénylphosphino)-1,1'-binaphtyle (100 mg, 0,147 mmoles) est dissous dans 4 ml de diméthylacétamide dégazé et le chlorure de téréphtaloyle (29,8 mg, 0,147 mmoles) est ajouté. Le mélange est agité 24 heures à température ambiante, puis 1 ml d'isopropanol dégazé est ajouté. Après 1 h d'agitation, le solvant est évaporé et 70 mg de polymère sont obtenus, soit un rendement de 60%.

Les données physico-chimiques de caractérisation sont les suivantes :
[α]_{D} = + 66,1 (c = 1, DMF)
¹H RMN (DMSO, 200 MHz): 1,22 ppm (CH₃ isopropyliques, les extrémités des chaînes ayant été désactivées par de l'isopropanol), 4,39 ppm (CH₂ benzyliques), 6,7-8,1 ppm (protons aromatiques)
³¹P RMN (DMSO, 81 MHz) : -15,82 ppm
Pf > 260° C.

### EXEMPLE 19

### Préparation d'un catalyseur au ruthénium au départ du polymère préparé à l'exemple 18

Une solution de 10 mg (0,012 mmol) du polymère de l'exemple 18 et 3,1 mg (0,006 mmol) de [RuCl₂(benzène)]₂ dans 1 ml de diméthylformamide est maintenue sous agitation à 100° C pendant 1 heure. Après refroidissement à 50° C, le solvant est évaporé sous pression réduite. Après refroidissement à température ambiante, l'évaporation est poursuivie sous pression réduite (0,1 mbar) pour conduire à la formation d'un solide orange.

### EXEMPLE 20

Cet exemple illustre l'hydrogénation d'un β-cétoester, l'acétoacétate de méthyle, en présence du catalyseur de l'exemple 19.

Le méthanol qui a été préalablement séché sur magnésium est ajouté (3 ml) sous argon dans le réacteur conique où le catalyseur de l'exemple 19 a été fraichement préparé. L'acétoacétate de méthyle est ensuite ajouté (rapport catalyseur/substrat de 1/1000). L'opération consistant à faire le vide et à remplir le réacteur d'argon est répétée trois fois. Le septum est alors remplacé par un bouchon percé puis le réacteur est placé dans un autoclave. L'autoclave est purgé trois fois sous argon puis trois fois sous hydrogène avant de recevoir 40 bars de pression d'hydrogène. L'autoclave est placé sur une plaque chauffante (50° C) et l'agitation est maintenue durant la nuit. le réacteur est ensuite récupéré, le bouchon étant remplacé par un septum, et l'argon est réinjecté dans ce réacteur. Le réacteur est mis dans une centrifugeuse, puis la solution est extraite à l'aide d'une seringue. Elle est placée dans un ballon de 50 ml et diluée dans 20 ml de méthanol, prête à être injectée dans une colonne de chromatographie pour chromatographie en phase gazeuse pour analyse de l'activité et de l'énantiosélectivité de la réaction.

L'hydrogénation de l'acétoacétate de méthyle conduit au 3-hydroxybutanoate de méthyle. La détermination des excès énantiomériques est effectuée par chromatographie en phase chirale sur une colonne de type Macheray-Nagel (Lipodex A 25 m x 0,25 mm).

Le taux de conversion obtenu est de 100 % et l'excès énantiomérique est de 78 %.

### EXEMPLE 21

### Préparation d'une polyurée au départ du (S,S)-6,6'-bis(aminométhyl)-2,2'-bis(diphénylphosphino)-1,1'-binaphtyle.

Sous atmosphère d'argon, dans un ballon de 25 mL, le (S)-6,6'-bis(aminométhyl)-2,2'-bis(diphenylphosphino)-1,1'-binaphtyle (255 mg, 0,375 mmoles) est dissous dans 1 mL de dichlorométhane dégazé et le 1,6-diisocyanatohexane (60mL, 0,375 mmoles) est ajouté. Le mélange est agité 24 heures à température ambiante, puis, 1,5 mL d'isopropanol dégazé est ajouté. Après 1h d'agitation, le solide obtenu est filtré et 250 mg de polymère sont ainsi isolés, soit un rendement de 96 %.
RMN ¹H (DMSO d₆, 200MHz) (δ : ppm): 1,0-1,3 (m, CH₃ isopropyliques, les extrémités des chaînes ayant été désactivées par de l'isopropanol) ; 3,2-3,7 (m, CH₂ benzyliques) ; 4,2-4,3 (m, CH) ; 6,5-8,0 (m, CH aromatiques)
RMN ³¹P (DMSO d₆, 81 MHz) (δ : ppm): -15,75
Maldi-Tof : 849,5 g ; 1526 g ; Pf : > 260°C

### EXEMPLE 22

### Préparation d'un catalyseur au ruthénium au départ du polymère préparé à l'exemple 21.

Sous atmosphère d'argon, une solution de 0,012 mmol de polymère et 0,006 mmol de [RuCl₂(benzène)]₂ dans 1 mL de diméthylformamide est maintenue sous agitation à 100°C pendant 1 heure. Après refroidissement à 50°C, le solvant est évaporé sous pression réduite. Après refroidissement à température ambiante, l'évaporation est poursuivie sous pression réduite (0,1 mbar) pour conduire à la formation d'un solide orange.

## Revendications

1. Polymère optiquement actif, pouvant être obtenu par polymérisation d'une diphosphine chirale présentant un axe de symétrie C₂, à l'exclusion de tout autre élément de symétrie, avec un ou plusieurs monomères polymérisables, ladite diphosphine chirale étant constituée d'un corps chiral portant deux groupes fonctionnels identiques capables de réagir avec lesdits monomères polymérisables.

2. Polymère selon la revendication 1, **caractérisé en ce que** les groupes fonctionnels sont choisis parmi les fonctions amino, halogène, hydroxy, thiol, carboxy, isocyanate et thioisocyanate.

3. Polymère selon l'une quelconque des revendications précédentes, **caractérisé en ce que** le corps chiral de la diphosphine a pour structure : 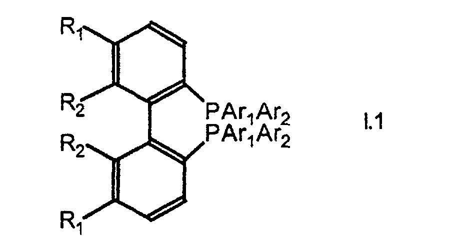 dans laquelle :
- Ar₁, Ar₂ représentent indépendamment un carbocycle saturé, insaturé ou aromatique ;
- R₁, R₂ représentent indépendammant un atome d'hydrogène ; un groupe Z ; ou un groupe -XZ où X représente O, S ou -NT ; et
- Z et T sont indépendamment choisis parmi un groupe hydrocarboné aliphatique saturé, insaturé ou aromatique ; ou un groupe hydrocarboné aliphatique saturé substitué par un ou plusieurs groupes carbocycliques saturés, insaturés ou aromatiques, dans lequel le groupe aliphatique est éventuellement interrompu par O, S et/ou N ; étant entendu que T peut représenter en outre un atome d'hydrogène ; ou bien
deux groupes R₁ et R₂ rattachés au même noyau phényle forment ensemble un carbocycle insaturé ou aromatique, ou encore forment ensemble un hétérocycle insaturé ou aromatique.

4. Polymère selon la revendication 3, **caractérisé en ce que** :
- Ar₁, Ar₂ représentent indépendamment un carbocycle monocyclique saturé, insaturé ou aromatique éventuellement substitué par un ou plusieurs groupes (C₁-C₆)alkyle ou (C₁-C₆)alcoxy et présentant de 3 à 8 atomes de carbone ;
- R₁ et R₂ sont indépendamment choisis parmi un atome d'hydrogène, un groupe (C₁-C₆)alkyle ou un groupe (C₁-C₆)alcoxy ; ou bien
- R₁ et R₂ forment ensemble avec les atomes de carbone qui les portent (i) un carbocycle monocyclique ou polycyclique, insaturé ou aromatique présentant de 5 à 13 atomes de carbone, ou (ii) un hétérocycle monocyclique ou polycyclique, insaturé ou aromatique présentent de 4 à 12 atomes de carbone et un ou plusieurs hétéroatomes choisis parmi O, S et N, lesdits hétérocycle et carbocycle étant éventuellement substitués par un ou plusieurs (C₁-C₆)alkyle ou (C₁-C₆)alcoxy.

5. Polymère selon la revendication 4, **caractérisé en ce que** Ar₁ et Ar₂ sont identiques et représentent un groupe phényle éventuellement substitué par un ou plusieurs (C₁-C₆)alkyle ou (C₁-C₆)alcoxy ; ou un groupe (C₄-C₈)cycloalkyle éventuellement substitué par un ou plusieurs groupes (C₁-C₆)alkyle.

6. Polymère selon l'une quelconque des revendications 3 à 5, **caractérisé en ce que** Ar₁ et Ar₂ sont identiques et représentent cyclohexyle, phényle ou tolyle.

7. Polymère selon l'une quelconque des revendications 3 à 6, **caractérisé en ce que** R₁ et R₂ sont indépendamment choisis parmi un atome d'hydrogène, (C₁-C₆)alkyle ou (C₁-C₆)alcoxy ou bien R₁ et R₂ forment ensemble avec les atomes de carbone qui les portent cyclohexényle, à une seule insaturation éventuellement substitué par un ou plusieurs (C₁-C₆)alkyle ou (C₁-C₆)alcoxy ; ou phényle éventuellement substitué par un ou plusieurs (C₁-C₆)alkyle ou (C₁-C₆)alcoxy.

8. Polymère selon l'une quelconque des revendications 3 à 7, **caractérisé en ce que** le corps chiral de la diphosphine a l'une des structures suivantes :

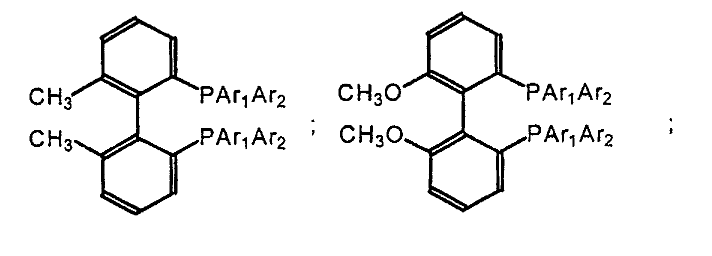

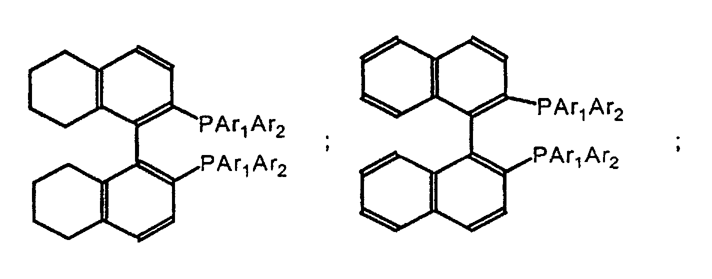

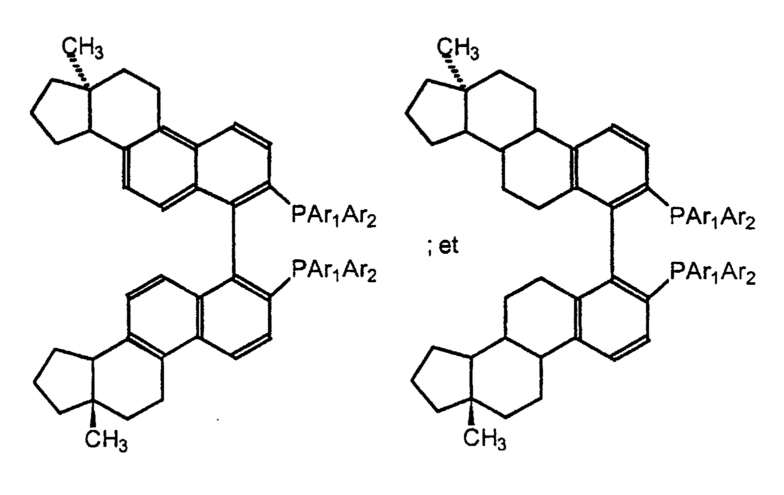

9. Polymère selon l'une quelconque des revendications 1 ou 2, **caractérisé en ce que** le corps chiral de la diphosphine a pour structure :
R₅R₆P-A-PR₅R₆ I.2
dans laquelle :
- A représente un groupe hydrocarboné aliphatique divalent saturé ; un groupe carbocyclique divalent saturé ou aromatique ; ou un groupe hydrocarboné aliphatique divalent saturé interrompu par un groupe carbocyclique divalent saturé ou aromatique ;
- R₅ et R₆ sont différents et représentent un groupe hydrocarboné aliphatique saturé ; un groupe carbocyclique aromatique ou hétérocyclique aromatique.

10. Polymère selon la revendication 9, **caractérisé en ce que** :
- A représente une chaîne alkylène en C₁-C₆ éventuellement substituée par un ou plusieurs groupes (C₁-C₆)alcoxy, di(C₁-C₆)alkylamino ou (C₁-C₆)alkylthio ; un groupe (C₃-C₈)cycloalkylène éventuellement substitué par un ou plusieurs groupes (C₁-C₆)alcoxy, di(C₁-C₆)alkylamino ou (C₁-C₆)alkylthio ; un groupe (C₆-C₁₀)arylène éventuellement substitué par un ou plusieurs groupes (C₁-C₆)alcoxy, di(C₁-C₆)alkylamino ou (C₁-C₆)alkylthio ; ou un groupe -(CH₂)ⱼ-B"-(CH₂)ⱼ- où j représente un nombre entier de 1 à 3 et B" représente (C₃-C₈)cycloalkylène éventuellement substitué par un ou plusieurs (C₁-C₆)alcoxy, di(C₁-C₆)alkylamino ou (C₁-C₆)alkylthio ou (C₆-C₁₀)arylène éventuellement substitué par un ou plusieurs (C₁-C₆)alcoxy, di(C₁-C₆)alkylamino ou (C₁-C₆)alkylthio ;
- R₅ et R₆ sont différents et représentent un hétérocycle monocyclique aromatique présentant de 3 à 7 atomes de carbone et un ou plusieurs hétéroatomes choisis parmi O, N et S ; un groupe (C₆-C₁₀)aryle, ledit hétérocycle et ledit groupe aryle étant éventuellement substitués par un ou plusieurs groupes (C₁-C₆)alkyle ou (C₁-C₆)alcoxy ; ou bien (C₁-C₆)alkyle éventuellement substitué par un ou plusieurs (C₁-C₆)alcoxy.

11. Polymère selon la revendication 10, **caractérisé en ce que** A représente éthylène.

12. Polymère selon l'une quelconque des revendications 10 à 11, **caractérisé en ce que** R₅ et R₆ sont indépendamment choisis parmi phényle éventuellement substitué par un ou plusieurs (C₁-C₆)alkyle ou (C₁-C₆)alcoxy.

13. Polymère selon l'une quelconque des revendications 10 à 12, **caractérisé en ce que** le corps chiral de la diphosphine a pour structure :

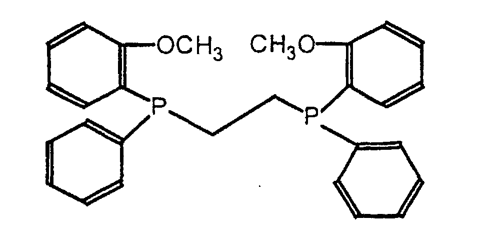

14. Polymère selon l'une quelconque des revendications 1 ou 2, **caractérisé en ce que** le corps chiral de la diphosphine a pour structure : 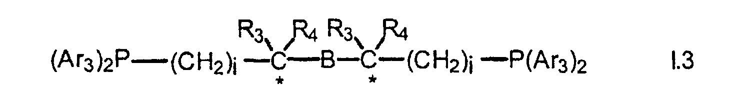 dans laquelle :
- * désigne un centre asymétrique ;
- i représente 0 ou 1 ;
- R₃ et R₄ représentent indépendamment un atome d'hydrogène ou un groupe hydrocarboné aliphatique saturé, ou bien encore les radicaux R₄ sont tels que définis ci-dessus et les radicaux R₃ forment ensemble une chaîne hydrocarbonée aliphatique divalente saturée éventuellement interrompue par deux groupes X, X étant tel que défini ci-dessus à la revendication 3, de préférence par deux groupes X identiques ;
- B représente une liaison ou bien est tel que défini ci-dessus pour A à la revendication 9 ;
- Ar₃ est tel que défini ci-dessus pour R₅ et R₆, à la revendication 9.

15. Polymère selon la revendication 14, **caractérisé en ce que** :
- R₃ et R₄ sont indépendamment choisis parmi un atome d'hydrogène et un groupe (C₁-C₆)alkyle ; ou bien les deux groupes R₃ forment ensemble une chaîne (C₁-C₆)alkylène éventuellement interrompue par deux atomes d'oxygène ou de soufre, et R₄ est tel que défini ci-dessus ;
- B représente une liaison ou bien est tel que défini ci-dessus pour A à la revendication 7 ;
- Ar₃ représente un hétérocycle monocyclique aromatique présentant de 3 à 7 atomes de carbone et un ou plusieurs hétéroatomes choisis parmi O, N et S ; un groupe (C₆-C₁₀)aryle, ledit hétérocycle et ledit groupe aryle étant éventuellement substitués par un ou plusieurs groupes (C₁-C₆)alkyle ou (C₁-C₆)alcoxy ; ou bien (C₁-C₆)alkyle éventuellement substitué par un ou plusieurs (C₁-C₆)alcoxy.

16. Polymère selon la revendication 15, **caractérisé en ce que** Ar₃ représente phényle éventuellement substitué par un ou plusieurs (C₁-C₆)alkyle ou (C₁-C₆)alcoxy.

17. Polymère selon la revendication 15 ou 16, **caractérisé en ce que** le corps chiral de la diphosphine a l'une des structures suivantes : 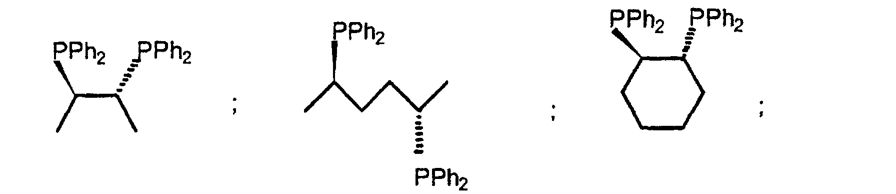 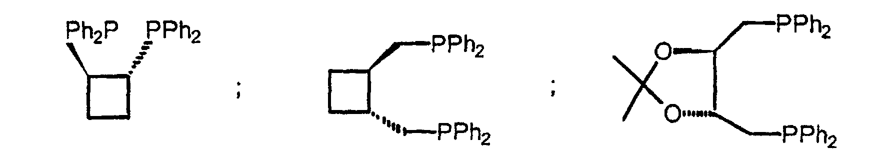 où Ph représente phényle,
ou l'une des formes énantiomères de ces structures.

18. Polymère selon l'une des revendications 1 ou 2, **caractérisé en ce que** le corps chiral de la diphosphine a pour structure : 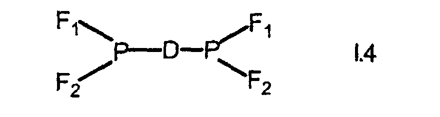 dans laquelle :
- D est tel que défini ci-dessus pour A à la revendication 9 ;
- F₁ et F₂ sont identiques et représentent un groupe hydrocarboné aliphatique saturé, ledit groupe portant au moins un centre chiral ; un groupe carbocyclique saturé portant au moins un centre chiral ; ou bien
- F₁ et F₂ forment ensemble une chaîne hydrocarbonée aliphatique divalente saturée, éventuellement interrompue par deux groupes X, X étant tel que défini ci-dessus à la revendication 3, deux des carbones de ladite chaîne constituant des centres asymétriques.

19. Polymère selon la revendication 18, **caractérisé en ce que** :
- D représente une chaîne alkylène en C₁-C₆ éventuellement substituée par un ou plusieurs groupes (C₁-C₆)alcoxy, di (C₁-C₆)alkylamino ou (C₁-C₆)alkylthio ; un groupe (C₃-C₈)cycloalkylène éventuellement substitué par un ou plusieurs groupes (C₁-C₆)alcoxy, di(C₁-C₆)alkylamino ou (C₁-C₆)alkylthio ; un groupe (C₆-C₁₀)arylène éventuellement substitué par un ou plusieurs groupes (C₁-C₆)alcoxy, di(C₁-C₆)alkylamino ou (C₁-C₆)alkylthio ; ou un groupe -(CH₂)ⱼ-B"-(CH₂)ⱼ- où j représente un nombre entier compris entre 1 et 3 et B" représente (C₃-C₈)cycloalkylène (éventuellement substitué par un ou plusieurs (C₁-C₆)alcoxy, di(C₁-C₆)alkylamino ou (C₁-C₆)alkylthio) ou (C₆-C₁₀)arylène (éventuellement substitué par un ou plusieurs groupes (C₁-C₆)alcoxy, di(C₁-C₆)alkylamino ou (C₁-C₆)alkylthio) ;
- F₁ et F₂ sont identiques et représentent (C₁-C₆)alkyle éventuellement substitué par un ou plusieurs (C₁-C₆)alcoxy, ledit groupe alkyle portant au moins un centre chiral ; (C₃-C₈)cycloalkyle substitué par un ou plusieurs (C₁-C₆)alcoxy ou (C₁-C₆)alkyle, ledit cycloalkyle portant au moins un centre chiral ; ou bien F₁ et F₂ forment ensemble une chaîne (C₁-C₆)alkylène éventuellement interrompue par deux atomes d'oxygène ou de soufre, ladite chaîne étant substituée par un ou plusieurs groupes (C₁-C₆)alkyle ou (C₁-C₆)alcoxy, deux des carbones de ladite chaîne constituant des centres asymétriques.

20. Polymère selon la revendication 18 ou 19, **caractérisé en ce que** le corps chiral de la diphosphine a pour structure : 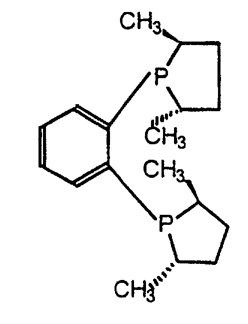 ou certaine forme diastéréoisomérique.

21. Polymère selon l'une quelconque des revendications précédentes, **caractérisé en ce que** ladite diphosphine est constituée d'un corps chiral portant deux groupes -CH₂NH₂.

22. Polymère selon la revendication 21, **caractérisé en ce qu'**il a pour motif récurrent : 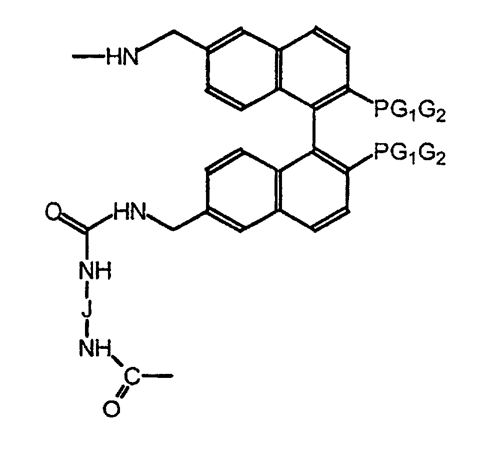 dans lequel
G₁ et G₂ représentent indépendamment un groupe carbocyclique saturé ou aromatique ; et
J représente un radical divalent hydrocarboné à caractère aliphatique, alicyclique et/ou aromatique ;
le degré de polymérisation étant compris entre 2 et 100, de préférence entre 2 et 50.

23. Polymère selon l'une quelconque des revendications précédentes, **caractérisé en ce que** le polymère peut être obtenu par polymérisation de ladite diphosphine avec un monomère polymérisable présentant un plan de symétrie ou un axe de symétrie C₂, de préférence un axe de symétrie C₂.

24. Polymère selon l'une quelconque des revendications précédentes, **caractérisé en ce que** les monomères polymérisables sont des diisocyanates.

25. Procédé pour la préparation d'un polymère optiquement actif, selon l'une quelconque des revendications 1 à 24, **caractérisé en ce que** l'on polymérise une diphosphine chirale présentant un plan de symétrie C₂, à l'exclusion de tout autre élément de symétrie, avec un ou plusieurs monomères polymérisables, ladite diphosphine chirale étant constituée d'un corps chiral portant deux groupes fonctionnels identiques capables de réagir avec lesdits monomères polymérisables.

26. Utilisation d'un polymère selon l'une quelconque des revendications 1 à 24 comme ligand pour la préparation d'un complexe métallique utile dans la catalyse asymétrique.

27. Utilisation selon la revendication 26, **caractérisé en ce que** le complexe métallique est un complexe du ruthénium ou du rhodium.

28. Utilisation selon la revendication 27, dans l'hydrogénation de liaisons C=O ou de liaisons C=C.

29. Polymère racémique correspondant au polymère selon l'une quelconque des revendications 1 à 24.

30. Utilisation d'une association d'un polymère optiquement actif selon l'une quelconque des revendications 1 à 24, avec une diamine, pour la réduction sélective de cétones.

31. Utilisation d'une association d'un polymère racémique selon la revendication 29 avec une diamine chirale, pour la réduction sélective de cétones.

32. Utilisation selon l'une quelconque des revendications 30 et 31 dans laquelle la diamine est le 1,2-diamino-1,2-diphényléthane.

33. Polymère selon la revendication 21, **caractérisé en ce qu'**il a pour motif récurrent : 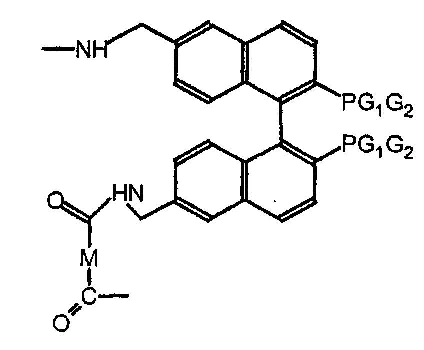 dans lequel
G₁ et G₂ sont tels que définis à la revendication 22 ; et
M est tel que défini pour J à la revendication 22.

## Patentansprüche

1. Optisch aktives Polymer, das durch Polymerisation eines chiralen Diphosphins, das eine C₂-Symmetrieachse, aber kein anderes Symmetrieelement aufweist, mit einem oder mehreren polymerisierbaren Monomeren erhalten werden kann, wobei das chirale Diphosphin aus einem chiralen Körper besteht, der zwei gleiche funktionelle Gruppen trägt, die in der Lage sind, mit den polymerisierbaren Monomeren zu reagieren.

2. Polymer nach Anspruch 1, **dadurch gekennzeichnet, dass** die funktionellen Gruppen aus Amino-, Halogen-, Hydroxy-, Thiol-, Carboxy-, Isocyanat- und Thioisocyanatfunktionen ausgewählt sind.

3. Polymer nach einem der vorhergehenden Ansprüche, **dadurch gekennzeichnet, dass** der chirale Körper des Diphosphins die Struktur 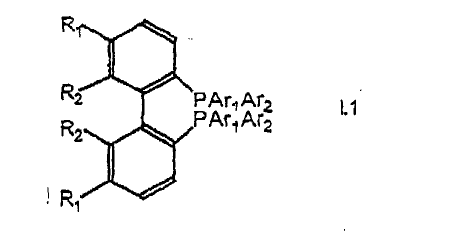 hat, in welcher
- Ar₁ und Ar₂ unabhängig voneinander einen gesättigten, ungesättigten oder aromatischen Carbocyclus und
- R₁ und R₂ unabhängig voneinander ein Wasserstoffatom, eine Gruppe Z oder eine Gruppe -XZ, worin X O, S bzw. -NT bedeutet, bedeuten und
- Z und T unabhängig voneinander aus einer gesättigten bzw. ungesättigten aliphatischen oder aromatischen Kohlenwasserstoffgruppe odex einer gesättigten aliphatischen Kohlenwasserstoffgruppe, die durch eine oder mehrere gesättigte, ungesättigte oder aromatische carbocyclische Gruppen substituiert ist, in welcher die aliphatische Gruppe gegebenenfalls durch O, S und/oder N unterbrochen ist, ausgewählt sind, wobei es selbstverständlich ist, dass T außerdem ein Wasserstoffatom bedeuten kann, oder
zwei am selben Phenylkern angelagerte Gruppen R₁ und R₂ zusammen einen ungesättigten oder aromatischen Carbocyclus oder auch zusammen einen ungesättigten oder aromatischen Heterocyclus bilden,

4. Polymer nach Anspruch 3, **dadurch gekennzeichnet, dass**
- Ar₁ und Ar₂ unabhängig voneinander einen gesättigten, ungesättigten oder aromatischen monocyclischen Carbocyclus bedeuten, der 3 bis 8 Kohlenstoffatome aufweist und gegebenenfalls durch eine oder mehrere (C₁- bis C₆-)Alkylgruppen bzw. (C₁- bis C₆-)Alkoxygruppen substituiert ist,
- R₁ und R₂ unabhängig voneinander aus einem Wasserstoffatom, einer (C₁- bis C₆-)Alkylgruppe oder einer (C₁- bis C₆-)Alkoxygruppe ausgewählt sind oder
- R₁ und R₂ zusammen mit den Kohlenstoffatomen, die sie tragen, (i) einen ungesättigten oder aromatischen monocyclischen bzw. polycyclischen Carbocyclus, der 5 bis 13 Kohlenstoffatome aufweist, oder (ii) einen ungesättigten oder aromatischen monocyclischen bzw. polycyclischen Heterocyolus bilden, der 4 bis 12 Kohlenstoffatome und ein oder mehrere aus O, S und N ausgewählte Heteroatome aufweist, wobei Heterocyclus und Carbocyclus gegebenenfalls durch eine oder mehrere (C₁- bis C₆-)Alkylgruppen oder (C₁- bis C₆-)Alkoxygruppen substituiert sind.

5. Polymer nach Anspruch 4, **dadurch gekennzeichnet, dass** Ar₁ und Ar₂ gleich sind und eine Phenylgruppe, die gegebenenfalls durch ein oder mehrere (C₁- bis C₆-)Alkyl bzw. (C₁- bis C₆-)Alkoxy substituiert ist, oder eine gegebenenfalls durch eine oder mehrere (C₁bis C₆-)Alkylgruppen substituierte (C₄- bis C₈-)Cycloalkylgruppe bedeuten.

6. Polymer nach einem der Ansprüche 3 bis 5, **dadurch gekennzeichnet, dass** Ar₁ und Ar₂ gleich sind und Cyclohexyl, phenyl oder Tolyl bedeuten.

7. Polymer nach einem der Ansprüche 3 bis 6, **dadurch gekennzeichnet, dass** R₁ und R₂ unabhängig voneinander aus einem Wasserstoffatom, (C₁- bis C₆-)Alkyl bzw. (C₁- bis C₆-)Alkoxy ausgewählt sind oder R₁ und R₂ zusämmen mit den Kohlenstoffatomen, die sie tragen, einen Cyclohexenylrest mit einer einzigen ungesättigten Funktion, der gegebenenfalls durch ein oder mehrere (C₁- bis C₆-)Alkyl bzw. (C₁- bis C₆-)Alkoxy substituiert ist, oder einen Phenylrest, der gegebenenfalls durch ein oder mehrere (C₁- bis C₆-)Alkyl bzw. (C₁- bis C₆-)Alkoxy substituiert ist, bilden.

8. Polymer nach einem der Ansprüche 3 bis 7, **dadurch gekennzeichnet, dass** der chirale Körper des Diphosphins eine der folgenden Strukturen hat:

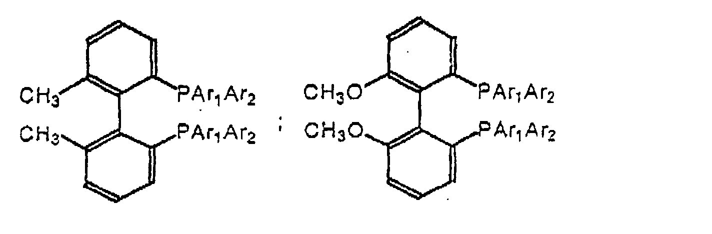

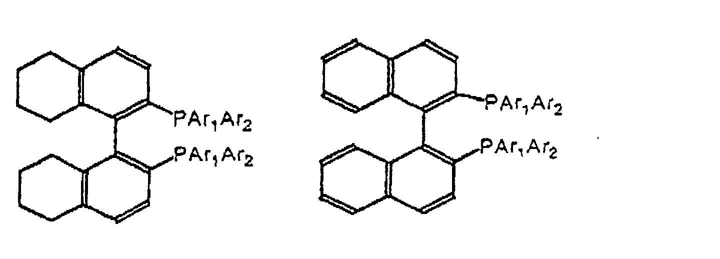

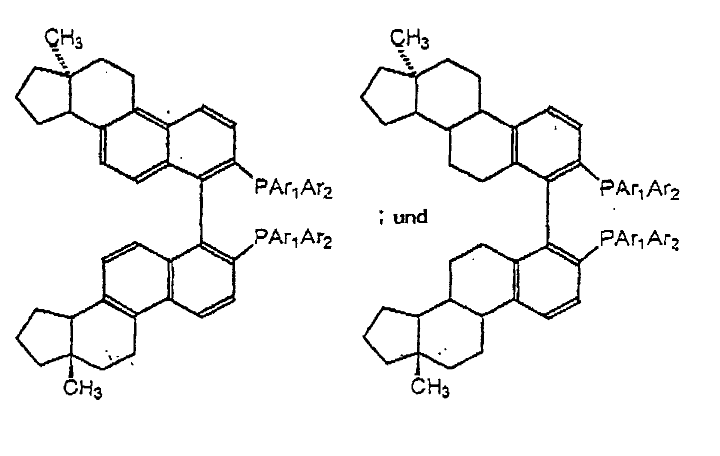

9. Polymer nach Anspruch 1 oder 2, **dadurch gekennzeichnet, dass** der chirale Körper des Diphosphins die Struktur
R₅R₆P-A-PR₅R₆ I.2
hat, in welcher
- A eine zweiwertige gesättigte aliphatische Kohlenwasserstoffgruppe, eine zweiwertige gesättigte oder aromatische carbocyclische Gruppe oder eine zweiwertige gesättigte aliphatische Kohlenwasserstoffgruppe, die durch eine zweiwertige gesättigte oder aromatische carbocyclische Gruppe unterbrochen ist, bedeutet, wobei
- R₅ und R₆ voneinander verschieden sind und eine gesättigte aliphatische Kohlenwasserstoffgruppe, eine aromatische carbocyclische oder eine aromatische heterocyclische Gruppe bedeuten.

10. Polymer nach Anspruch 9, **dadurch gekennzeichnet, dass**
- A eine C₁- bis C₆-Alkylenkette, die gegebenenfalls durch eine oder mehrere (C₁- bis C₆-)Alkoxy-, Di(C₁bis C₆-)Alkylamino- oder (C₁- bis C₆-)Alkylthiogruppen substituiert ist, eine (C₃- bis C₈-)Cycloalkylengruppe, die gegebenenfalls durch eine oder mehrere (C₁- bis C₆-)Alkoxy-, Di(C₁- bis C₆-)Alkylamino- oder (C₁- bis C₆-)Alkylthiogruppen substituiert ist, eine (C₆- bis C₁₀-)Arylengruppe, die gegebenenfalls durch eine oder mehrere (C₁- bis C₆-)Alkoxy-, Di(C₁- bis C₆-)Alkylamino- oder (C₁- bis C₆-)Alkylthiogruppen substituiert ist, eine Gruppe -(CH₂)ⱼ-B"-(CH₂)ⱼ-, worin j eine ganze Zahl von 1 bis 3 und B" (C₃- bis C₈-)Cycloalkylen bedeutet, das gegebenenfalls durch ein oder mehrere (C₁- bis C₆-)Alkoxy, Di(C₁- bis C₆-)Alkylamino oder (C₁- bis C₆-)Alkylthio substituiert ist, oder (C₆- bis C₁₀-)Arylen, das gegebenenfalls durch ein oder mehrere (C₁- bis C₆-)Alkoxy, Di(C₁- bis C₆-)Alkylamino oder (C₁- bis C₆-)Alkylthio substituiert ist, bedeutet, wobei
- R₅ und R₆ voneinander verschieden sind und einen aromatischen monocyclischen Heterocyclus, der 3 bis 7 Kohlenstoffatome und ein oder mehrere aus O, N und S ausgewählte Heteroatome enthält, eine (C₆- bis C₁₀-)Arylgruppe, wobei Heterocyclus und Arylgruppe gegebenenfalls durch eine oder mehrere (C₁- bis C₆-)Alkylgruppen bzw. (C₁- bis C₆-)Alkoxygruppen substituiert sind, oder eine gegebenenfalls durch ein oder mehrere (C₁- bis C₆-)Alkoxy substituierte (C₁- bis C₆-)Alkylgruppe bedeuten.

11. Polymer nach Anspruch 10, **dadurch gekennzeichnet, dass** A Ethylen bedeutet.

12. Polymer nach Anspruch 10 oder 11, **dadurch gekennzeichnet, dass** R₅ und R₆ unabhängig voneinander aus Phenyl, das gegebenenfalls durch ein oder mehrere (C₁- bis C₆-)Alkyl oder (C₁- bis C₆-)Alkoxy substituiert ist, ausgewählt sind.

13. Polymer nach einem der Ansprüche 1 bis 12, **dadurch gekennzeichnet, dass** der chirale Körper des Diphosphins die Struktur hat:

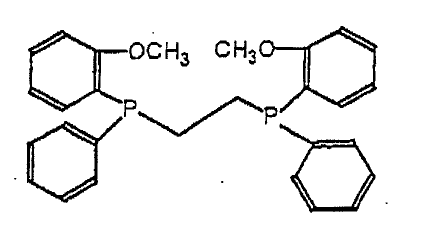

14. Polymer nach Anspruch 1 oder 2, **dadurch gekennzeichnet, dass** der chirale Körper des Diphosphins die Struktur 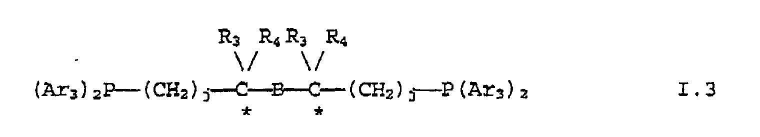 hat, in welcher
- * ein asymmetrisches Zentrum und
- j 0 oder 1 bedeutet und
- R₃ und R₄ unabhängig voneinander ein Wasserstoffatom oder eine gesättigte aliphatische Kohlenwasserstoffgruppe bedeuten oder die Reste R₄ wie weiter oben definiert sind und die Reste R₃ zusammen eine zweiwertige gesättigte aliphatische Kohlenwasserstoffkette bilden, die gegebenenfalls durch zwei Gruppen X, X, die wie weiter oben im Anspruch 3 definiert sind, und vorzugsweise durch zwei gleiche Gruppen X unterbrochen ist,
- B eine Bindung oder wie weiter oben für A in Anspruch 9 definiert und
- Ar₃ wie weiter oben für R₅ und R₆ in Anspruch 9 definiert ist.

15. Polymer nach Anspruch 14, **dadurch gekennzeichnet, dass**
- R₃ und R₄ unabhängig voneinander aus einem Wasserstoffatom und einer (C₁- bis C₆-)Alkylgruppe ausgewählt sind oder die beiden Gruppen R₃ zusammen eine (C₁- bis C₆-)Alkylenkette bilden, die gegebenenfalls durch zwei Sauerstoff- oder Schwefelatome unterbrochen ist, und R₄ wie weiter oben definiert ist,
- B eine Bindung oder wie weiter oben für A in Anspruch 7 definiert ist und
- Ar₃ einen monocyclischen aromatischen Heterocyclus, der 3 bis 7 Kohlenstoffatome und ein oder mehrere aus O, N und S ausgewählte Heteroatome enthält, eine (C₆- bis C₁₀-)Arylgruppe, wobei Heterocyclus und Arylgruppe gegebenenfalls durch eine oder mehrere (C₁- bis C₆-)Alkylgruppen bzw. (C₁- bis C₆-)Alkaxygruppen substituiert sind, oder eine gegebenenfalls durch ein oder mehrere (C₁- bis C₆-)Alkoxy substituierte (C₁- bis C₆-)Alkylgruppe bedeutet.

16. Polymer nach Anspruch 15, **dadurch gekennzeichnet, dass** Ar₃ Phenyl, das gegebenenfalls durch ein oder mehrere (C₁- bis C₆-)Alkyl oder (C₁- bis C₆-)Alkoxy substituierte ist, bedeutet.

17. Polymer nach Anspruch 15 oder 16, **dadurch gekennzeichnet, dass** der chirale Körper des Diphosphins eine der folgenden Strukturen 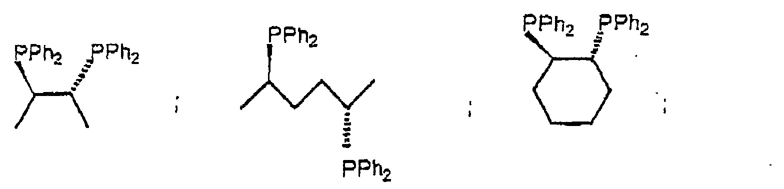 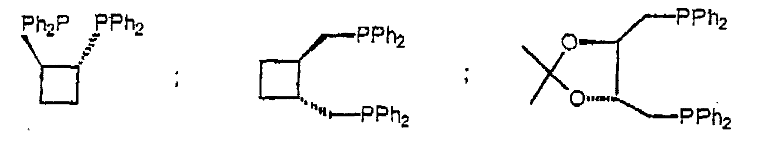 worin Ph phenyl bedeutet, oder eine der enantiomeren Formen dieser Strukturen hat.

18. Polymer nach Anspruch 1 oder 2, **dadurch gekennzeichnet, dass** der chirale Körper des Diphosphins die Struktur 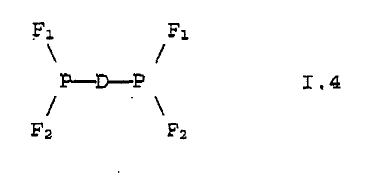 hat, in welcher
- D wie weiter oben für A in Anspruch 9 definiert ist,
- F₁ und F₂ gleich sind und eine gesättigte aliphatische Kohlenwasserstoffgruppe, die mindestens ein chirales Zentrum trägt, und eine gesättigte carbocyclische Gruppe, die mindestens ein chirales Zentrum trägt, bedeuten oder
- F₁ und F₂ zusammen eine zweiwertige gesättigte aliphatische Kohlenwasserstoffkette bilden, die gegebenenfalls durch zwei Gruppen X, X unterbrochen ist, die wie weiter oben in Anspruch 3 definiert sind, wobei zwei Kohlenstoffatome dieser Kette asymmetrische Zentren bilden.

19. Polymer nach Anspruch 18, **dadurch gekennzeichnet, dass**
- D eine C₁- bis C₆-Alkylenkette, die gegebenenfalls durch eine oder mehrere (C₁- bis C₆-)Alkoxy-, Di(C₁bis C₆-)Alkylamino- oder (C₁- bis C₆-)Alkylthiogruppen substituiert ist, eine (C₃- bis C₈-)Cycloalkylengruppe, die gegebenenfalls durch eine oder mehrere (C₁- bis C₆-)Alkoxy-, Di(C₁- bis C₆-)Alkylamino- oder (C₁- bis C₆-)Alkylthiogruppen substituiert ist, eine (C₆- bis C₁₀-)Arylergruppe, die gegebenenfalls durch eine oder mehrere (C₁- bis C₆-)Alkoxy-, Di(C₁- bis C₆-)Alkylamino- oder (C₁- bis C₆-)Alkylthiogruppen substituiert ist, oder eine Gruppe -(CH₂)ⱼ-B"-(CH₂)ⱼ-, worin j eine ganze Zahl von 1 bis 3 und B" (C₃- bis C₈-)Cycloalkylen bedeutet, das gegebenenfalls durch ein oder mehrere (C₁- bis C₆-)Alkoxy, Di(C₁- bis C₆-)Alkylamino oder (C₁- bis C₆-)Alkylthio substituiert ist, oder (C₆bis C₁₀-)Arylen, das gegebenenfalls durch eine oder mehrere (C₁- bis C₆-)Alkoxy-, Di(C₁- bis C₆-)Alkylamino- oder (C₁- bis C₆-)Alkylthiogruppen substituiert ist, bedeutet, wobei
- F₁ und F₂ gleich sind und (C₁- bis C₆-)Alkyl, das gegebenenfalls durch ein oder mehrere (C₁- bis C₆-)Alkoxy substituiert ist, wobei die Alkylgruppe mindestens ein chirale Zentrum trägt, und (C₃- bis C₆-)Cycloalkyl, das durch ein oder mehrere (C₁- bis C₆-)Alkoxy oder (C₁- bis C₆-)Alkyl substituiert ist, wobei das Cycloalkyl mindestens ein chirales Zentrum trägt, bedeuten, oder F₁ und F₂ zusammen eine (C₁- bis C₆-)Alkylenkette, die gegebenenfalls durch zwei Sauerstoff- bzw. Schwefelatome unterbrochen ist, bilden, wobei diese Kette durch eine oder mehrere (C₁- bis C₆-)Alkyl- oder (C₁- bis C₆-)Alkoxygruppen substituiert ist und zwei Kohlenstoffatome der Kette asymmetrische Zentren bilden.

20. Polymer nach Anspruch 18 oder 19, **dadurch gekennzeichnet, dass** der chirale Körper des Diphosphins die Struktur 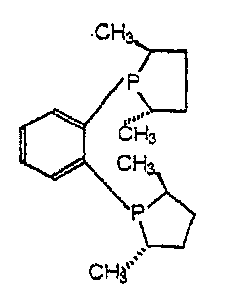 oder eine bestimmte diastereoisomere Form hat.

21. Polymer nach einem der vorhergehenden Ansprüche, **dadurch gekennzeichnet, dass** das Diphosphin aus einem chiralen Körper, der zwei -CH₂NH₂-Gruppen trägt, besteht.

22. Polymer nach Anspruch 21, **dadurch gekennzeichnet, dass** es als Repetiereinheit 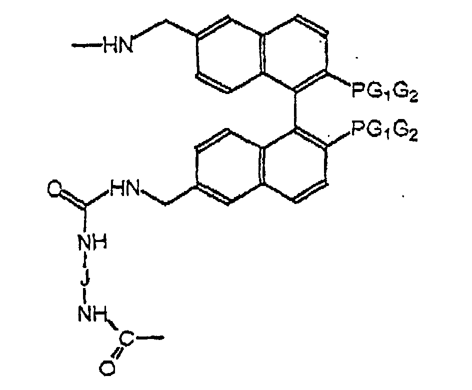 hat, in welcher
G₁ und G₂ unabhängig voneinander eine gesättigte oder aromatische carbocyclische Gruppe bedeuten und
J einen zweiwertigen Kohlenwasserstoffrest mit aliphatischem, alicyclischem und/oder aromatischem Charakter bedeutet, wobei
der Polymerisationsgrad 2 bis 100 und vorzugsweise zwischen 2 und 50 beträgt.

23. Polymer nach einem der vorhergehenden Ansprüche, **dadurch gekennzeichnet, dass** es durch Polymerisation des Diphosphins mit einem polymerisierbaren Monomer erhalten werden kann, das eine C₂-Symmetrieebene oder - achse und vorzugsweise eine C₂-Symmetrieachse aufweist.

24. Polymer nach einem der vorhergehenden Ansprüche, **dadurch gekennzeichnet, dass** die polymerisierbaren Monomere Diisocyanate sind.

25. Verfahren zur Herstellung eines optisch aktiven Polymers nach einem der Ansprüche 1 bis 24, **dadurch gekennzeichnet, dass** ein chirales Diphosphin, das eine C₂-Symmetrieebene, aber kein anderes Symmetrieelement aufweist, mit einem oder mehreren polymerisierbaren Monomeren polymerisiert wird, wobei das chirale Diphosphin aus einem chiralen Körper besteht, der zwei gleiche funktionelle Gruppen trägt, die in der Lage sind, mit den polymerisierbaren Monomeren zu reagieren.

26. Verwendung eines Polymers nach einem der Ansprüche 1 bis 24 als Ligand zur Herstellung eines bei der asymmetrischen Katalyse nützlichen Metallkomplexes.

27. Verwendung nach Anspruch 26, **dadurch gekennzeichnet, dass** der Metallkomplex ein Ruthenium- oder Rhodiumkomplex ist.

28. Verwendung nach Anspruch 27 bei der Hydrierung von C=O- oder C=C-Bindungen.

29. Racemisches Polymer, das dem Polymer nach einem der Ansprüche 1 bis 24 entspricht.

30. Verwendung einer Anlagerung eines optisch aktiven Polymers nach einem der Ansprüche 1 bis 24 an ein Diamin zur selektiven Reduzierung von Ketonen.

31. Verwendung einer Anlagerung eines racemischen Polymers nach Anspruch 29 an ein chirales Diamin zur selektiven Reduzierung von Ketonen.

32. Verwendung nach Anspruch 30 oder 31, bei welcher das Diamin 1,2-Diamino-1,2-diphenylethan ist.

33. Polymer nach Anspruch 21, **dadurch gekennzeichnet, dass** es als Repetiereinheit 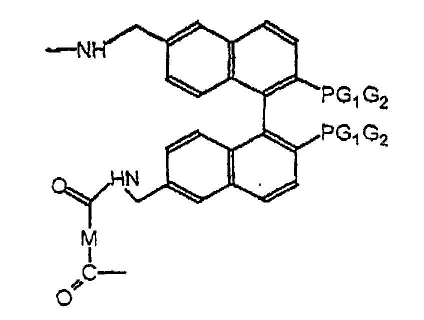 hat, in welcher
G₁ und G₂ wie in Anspruch 22 definiert sind und
M wie für J in Anspruch 22 definiert ist.

## Claims

1. Optically active polymer which may be obtained by polymerisation of a chiral diphosphine, which has a C₂ axis of symmetry to the exclusion of any other element of symmetry, with one or more polymerisable monomers, said chiral diphosphine being formed from a chiral body having two identical functional groups capable of reacting with said polymerisable monomers.

2. Polymer according to Claim 1, **characterised in that** the functional groups are selected from amino, halogen, hydroxy, thiol, carboxy, isocyanate and thio-isocyanate functions.

3. Polymer according to any one of the preceding claims, **characterised in that** the chiral body of the diphosphine has the following structure: 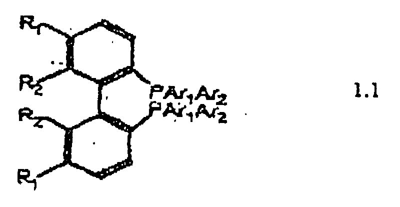 wherein:
• Ar₁, Ar₂ independently represent a saturated, unsaturated or aromatic carbocycle;
• R₁, R₂ independently represent a hydrogen atom, a Z group or an-XZ group, where X represents O, S or -NT; and
• Z and T are independently selected from a saturated, unsaturated or aromatic aliphatic hydrocarbon group, or a saturated alipharic hydrocarbon group substituted by one or more saturated, unsaturated or aromatic carbocyclic groups, in which the aliphatic group is possibly broken by O, S and/or N, it being understood that T can, moreover, represent a hydrogen atom; or two R₁ and R₂ groups attached to the same phenyl nucleus together form an unsaturated or aromatic carbocycle, or together form an unsaturated or aromatic heterocycle.

4. Polymer according to Claim 3, **characterised in that**:
• Ar₁, Ar₂ independently represent a saturated, unsaturated or aromatic monocyclic carbocycle possibly substituted by one or more (C₁-C₆)alkyl or (C₁-C₆)alcoxy groups and having 3 to 8 carbon atoms;
• R₁ and R₂ are independently selected from a hydrogen atom, a (C₁-C₆)alkyl group or a (C₁-C₆)alcoxy group; or
• R₁ and R₂ together with the carbon atoms carrying them form (i) an unsaturated or aromatic monocyclic or polycyclic carbocycle having 5 to 13 carbon atoms, or (ii) an unsaturated or aromatic monocyclic or polycyclic heterocycle having 4 to 12 carbon atoms and one or more heteroatoms selected from O, S and N, said heterocycle and carbocycle possibly being substituted by one or more (C₁-C₆)alkyl or (C₁-C₆)alcoxy.

5. Polymer according to Claim 4, **characterised in that** Ar₁ and Ar₂ are identical and represent a phenyl group possibly substituted by one or more (C₁-C₆)alkyl or (C₁-C₆)alcoxy, or a (C₄-C₈)cycloalkyl group possibly substituted by one or more (C₁-C₆)alkyl groups.

6. Polymer according to any one of Claims 3 to 5, **characterised in that** Ar₁ and Ar₂ are identical and represent cyclohexyl, phenyl or tolyl.

7. Polymer according to any one of Claims 3 to 6, **characterised in that** R₁ and R₂ are independently selected from a hydrogen atom, (C₁-C₆)alkyl or (C₁-C₆)alcoxy, or R₁ and R₂ together with the carbon atoms carrying them form monounsaturated cyclohexenyl possibly substituted by one or more (C₁-C₆)alkyl or (C₁-C₆)alcoxy, or phenyl possibly substituted by one or more (C₁-C₆)alkyl or (C₁-C₆)alcoxy.

8. Polymer according to any one of Claims 3 to 7, **characterised in that** the chiral body of the diphosphine has one of the following structures:

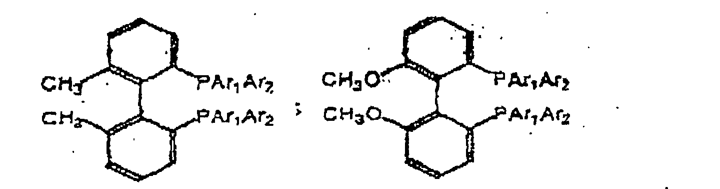

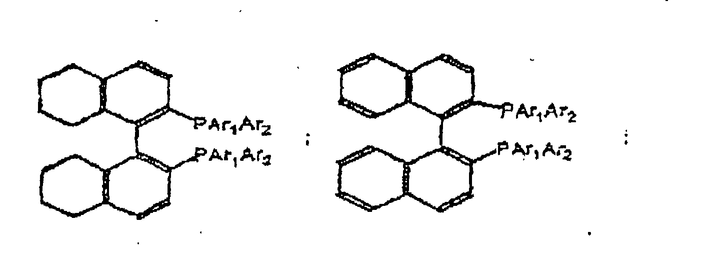

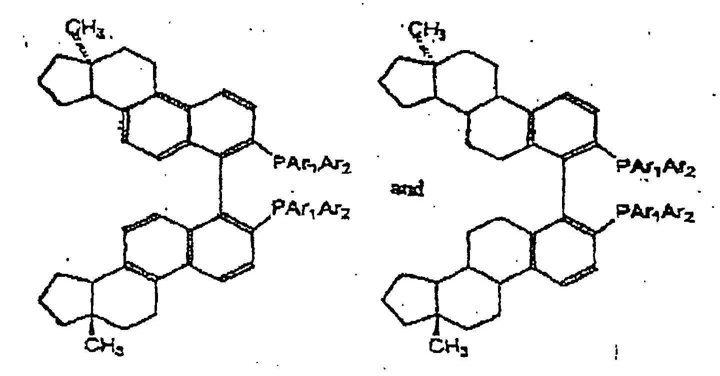

9. Polymer according to either one of Claims 1 or 2, **characterised in that** the chiral body of the diphosphine has the following structure:
R₅R₆P-A-PR₅R₆ I.2
wherein:
• A represents a divalent saturated aliphatic hydrocarbon group, a divalent saturated or aromatic carbocyclic group, or a divalent saturated aliphatic hydrocarbon group broken by a divalent saturated or aromatic carbocyclic group;
• R₅ and R₆ are different and represent a saturated aliphatic hydrocarbon group, an aromatic carbocyclic or aromatic heterocyclic group.

10. Polymer according to Claim 9, **characterised in that**:
• A represents a C₁-C₆ alkylene chain possibly substituted by one or more (C₁-C₆)alcoxy, di(C₁-C₆)alkyl amino or (C₁-C₆)alkyl thio groups, a (C₃-C₈)cycloalkylene group possibly substituted by one or more (C₁-C₆)alcoxy, di(C₁-C₆)alkyl amino or (C₁-C₆)alkyl thio groups, a (C₆-C₁₀)arylene group possibly substituted by one or more (C₁-C₆)alcoxy, di(C₁-C₆)alkyl amino or (C₁-C₆)alkyl thio groups, or a -(CH₂)ⱼ-B"-(CH₂)ⱼ- group, wherein j represents a whole number from 1 to 3 and B" represents (C₃-C₈)cycloalkylene possibly substituted by one or more (C₁-C₆)alcoxy, di(C₁-C₆)alkyl amino or (C₁-C₆)alkyl thio or (C₆-C₁₀)arylene possibly substituted by one or more (C₁-C₆)alcoxy, di(C₁-C₆)alkyl amino or (C₁-C₆)alkyl thio;
• R₃ and R₆ are different and represent an aromatic monocyclic heterocycle having 3 to 7 carbon atoms and one or more heteroatoms selected from O, N and S, a (C₆-C₁₀)aryl group, said heterocycle and said aryl group possibly being substituted by one or more (C₁-C₆)alkyl or (C₁-C₆)alcoxy, or (C₁-C₆)alkyl possibly substituted by one or more (C₁-C₆)alcoxy.

11. Polymer according to Claim 10, **characterised in that** A represents erhylene.

12. Polymer according to any one of Claims 10 to 11, **characterised in that** R₅ and R₆ are independently selected from phenyl possibly substituted by one or more (C₁-C₆)alkyl or (C₁-C₆)alcoxy.

13. Polymer according to any one of Claims 10 to 12, **characterised in that** the chiral body of the diphosphine has the following structure:

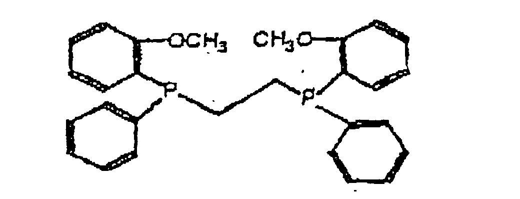

14. Polymer according to either one of Claims 1 or 2, **characterised in that** the chiral body of the diphosphine has the following structure: 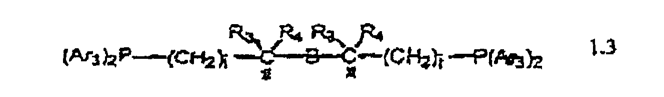 wherein:
• *designates an asymmetric centre;
• i represents 0 or 1;
• R₃ and R₄ independently represent a hydrogen atom or a saturated aliphatic hydrocarbon group, or the R₄ radials are as defined above and the R₃ radicals together form a divalent, saturated alipharic hydrocarbon chain possibly broken by two X groups, X being as defined above in Claim 3, and preferably by two identical X groups;
• B represents a bond or is as defined above for A in Claim 9;
• Ar₃ is as defined above for R₅ and R₆ in Claim 9.

15. Polymer according to Claim 14, **characterised in that**:
• R₃ and R₄ are independently selected from a hydrogen atom and a (C₁-C₆)alkyl group, or the two R₃ groups together form a (C₁-C₆)alkylene chain possibly broken by two oxygen or sulphur atoms, and R₄ is as defined above;
• B represents a bond or is as defined above for A in Claim 7;
• Ar₃ represents an aromatic monocylic heterocycle having 3 to 7 carbon atoms and one or several heteroatoms selected from O, N and S, a (C₆-C₁₀)aryl group, said heterocycle and said aryl group possibly being substituted by one or more (C₁-C₆)alkyl or (C₁-C₆)alcoxy groups, or (C₁-C₆)alkyl possibly substituted by one or more (C₁-C₆)alcoxy.

16. Polymer according to Claim 15, **characterised in that** Ar₃ represents phenyl possibly substituted by one or more (C₁-C₆)alkyl or (C₁-C₆)alcoxy.

17. Polymer according to Claim 15 or 16, **characterised in that** the chiral body of the diphosphine has one of the following structures: 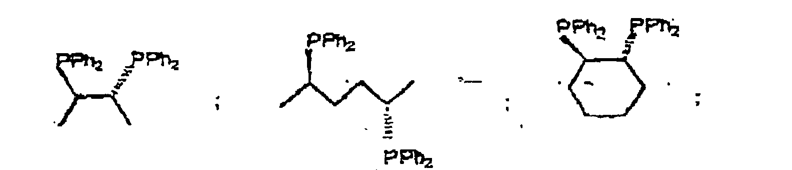 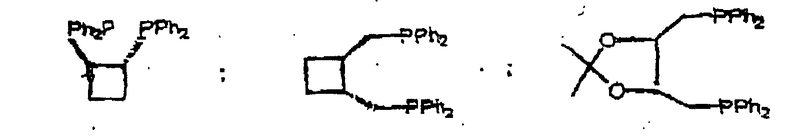 where Ph represents phenyl
or one of the enantiomeric forms of these structures.

18. Polymer according to either of Claims 1 or 2, **characterised in that** the chiral body of the diphosphine has the following structure: 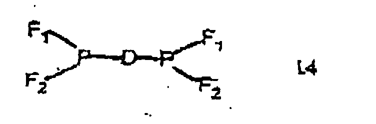 wherein:
• D is as defined above for A in Claim 9;
• F₁ and F₂ are identical and represent a saturated aliphatic hydrocarbon group, said group having at least one chiral centre, a saturated carbocyclic group having at least one chiral centre; or
• F₁ and F₂ together form a divalent saturated aliphatic hydrocarbon chain, possibly broken by two X groups, X being as defined above in Claim 3, two of the carbon atoms of said chain forming asymmetric centres.

19. Polymer according to Claim 18, **characterised in that**:
• D represents a C₁-C₆ alkylene chain possibly substituted by one or more (C₁-C₆)alkoxy, di(C₁-C₆)alkyl amino or (C₁-C₆)alkyl thio groups, a (C₃-C₈)cycloalkylene group possibly substituted by one or more (C₁-C₆)alcoxy, di(C₁-C₆)alkyl amino or (C₁-C₆)alkyl thio groups, a (C₆-C₁₀)arylene group possibly substituted by one or more (C₁-C₆)alcoxy, di(C₁-C₆)alkyl amino or (C₁-C₆)alkyl thio groups, or a -(CH₂)ⱼ-B^{"}-(CH₂)ⱼ- group, wherein j represents a whole number from 1 to 3 and B" represents (C₃-C₈)cycloalkylene (possibly substituted by one or more (C₁-C₆)alcoxy, di(C₁-C₆)alkyl amino or (C₁-C₆)alkyl thio) or (C₆-C₁₀)arylene (possibly substituted by one or more (C₁-C₆)alcoxy, di(C₁-C₆)alkyl amino or (C₁-C₆)alkyl thio groups);
• F₁ and F₂ are identical and represent (C₁-C₆)alkyl possibly substituted by one or more (C₁-C₆)alcoxy, said alkyl group having at least one chiral centre, (C₃-C₈)cycloalkyl substituted by one or more (C₁-C₆)alcoxy or (C₁-C₆)alkyl, said cycloalkyl having at least one chiral centre, or F₁ and F₂ together form a (C₁-C₆)alkylene chain possibly broken by two oxygen or sulphur atoms, said chain being substituted by one or more (C₁-C₆)alkyl or(C₁-C₆)alcoxy groups, two of the carbon atoms of said chain forming asymmetric centres.

20. Polymer according to Claim 18 or 19, **characterised in that** the chiral body of the diphosphine has the following structure: 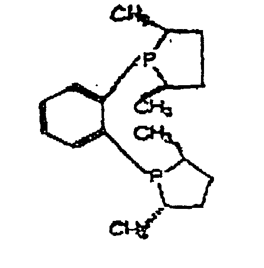 or certain dia-stereoisomeric form.

21. Polymer according to any one of the preceding claims, **characterised in that** said diphosphine is formed from a chiral body having two -CH₂NH₂ groups.

22. Polymer according to Claim 21, **characterised in that** it has the following recurrent pattern: 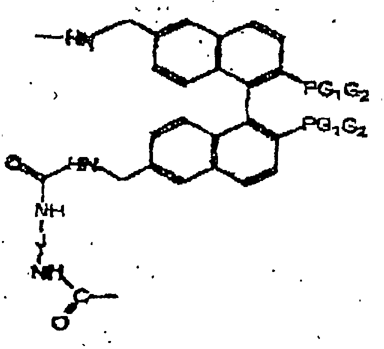 wherein:
• G₁ and G₂ independently represent a saturated or aromatic carbocyclic group; and
• J represents a divalent hydrocarbon radical of aliphatic, alicyclic and/or aromatic nature;
the degree of polymerisation being in the range of between 2 and 100, preferably between 2 and 50.

23. Polymer according to any one of the preceding claims, **characterised in that** the polymer may be obtained by polymerisation of said diphosphine with a polymerisable monomer having a plane of symmetry or a C₂ axis of symmetry, preferably a C₂ axis of symmetry.

24. Polymer according to any one of the preceding claims, **characterised in that** the polymerisable monomers are di-isocyanates.

25. Process for the preparation of an optically active polymer according to any one of Claims 1 to 24, **characterised in that** a chiral diphosphine having a C₂ plane of symmetry to the exclusion of any other element of symmetry, is polymerised with one or more polymerisable monomers, said chiral diphosphine being formed from a chiral body having two identical functional groups capable of reacting with said polymerisable monomers.

26. Use of a polymer according to any one of Claims 1 to 24 as ligand for the preparation of a metallic complex suitable for asymmetric catalysis.

27. Use according to Claim 26, **characterised in that** the metallic complex is a ruthenium, or rhodium complex.

28. Use according to Claim 27 in the hydrogenarion of C=O bonds or C=C bonds.

29. Racemic polymer corresponding to the polymer according to any one of Claims 1 to 24.

30. Use of an association of an optically active polymer according to any one of Claims 1 to 24 with a diamine for the selective reduction of ketones.

31. Use of an association of a racemic polymer according to Claim 29 with a chiral diamine for the selective reduction of ketones.

32. Use according to either of Claims 30 and 31, wherein the diamine is 1,2-diamino-1,2-diphenylethane.

33. Polymer according to Claim 21, **characterised in that** it has the following recurrent pattern: 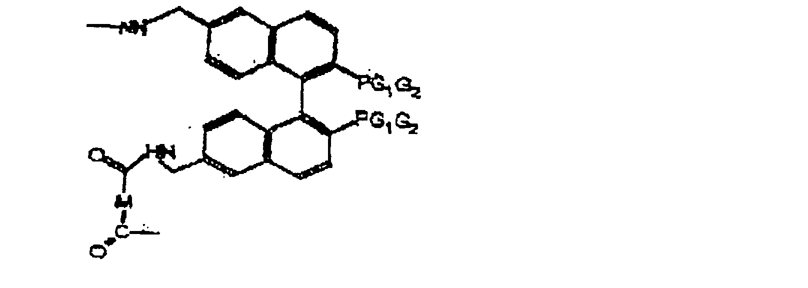 wherein:
• G₁ and G₂ are as defined in Claim 22; and
• H is as defined for J in Claim 22.
